# EUROPÄISCHE PATENTSCHRIFT

(11) **EP 1 414 817 B1**
(45) Veröffentlichungstag und Bekanntmachung des Hinweises auf die Patenterteilung: **06.04.2005**
(21) Anmeldenummer: 02751145.0
(22) Anmeldetag: 17.07.2002
(51) Int. Cl.: C07D 409/12, C07D 409/14, C07D 495/04, C07D 521/00, A61K 31/40, A61K 31/415, A61K 31/44, A61K 31/445, A61K 31/495, A61K 31/505, A61K 31/535, A61K 31/55, A61P 7/02

(54) **SUBSTITUIERTE ISOINDOLE UND IHRE VERWENDUNG**
substituted isoindoles and the use thereof
ISOINDOLES SUBSTITUES ET LEUR UTILISATION

(30) Priorität: 30.07.2001 DE 10137163
(43) Veröffentlichungstag der Anmeldung: 06.05.2004
(73) Patentinhaber: Bayer HealthCare AG, 51368 Leverkusen (DE)
(72) Erfinder: STRAUB, Alexander, 42117 Wuppertal (DE); LAMPE, Thomas, 40223 Düsseldorf (DE); POHLMANN, Jens, 42285 Wuppertal (DE); RÖHRIG, Susanne, 45276 Essen (DE); PERZBORN, Elisabeth, 42327 Wuppertal (DE); SCHLEMMER, Karl-Heinz, 42113 Wuppertal (DE)
(74) Vertreter: Linkenheil, Dieter
(86) Internationale Anmeldenummer: PCT/EP2002/007957
(87) Internationale Veröffentlichungsnummer: WO 2003/011858

(56) Entgegenhaltungen:
- WO-A-01/77075
- WO-A-02/34711
- WO-A-99/00121
- US-A- 6 140 351
- DATABASE CHEMCATS [Online] Chemical Abstracts Service, Columbus, Ohio, US; retrieved from STN, XP002215164 & "Interchim Intermediates" 9. Juli 2002 (2002-07-09) , INTERCHIM , 213 AVENUE KENNEDY, BP 1140, MONTLUCON, CEDEX 03103, FRANCE

## Beschreibung

Die vorliegende Erfindung betrifft das Gebiet der Blutgerinnung. Insbesondere betrifft die vorliegende Erfindung neue Isoindol-Derivate, Verfahren zu ihrer Herstellung sowie ihre Verwendung als Wirkstoffe in Arzneimitteln.

Die Blutgerinnung ist ein Schutzmechanismus des Organismus, mit dessen Hilfe Defekte in der Gefäßwand rasch und zuverlässig "abgedichtet" werden können. So kann ein Blutverlust vermieden bzw. minimiert werden. Die Blutstillung nach Gefäßverletzung erfolgt im wesentlichen durch das Gerinnungssystem, bei dem eine enzymatische Kaskade komplexer Reaktionen von Plasmaproteinen ausgelöst wird. Hierbei sind zahlreiche Blutgerinnungsfaktoren beteiligt, von denen jeder, sobald aktiviert, die jeweils nächste inaktive Vorstufe in ihre aktive Form überführt. Am Ende der Kaskade steht die Umwandlung des löslichen Fibrinogens in das unlösliche Fibrin, so dass es zu einem Blutgerinnsel kommt Traditionell unterscheidet man bei der Blutgerinnung zwischen dem intrinsischen und extrinsischen System, die in einem abschließenden gemeinsamen Reaktionsweg münden. Hierbei kommt dem Faktor Xa, der aus dem Proenzym Faktor X gebildet wird, eine Schlüsselrolle zu, da er beide Gerinnungswege verbindet Die aktivierte Serinprotease Xa spaltet Prothrombin zu Thrombin. Das entstandene Thrombin wiederum spaltet seinerseits Fibrinogen zu Fibrin, einem faserig-gallertigem Gerinnungsstoff. Darüber hinaus ist Thrombin ein potenter Auslöser der Thrombozytenaggregation, die ebenfalls einen erheblichen Beitrag bei der Hämostase leistet.

Die Aufrechterhaltung der normalen Hämostase - zwischen Blutung und Thrombose - unterliegt einem komplexen Regulationsmechanismus. Die unkontrollierte Aktivierung des Gerinnungssystems oder eine defekte Hemmung der Aktivierungsprozesse kann die Bildung von lokalen Thromben oder Embolien in Gefäßen (Arterien, Venen, Lymphgefäßen) oder Herzhöhlen bewirken. Dies kann zu schwerwiegenden Erkrankungen wie Herzinfarkt, Angina Pectoris (eingeschlossen instabile Angina), Reokklusionen und Restenosen nach einer Angioplastie oder aortokoronarem Bypass, Hirnschlag, transitorische ischämische Attacken, periphere arterielle Verschlusskrankheiten, Lungenembolien oder tiefen venösen Thrombosen führen; diese Erkrankungen werden im folgenden zusammenfassend auch als thromboembolische Erkrankungen bezeichnet. Darüber hinaus kann eine Hyperkoagulabilität - systemisch - bei einer Verbrauchskoagulopathie zur disseminierten intravasalen Gerinnung führen.

Diese thromboembolischen Erkrankungen sind die häufigste Ursache von Morbidität und Mortalität in den meisten industrialisierten Ländern (Pschyrembel, Klinisches Wörterbuch, 257. Auflage, 1994, Walter de Gruyter Verlag, Seite 199 ff., Stichwort "Blutgerinnung"; Römpp Lexikon Chemie, Version 1.5, 1998, Georg Thieme Verlag Stuttgart, Stichwort "Blutgerinnung"; Lubert Stryer, Biochemie, Spektrum der Wissenschaft Verlagsgesellschaft mbH Heidelberg, 1990, Seiten 259 ff.).

Die aus dem Stand der Technik bekannten Antikoagulantien, d.h. Stoffe zur Hemmung oder Verhinderung der Blutgerinnung, weisen verschiedene, oftmals gravierende Nachteile auf. Eine effiziente Behandlungsmethode bzw. Prävention von thromboembolischen Erkrankungen erweist sich in der Praxis deshalb als sehr schwierig und unbefriedigend.

Für die Therapie und Prävention von thromboembolischen Erkrankungen findet zum einen Heparin Verwendung, das parenteral oder subkutan appliziert wird. Aufgrund günstigerer pharmakokinetischer Eigenschaften wird zwar heutzutage zunehmend niedermolekulares Heparin bevorzugt; allerdings können auch hierdurch die im folgenden geschilderten bekannten Nachteile nicht vermieden werden, die bei der Therapierung mit Heparin bestehen. So ist Heparin oral unwirksam und besitzt nur eine vergleichsweise geringe Halbwertszeit. Da Heparin gleichzeitig mehrere Faktoren der Blutgerinnungskaskade hemmt, kommt es zu einer unselektiven Wirkung. Darüber hinaus besteht ein hohes Blutungsrisiko, insbesondere können Hirnblutungen und Blutungen im Gastrointestinaltrakt auftreten, und es kann zu Thrombopenie, Alopecia medicomentosa oder Osteoporose kommen (Pschyrembel, Klinisches Wörterbuch, 257. Auflage, 1994, Walter de Gruyter Verlag, Seite 610, Stichwort "Heparin"; Römpp Lexikon Chemie, Version 1.5, 1998, Georg Thieme Verlag Stuttgart, Stichwort "Heparin").

Eine zweite Klasse von Antikoagulantien stellen die Vitamin K-Antagonisten dar. Hierzu gehören beispielsweise 1,3-Indandione, vor allem aber Verbindungen wie Warfarin, Phenprocoumon, Dicumarol und andere Cumarin-Derivate, die unselektiv die Synthese verschiedener Produkte bestimmter Vitamin K-abhängiger Gerinnungsfaktoren in der Leber hemmen. Durch den Wirkmechanismus bedingt, setzt die Wirkung aber nur sehr langsam ein (Latenzzeit bis zum Wirkeintritt 36 bis 48 Stunden). Die Verbindungen können zwar oral appliziert werden, aufgrund des hohen Blutungsrisikos und des engen therapeutischen Indexes ist aber eine aufwendige individuelle Einstellung und Beobachtung des Patienten notwendig. Darüber hinaus sind weitere Nebenwirkungen wie gastrointestinale Störungen, Haarausfall und Hautnekrosen beschrieben (Pschyrembel, Klinisches Wörterbuch, 257. Auflage, 1994, Walter de Gruyter Verlag, Seite 292 ff., Stichwort "Cumarinderivate"; Ulimann's Encyclopedia of Industrial Chemistry, 5. Auflage, VCH Verlagsgesellschaft, Weinheim, 1985 - 1996, Stichwort "Vitamin K").

In jüngster Zeit ist ein neuer Therapieansatz für die Behandlung und Prävention von thromboembolischen Erkrankungen beschrieben worden. Ziel dieses neuen Therapieansatzes ist die Inhibierung von Faktor Xa (vgl. WO 02/34711, WO 99/00121, US 6 140 351, WO-A-99/37304; WO-A-99/06371; J. Hauptmann, J. Stürzebecher, Thrombosis Research 1999, 93, 203; F. Al-Obeidi, J. A. Ostrem, Factor Xa inhibitors by classical and combinatorial chemistry, DDT 1998, *3*, 223; F. Al-Obeidi, J. A. Ostrem, Factor Xa inhibitors, Exp. Opin. Ther. Patents **1999,** *9*, 931; B. Kaiser, Thrombin and factor Xa inhibitors, Drugs of the Future **1998,** *23*, 423; A. Uzan, Antithrombotic agents, Emerging Drugs **1998,** *3*, 189; B.-Y. Zhu, R M. Scarborough, Curr. Opin. Card. Pulm. Ren. Inv. Drugs **1999,** *1 (1),* 63). Dabei ist gezeigt worden, dass verschiedene, sowohl peptidische wie nichtpeptidische Verbindungen in Tiermodellen als Faktor Xa-Inhibitoren wirksam sind.

Aufgabe der vorliegenden Erfindung ist nunmehr die Bereitstellung neuer Substanzen zur Bekämpfung von Erkrankungen, die eine große therapeutische Bandbreite aufweisen.

Sie sollen insbesondere zur effizienteren Prävention und/oder Behandlung von thromboembolischen Erkrankungen geeignet sein und hierbei die zuvor geschilderten Nachteile des Standes der Technik - zumindest teilweise - vermeiden, wobei unter dem Begriff "thromboembolische Erkrankungen" im Sinne der vorliegenden Erfindung insbesondere schwerwiegende Erkrankungen wie Herzinfarkt, Angina Pectoris (eingeschlossen instabile Angina), Reokklusionen und Restenosen nach einer Angioplastie oder aortokoronarem Bypass, Himschlag, transitorische ischämische Attacken, periphere arterielle Verschlusskrankheiten, Lungenembolien oder tiefe venöse Thrombosen verstanden werden.

Weitere Aufgabe der vorliegenden Erfindung ist die Bereitstellung neuer Antikoagulantien, welche mit erhöhter Selektivität den Blutgerinnungsfaktor Xa inhibieren und hierbei die Probleme der aus dem Stand der Technik bekannten Therapiemethoden für thromboembolische Erkrankungen - zumindest teilweise - vermeiden sollen.

Gegenstand der vorliegenden Erfindung sind Verbindungen der Formel (I) 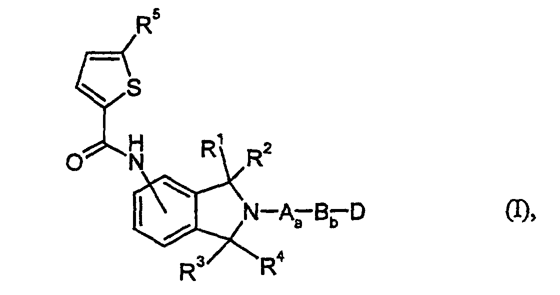 worin
- R¹ und R²: gemeinsam für O stehen und
- R³ und R⁴: gemeinsam für O stehen,
oder
- R¹: Wasserstoff, Hydroxy oder (C₁-C₄)-Alkoxy bedeutet,
- R²: Wasserstoff bedeutet und
- R³ und R⁴: gemeinsam für O stehen,
oder
- R¹ und R²: gemeinsam für O stehen,
- R³: Wasserstoff, Hydroxy oder (C₁-C₄)-Alkoxy bedeutet und
- R⁴: Wasserstoff bedeutet,
- R⁵: Halogen, Trifluormethyl oder Methyl bedeutet,
- A: (C₁-C₄)-Alkandiyl bedeutet, das durch Hydroxy oder (C₁-C₄)-Alkoxy substituiert sein kann,
- a: 0 oder 1 bedeutet,
- B: für eine Gruppe 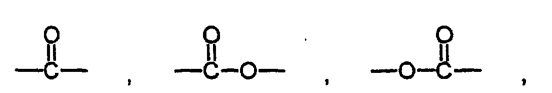 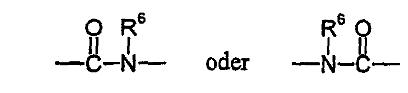 steht,
worin
R⁶ Wasserstoff oder (C₁-C₄)-Alkyl bedeutet,
- b: 0 oder 1 bedeutet,
- D: 5- bis 7-gliedriges Heterocyclyl,
das ein- oder zweifach, unabhängig voneinander, durch Hydroxy, Carbamoyl, (C₁-C₄)-Alkanoyl, (C₃-C₇)-Cycloalkanoyl, (C₃-C₇)-Cycloalkyl, 5- bis 10-gliedriges Heterocyclyl, (C₁-C₄)-Alkoxycarbonyl, (C₁-C₆)-Alkyl,
welches seinerseits durch Hydroxy, Cyano, (C₁-C₄)-Alkoxy, Mono- oder Di-(C₁-C₆)-alkylamino, Mono- oder Di-(C₁-C₆)-alkylaminocarbonyl, 5- bis 10-gliedriges Heterocyclyl, 5- oder' 6-gliedriges Heterocyclylcarbonyl oder 5- bis 10-gliedriges Heteroaryl substituiert sein kann,
(C₆-C₁₀)-Aryl,
welches seinerseits durch Halogen, Trifluormethyl, Nitro, (C₁-C₄)-Alkyl oder (C₁-C₄)-Alkoxy substituiert sein kann, 5- bis 10-gliedriges Heteroaryl,
welches seinerseits durch Cyano, Amino oder (C₁-C₄)-Alkyl substituiert sein kann,
oder 5- bis 10-gliedriges Heteroarylcarbonyl substituiert sein kann,
(C₁-C₆)-Alkyl,
das durch Cyano, Amino, Mono- oder Di-(C₁-C₆)-alkylamino, Amidino, 5- bis 10-gliedriges Heteroaryl, gegebenenfalls durch Halogen substituiertes 5- bis 10-gliedriges Heteroarylamino, gegebenenfalls durch 5- bis 10-gliedriges Heteroaryl substituiertes 5- bis 10-gliedriges Heterocyclyl oder (C₁-C₄)-Alkanoylamino substituiert sein kann,
oder
- 5- bis: 10-gliedriges Heteroaryl,
das durch Halogen oder (C₁-C₄)-Alkyl substituiert sein kann, bedeutet,
sowie deren Salze, Hydrate, Hydrate der Salze und Solvate.

Die erfindungsgemäßen Verbindungen der Formel (I) können in Abhängigkeit von dem Substitutionsmuster in stereoisomeren Formen, die sich entweder wie Bild und Spiegelbild (Enantiomere) oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen.

Weiterhin können bestimmte Verbindungen der Formel (I) in tautomeren Formen vorliegen. Dies ist dem Fachmann bekannt, und derartige Verbindungen sind ebenfalls vom Umfang der Erfindung umfasst.

Salze der erfindungsgemäßen Verbindungen sind physiologisch unbedenkliche Salze der erfindungsgemäßen Verbindungen mit anorganischen oder organischen Säuren. Bevorzugt werden Salze mit anorganischen Säuren wie beispielsweise Chlorwasserstoffsäure, Bromwasserstoffsäure, Phosphorsäure oder Schwefelsäure, oder Salze mit organischen Carbon- oder Sulfonsäuren wie beispielsweise Essigsäure, Trifluoressigsäure, Propionsäure, Maleinsäure, Fumarsäure, Äpfelsäure, Zitronensäure, Weinsäure, Milchsäure, Benzoesäure, oder Methansulfonsäure, Ethansulfonsäure, Benzolsulfonsäure, Toluolsulfonsäure oder Naphthalindisulfonsäure.

Salze können ebenso physiologisch unbedenkliche Salze mit üblichen Basen sein, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldüsopropylamin, Prokain, Dibenzylamin, N-Methylmorpholin oder Methylpiperidin.

Ausserdem umfasst die Erfindung auch Prodrugs der erfindungsgemäßen Verbindungen. Als Prodrugs werden erfindungsgemäß solche Formen der Verbindungen der Formel (I) bezeichnet, welche selbst biologisch aktiv oder inaktiv sein können, jedoch unter physiologischen Bedingungen in die entsprechende biologisch aktive Form überführt werden können (beispielsweise metabolisch oder solvolytisch).

Als "Hydrate" bzw. "Solvate" werden erfindungsgemäß solche Formen der Verbindungen der Formel (I) bezeichnet, welche in festem oder flüssigem Zustand durch Hydratation mit Wasser oder Koordination mit Lösungsmittelmolekülen eine Molekül-Verbindung bzw. einen Komplex bilden. Beispiele für Hydrate sind Sesquihydrate, Monohydrate, Dihydrate oder Trihydrate. Gleichermaßen kommen auch die Hydrate bzw. Solvate von Salzen der erfindungsgemäßen Verbindungen in Betracht.

Halogen steht für Fluor, Chlor, Brom und Iod. Bevorzugt sind Chlor, Brom oder Fluor.

(C₁-C₆)-Alkyl steht für einen geradkettigen oder verzweigten Alkylrest mit 1 bis 6 Kohlenstoffatomen. Beispielsweise seien genannt: Methyl, Ethyl, n-Propyl, Isopropyl, n-Butyl, Isobutyl und tert.-Butyl. Aus dieser Definition leiten sich analog die entsprechenden Alkylgruppen mit weniger Kohlenstoffatomen wie z.B. (C₁-C₄)-Alkyl oder (C₁-C₃)-Alkyl ab. Im Allgemeinen gilt, dass (C₁-C₃)-Alkyl bevorzugt ist.

Aus dieser Definition leitet sich auch die Bedeutung des entsprechenden Bestandteils anderer komplexerer Substituenten ab wie z.B. bei Mono- -oder Dialkylamino oder Mono- oder Dialkylaminocarbonyl.

Monoalkylamino steht für eine Amino-Gruppe mit einem wie oben definierten Alkylsubstituenten. Dialkylamino steht für eine Amino-Gruppe mit zwei, gleichen oder verschiedenen, wie oben definierten Alkylsubstituenten. Mono- oder Dialkylaminocarbonyl steht für eine wie oben definierte Mono- oder Dialkylamino-Gruppe, die über eine Carbonylgruppe verknüpft ist.

(C₁-C₄)-Alkandiyl steht für einen geradkettigen oder verzweigten Alkandiylrest mit 1 bis 4 Kohlenstoffatomen. Bevorzugt ist ein geradkettiger oder verzweigter Alkandiylrest mit 1 bis 3 Kohlenstoffatomen. Beispielsweise seien genannt: Methandiyl, Ethandiyl, Propan-1,3-diyl, Propan-1,2-diyl, Propan-2,2-diyl, Butan-1,4-diyl, Butan-1,3-diyl.

(C₃-C₇)-Cycloalkyl steht für einen cyclischen Alkylrest mit 3 bis 7 Kohlenstoffatomen. Beispielsweise seien genannt: Cyclopropyl, Cyclobutyl, Cyclopentyl, Cyclohexyl oder Cycloheptyl. Aus dieser Definition leiten sich die entsprechenden Cycloalkylreste mit weniger Kohlenstoffatomen wie (C₃-C₆)-Cycloalkyl ab. Bevorzugt sind Cyclopropyl, Cyclopentyl und Cyclohexyl.

Aus dieser Definition leitet sich auch die Bedeutung des entsprechenden Bestandteils anderer komplexerer Substituenten ab wie z.B. Cycloalkylamino. Cycloalkylamino steht für einen wie oben definierten Cycloalkylrest, der als Substituenten eine eine Aminogruppe trägt und über das Aminostickstoffatom verknüpft ist.

(C₁-C₄)-Alkanoyl steht für einen geradkettigen oder verzweigten Alkylrest mit 1 bis 4 Kohlenstoffatomen, der in der 1-Position ein doppelt gebundenes Sauerstoffatom trägt und über die 1-Position verknüpft ist. Beispielsweise seien genannt: Formyl, Acetyl, Propionyl, n-Butyryl, i-Butyryl.

Aus dieser Definition leitet sich auch die Bedeutung des entsprechenden Bestandteils anderer komplexerer Substituenten ab wie z.B. Cycloalkanoyl oder Alkanoylamino. Cycloalkanoyl steht für einen wie oben definierten Cycloalkylrest, der als Substituenten eine Carbonylgruppe trägt und über diese Carbonylgruppe verknüpft ist. Alkanoylamino steht für einen wie oben definierten Alkanoylrest, der an der 1-Position neben dem doppelt gebundenen Sauerstoffatom zusätzlich als Substituenten eine Aminogruppe trägt und über dieses Aminostickstoffatom verknüpft ist.

(C₁-C₄)-Alkoxy steht für einen geradkettigen oder verzweigten Alkoxyrest mit 1 bis 4 Kohlenstoffatomen. Beispielsweise seien genannt: Methoxy, Ethoxy, n-Propoxy, Isopropoxy, n-Butoxy, Isobutoxy, tert.-Butoxy. Aus dieser Definition leiten sich analog die entsprechenden Alkoxygruppen mit weniger Kohlenstoffatomen wie z.B. (C₁-C₃)-Alkoxy ab. Im Allgemeinen gilt, dass (C₁-C₃)-Alkoxy bevorzugt ist.

(C₁-C₄)-Alkoxycarbonyl steht für einen geradkettigen oder verzweigten Alkoxyrest mit 1 bis 4 Kohlenstoffatomen, der über eine Carbonylgruppe verknüpft ist. Beispielsweise seien genannt Methoxycarbonyl, Ethoxycarbonyl, n-Propoxycarbonyl, Isopropoxycarbonyl und t-Butoxycarbonyl. Bevorzugt ist ein Alkoxycarbonylrest mit 1 oder 2 Kohlenstoffatomen.

(C₆-C₁₀)-Aryl steht für einen aromatischen Rest mit 6 bis 10 Kohlenstoffatomen. Beispielsweise seien genannt: Phenyl und Naphthyl. Im Allgemeinen gilt, dass Phenyl bevorzugt ist.

5- bis 10-gliedriges Heteroaryl steht für einen mono- oder bicyclischen gegebenenfalls benzokondensierten aromatischen Heterocyclus (Heteroaromaten) mit bis zu 3 Heteroatomen aus der Reihe N, O und/oder S, der über ein Ringkohlenstoffatom oder Ringstickstoffatom des Heteroaromaten verknüpft ist. Beispielsweise seien genannt: Pyridyl, Pyridyl-N-oxid, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, Indazolyl, Chinolyl, Isochinolyl, Naphthyridinyl, Chinazolinyl. Aus dieser Definition leiten sich analog die entsprechenden Heteroaromaten mit geringerer Ringgröße wie z.B. 5- oder 6-gliedriges Heteroaryl ab. Im Allgemeinen gilt, dass 5- oder 6-gliedriges Heteroaryl wie z.B. Pyridyl, Pyrimidyl, Pyridazinyl und Pyrazinyl bevorzugt sind.

Aus dieser Definition leitet sich auch die Bedeutung des entsprechenden Bestandteils anderer komplexerer Substituenten ab wie z.B. Heteroarylcarbonyl. Heteroarylcarbonyl steht für einen wie oben definierten Heteroarylrest, der als Substituenten eine Carbonylgruppe trägt und über das Kohlenstoffatom dieser Carbonylgruppe verknüpft ist.

Aus dieser Definition leitet sich auch die Bedeutung des entsprechenden Bestandteils anderer komplexerer Substituenten ab wie z.B. Heteroarylamino. Heteroarylamino steht für einen wie oben definierten Heteroarylrest, der als Substituenten eine Aminogruppe trägt und über das Stickstoffatom dieser Aminogruppe verknüpft ist.

3- bis 10-gliedriges Heterocyclyl steht für einen gesättigten oder teilweise ungesättigten, mono- oder bicyclischen, gegebenenfalls benzokondensierten Heterocyclus mit bis zu 3 Heteroatomen aus der Reihe S, N und/oder O, der über ein Ringkohlenstoffatom oder ein Ringstickstoffatom verknüpft ist. Beispielsweise seien genannt: Oxiranyl, Tetrahydrofuryl, Pyrrolidinyl, Pyrrolinyl, Piperidinyl, 1,2-Dihydropyridinyl, 1,4-Dihydmpyridinyl, Piperazinyl, Hexahydrodiazepinyl, Morpholinyl, Morpholinyl-N-oxid, Thiomorpholinyl, Azepinyl und 1,4-Diazepinyl. Aus dieser Definition leiten sich analog die entsprechenden Heterocyclen mit geringerer Ringgröße wie z.B. 5-bis 7-gliedrige Heterocyclen oder 5- oder 6-gliedrige Heterocyclen ab. Im Allgemeinen gilt, dass 5- bis 7-gliedrige Heterocyclen wie Piperidinyl, Morpholinyl, Hexahydrodiazepinyl, Piperazinyl und Pyrrolidinyl bevorzugt sind.

Aus dieser Definition leitet sich auch die Bedeutung des entsprechenden Bestandteils anderer komplexerer Substituenten ab wie z.B. Heterocyclylcarbonyl, Heterocyclylcarbonyl steht für einen wie oben definierten Heterocyclylrest, der als Substituenten eine Carbonylgruppe trägt und über das Kohlenstoffatom dieser Carbonylgruppe verknüpft ist.

Bevorzugt sind Verbindungen der Formel (I),
worin
der Thiophencarbonsäuresubstituent in ortho-Position zur Anknüpfungsstelle des annelierten Heterocyclus mit dem Phenylring verknüpft ist,
- R¹ und R²: gemeinsam für O stehen und
- R³ und R⁴: gemeinsam für O stehen,
oder
- R¹: Wasserstoff, Hydroxy, Methoxy oder Ethoxy bedeutet,
- R²: Wasserstoff bedeutet und
- R³ und R⁴: gemeinsam für O stehen,
oder
- R¹ und R²: gemeinsam für O stehen,
- R³: Wasserstoff, Hydroxy, Methoxy oder Ethoxy bedeutet und
- R⁴: Wasserstoff bedeutet,
- R⁵: Halogen oder Trifluormethyl bedeutet,
- A: (C₁-C₄)-Alkandiyl bedeutet, das durch Hydroxy substituiert sein kann,
- a: 0 oder 1 bedeutet,
- B: für eine Gruppe 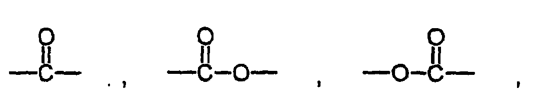 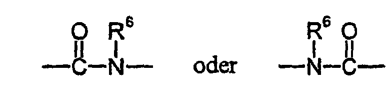 steht,
worin
R⁶ Wasserstoff bedeutet,
- b: 0 oder 1 bedeutet,
- D: 5- bis 7-gliedriges Heterocyclyl,
das ein- oder zweifach, unabhängig voneinander, durch Hydroxy, Carbamoyl, Acetyl, Cyclopropanoyl, (C₃-C₆)-Cycloalkyl, 5- bis 10-gliedriges Heterocyclyl, (C₁-C₃)-Alkyl,
welches seinerseits durch Hydroxy, Methoxy, Mono- oder Dimethylamino, Mono- oder Di-(C₁-C₃)-alkylaminocarbonyl, 5- oder 6-gliedriges Heterocyclyl, 5- oder 6-gliedriges Heterocyclylcarbonyl oder 5- oder 6-gliedriges Heteroaryl substituiert sein kann,
Phenyl,
welches seinerseits durch Fluor, Chlor, Trifluormethyl, Methyl oder Methoxy substituiert sein kann,
oder 5- oder 6-gliedriges Heteroaryl substituiert sein kann,
welches seinerseits durch Cyano, Amino oder Methyl substituiert sein kann,
(C₁-C₆)-Alkyl,
das durch Amino, Mono- oder Di-methylamino, Amidino, 5- oder 6-gliedriges Heteroaryl, 5- oder 6-gliedriges Heteroarylamino, gegebenenfalls durch 5- oder 6-gliedriges Heteroaryl substituiertes 5- oder 6-gliedriges Heterocyclyl oder (C₁-C₄)-Alkanoylamino substituiert sein kann,
oder
5- bis 9-gliedriges Heteroaryl bedeutet,
sowie deren Salze, Hydrate, Hydrate der Salze und Solvate.

Besonders bevorzugt sind Verbindungen der Formel (I),
worin
der Thiophencarbonsäuresubstituent in ortho-Position zur Anknüpfungsstelle des annelierten Heterocyclus mit dem Phenylring verknüpft ist,
- R¹ und R²: gemeinsam für O stehen und
- R³ und R⁴: gemeinsam für O stehen,
oder
- R¹: Wasserstoff, Hydroxy oder Methoxy bedeutet,
- R²: Wasserstoff bedeutet und
- R³ und R⁴: gemeinsam für O stehen,
oder
- R¹ und R²: gemeinsam für O stehen,
- R³: Wasserstoff, Hydroxy oder Methoxy bedeutet und
- R⁴: Wasserstoff bedeutet,
- R⁵: Chlor oder Brom bedeutet,
- A: Methandiyl, Ethandiyl oder Propan-1,3-diyl bedeutet, die durch Hydroxy substituiert sein können,
- a: 0 oder 1 bedeutet,
- B: für eine Gruppe 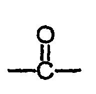 steht,
- b: 0 oder 1 bedeutet,
- D: Pyrrolidin, Piperidin oder Piperazin,
die ein- oder zweifach, unabhängig voneinander, durch Methyl, Ethyl, n-Propyl oder Isopropyl,
welche ihrerseits durch Hydroxy oder Pyridyl substituiert sein können, oder Pyridyl substituiert sein können,
welches seinerseits durch Amino oder Methyl substituiert sein kann,
oder
(C₁-C₃)-Alkyl bedeutet,
das durch Amidino, gegebenenfalls durch Pyridyl substituiertes Piperidinyl oder Acetylamino substituiert sein kann,
sowie deren Salze, Hydrate, Hydrate der Salze und Solvate.

Gegenstand der vorliegenden Erfindung ist auch ein Verfahren zur Herstellung der erfindungsgemäßen Verbindungen der Formel (I), wobei man
entweder
[A] Verbindungen der Formel (II) 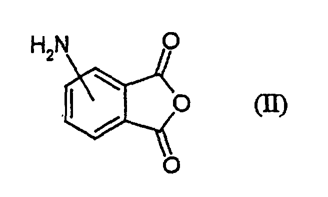
   [A.1] mit Verbindungen der Formel (III) 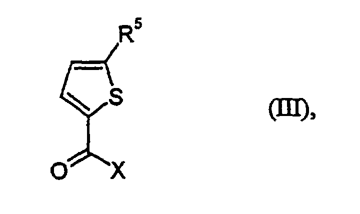 worin R⁵ die oben angegebene Bedeutung hat und X für eine Abgangsgruppe steht,
      in Verbindungen der Formel (IV) 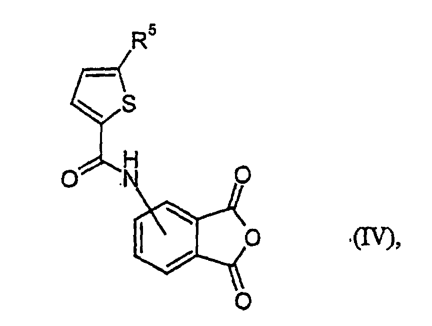 worin R⁵ die oben angegebene Bedeutung besitzt,
      überführt und diese anschließend
      entweder
      [A.1.1] mit Verbindungen der Formel (V)

         H₂N-Aₐ-B_{b}-D (V),

         worin A, a, B, b und D die oben angegebene Bedeutung besitzen,
         oder
      [A.1.2] mit Verbindungen der Formel (VI)

         H₂N-A-C(=O)-OH (VI),

         worin A die oben angegebene Bedeutung besitzt,
         über die Stufe von Verbindungen der Formel (VII) 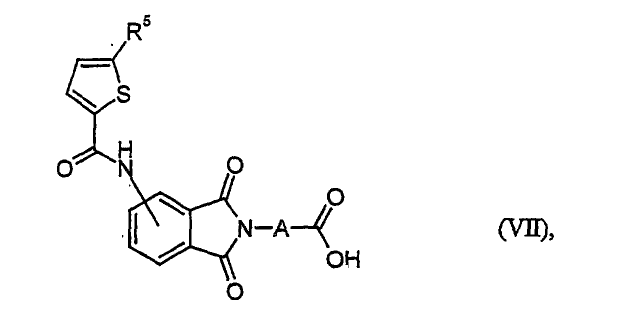 worin A und R⁵ die oben angegebene Bedeutung besitzen,
         und anschließende Reaktion mit Aminen oder Alkoholen
         zu Verbindungen der Formel (I) umsetzt
         oder
   [A.2] Verbindungen der Formel (II)
      entweder
      [A.2.1] mit Verbindungen der Formel (V)
         oder
      [A.2.2] mit Verbindungen der Formel (VI)
         über die Stufe von Verbindungen der Formel (VIII) 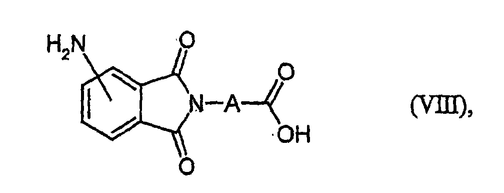 worin A die oben angegebene Bedeutung besitzt,
         und anschließende Reaktion mit Aminen oder Alkoholen
         in Verbindungen der Formel (IX) 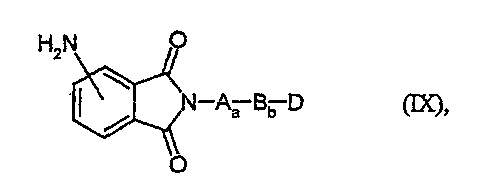 worin a, b, A, B und D die oben angegebene Bedeutung besitzen,
         überführt und dann mit Verbindungen der Formel (III) zu Verbindungen der Formel (I) umsetzt
      oder
[B] Verbindungen der Formel (X) 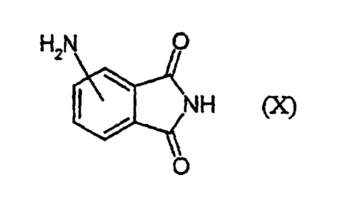
   [B.1] mit Verbindungen der Formel (III) in Verbindungen der Formel (XI) 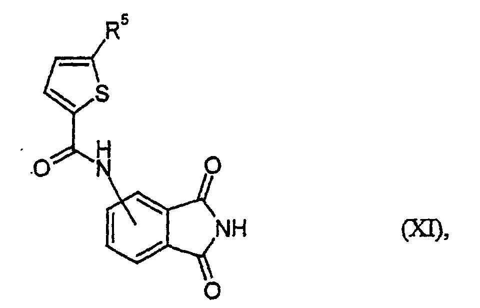 worin R⁵ die oben angegebene Bedeutung besitzt,
      überführt und diese anschließend mit Verbindungen der Formel (XII)

      HO-Aₐ-B_{b}-D (XII),

      worin A, a, B, b und D die oben angegebene Bedeutung besitzen,
      zu Verbindungen der Formel (I) umsetzt
      oder
   [B.2] mit Verbindungen der Formel (XII) in Verbindungen der Formel (IX) 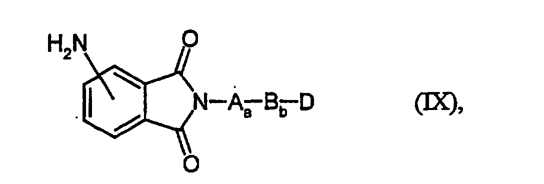 worin A, a, B, b und D die oben angegebene Bedeutung besitzen,
      überführt und diese anschließend mit Verbindungen der Formel (III)
      zu Verbindungen der Formel (I) umsetzt
      oder
[C] Verbindungen der Formel (XIV) 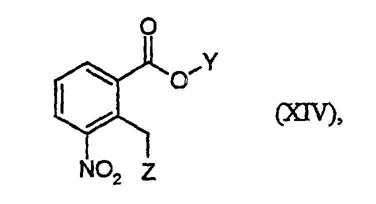 worin Y für (C₁-C₄)-Alkyl und Z für eine Abgangsgruppe steht,
   mit Verbindungen der Formel (V) zu Verbindungen der Formel (XV) 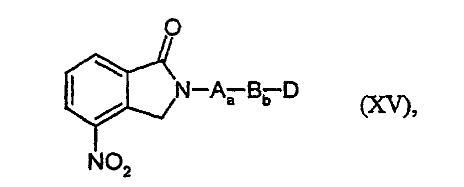 worin A, a, B, b und D die oben angegebene Bedeutung besitzen,
   umsetzt, dann durch Reduktion der Nitrogruppe in Verbindungen der Formel (XVI) 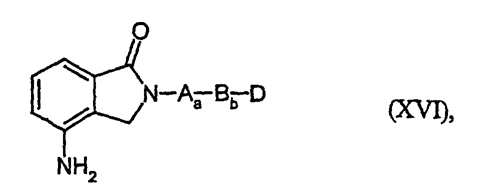 worin A, a, B, b und D die oben angegebene Bedeutung besitzen,
   überführt und dann mit Verbindungen der Formel (III) zu Verbindungen der Formel (I) umsetzt,
wobei sich bei den so erhaltenen Verbindungen der Formel (I) gegebenenfalls weitere Derivatisierungen, die nach üblichen Methoden durchgeführt werden können, anschließen können.

Das erfindungsgemäße Verfahren kann durch folgendes Formelschema beispielhaft erläutert werden:

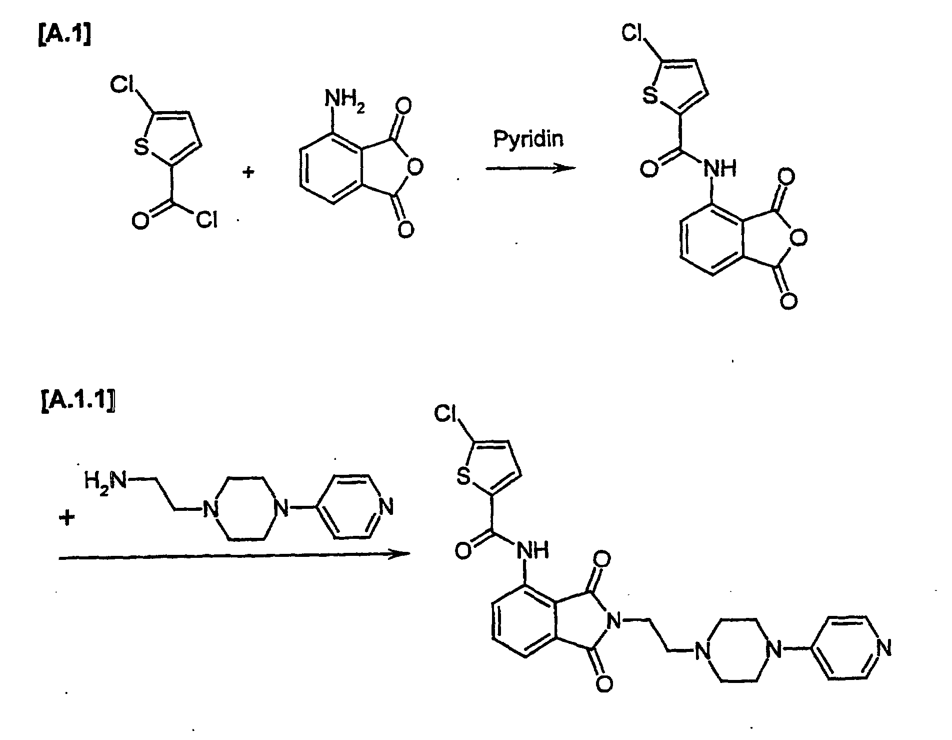

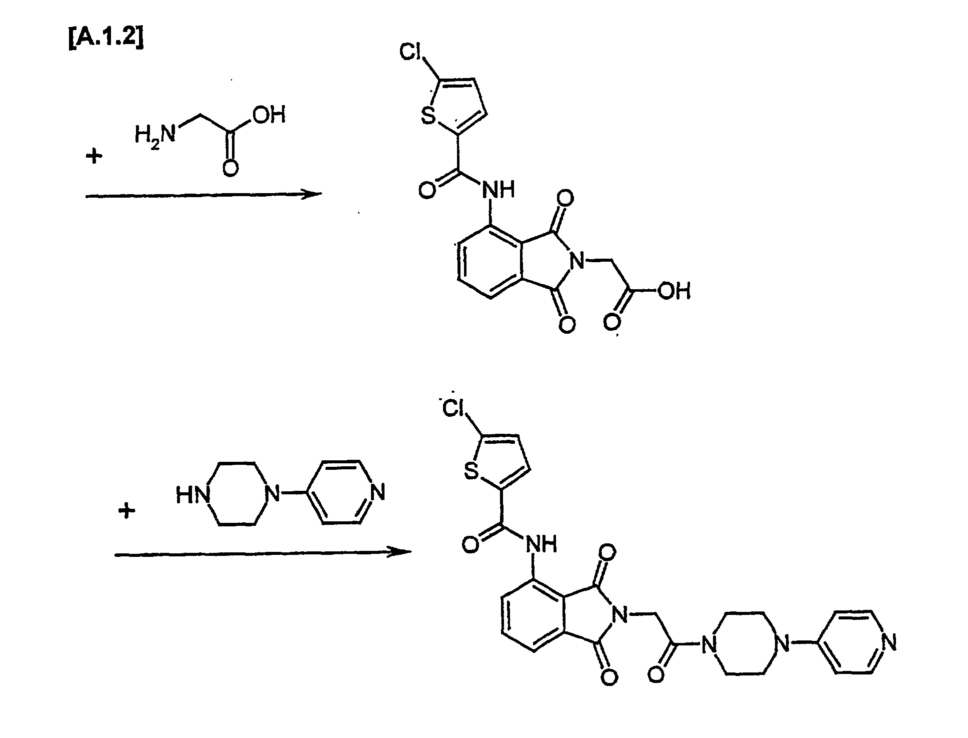

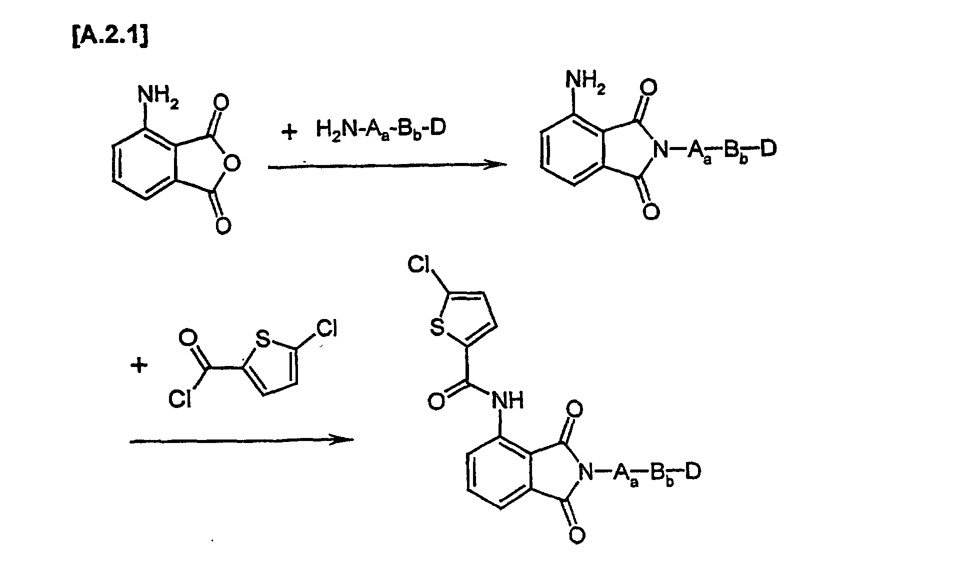

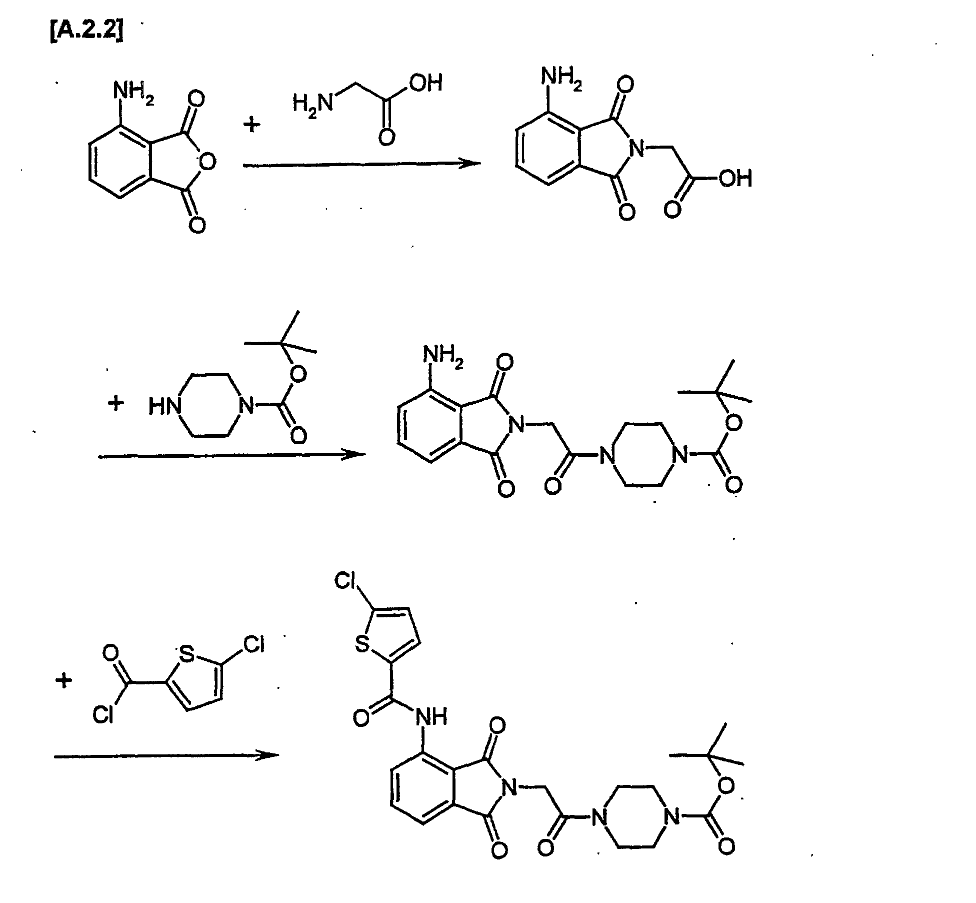

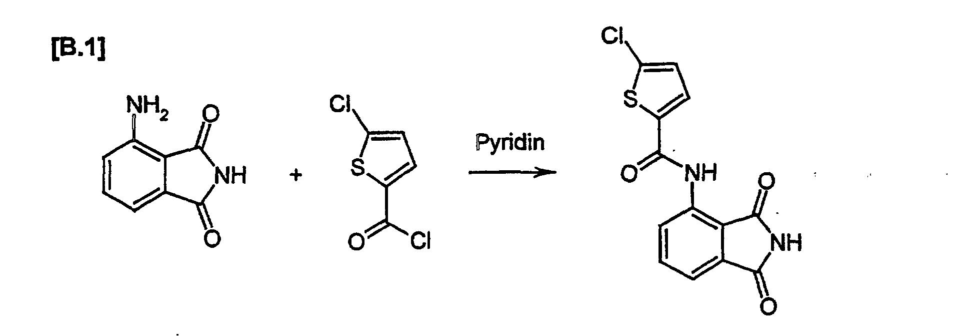

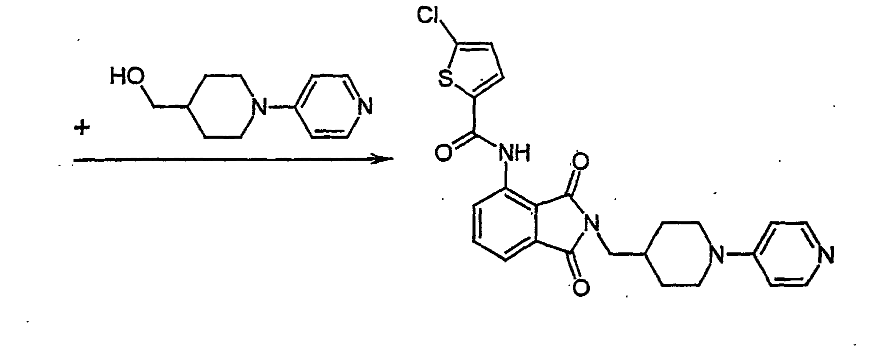

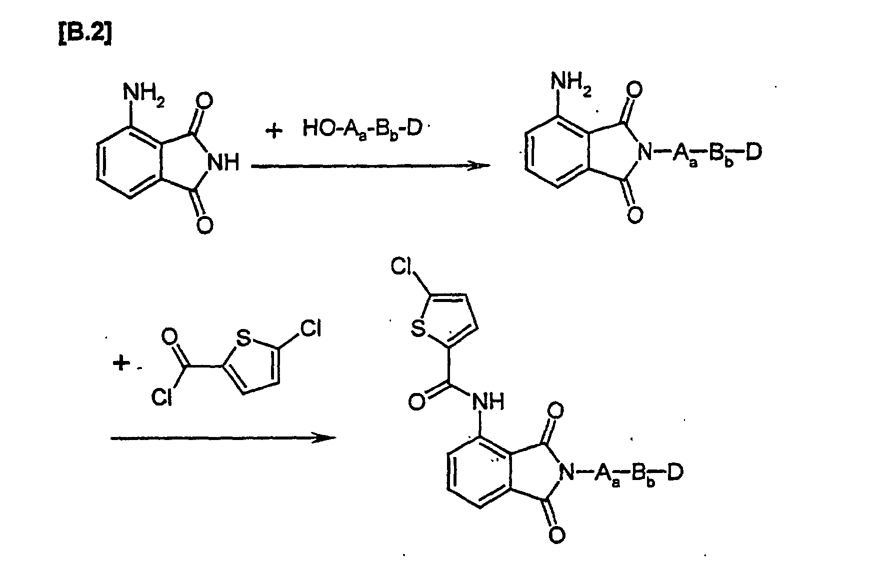

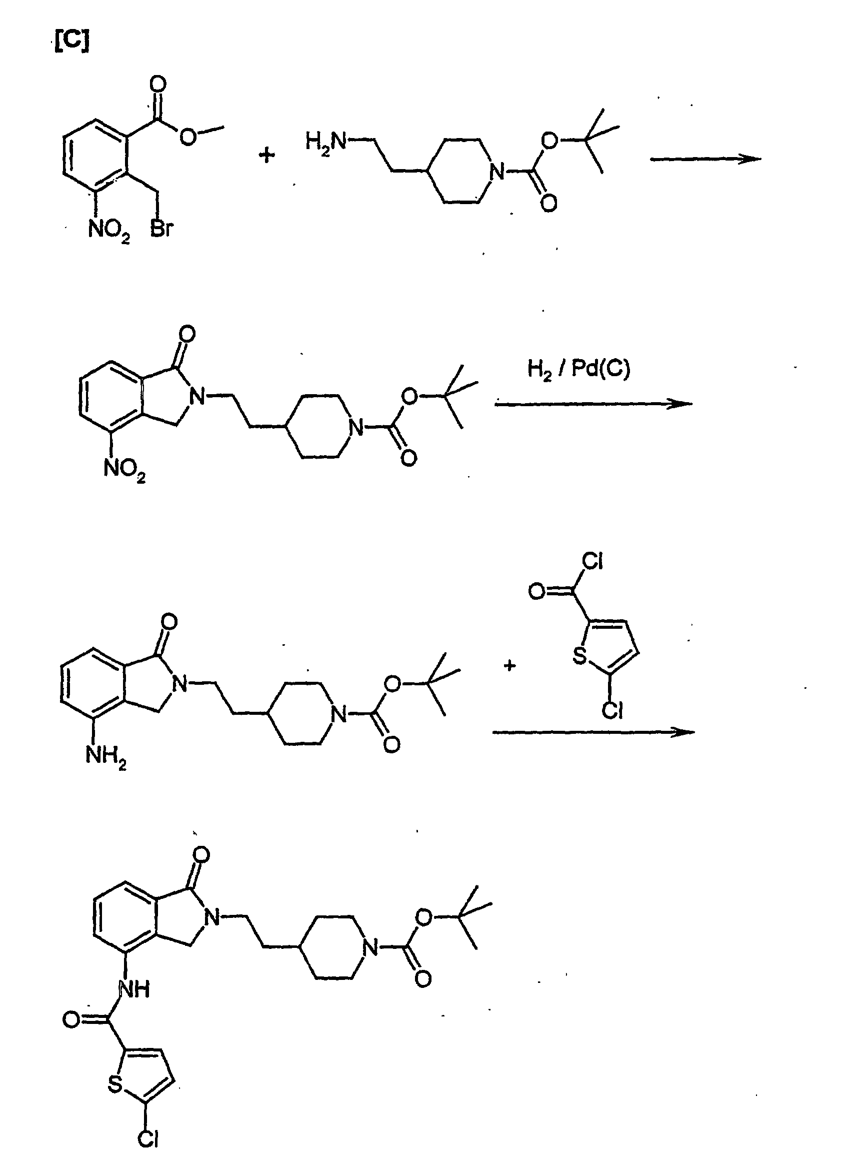

Als Lösemittel für das erfindungsgemäße Verfahren eignen sich alle organischen Lösemittel, die unter den Reaktionsbedingungen inert sind, oder Wasser. Hierzu gehören Alkohole wie Methanol, Ethanol und Isopropanol, Ketone wie Aceton und Methylethylketon, acyclische und cyclische Ether wie Diethylether, Tetrahydrofuran und Dioxan, Ester wie Essigsäureethylester oder Essigsäurebutylester, Kohlenwasserstoffe wie Benzol, Xylol, Toluol, Hexan oder Cyclohexan, Essigsäure, Dimethylformamid, Acetonitril, Pyridin, Dimethylsulfoxid (DMSO), chlorierte Kohlenwasserstoffe wie Dichlormethan, Chlorbenzol oder Dichlorethan. Ebenso ist es möglich, Gemische der zuvor genannten Lösemittel einzusetzen.

Als Basen eignen sich die üblichen anorganischen oder organischen Basen. Hierzu gehören bevorzugt Alkalihydroxide wie beispielsweise Natrium- oder Kaliumhydroxid oder Alkalicarbonate wie Natrium- oder Kaliumcarbonat oder Natriumoder Kaliumhydrogencarbonat oder Natrium- oder Kaliummethanolat oder Natriumoder Kaliumethanolat oder Kalium-tert.-butylat oder aber Amide wie Natriumamid, Lithium-bis-(trimethylsilyl)amid oder Lithiumdiisopropylamid oder metallorganische Verbindungen wie Butyllithium oder Phenyllithium oder aber auch Amine wie Trialkylamine z.B. Triethylamin, N-Methylmorpholin (NMM), N-Methylpiperidin, Diisopropylethylamin (Hünigbase) oder 4-*N,N-*Dimethylaminopyridin (DMAP) oder Pyridin.

Das erfindungsgemäße Verfahren kann bei normalem, erhöhtem oder erniedrigtem Druck durchgeführt werden (z.B. im Bereich von 0,5 bis 5 bar). Im Allgemeinen arbeitet man bei Normaldruck.

Die Umsetzung mit Thiophencarbonsäurederivaten der Formel (III) wie im Verfahrensschritt [A.1]: (II) + (III) → (IV); zweiten Verfahrensschritt [A.2]: (IX) + (III) → (I); ersten Verfabrensschritt [B.2]: (X) + (III) → (XI); zweiten Verfahrensschritt [B.2]: (XIII) + (III) → (I) und dritten Verfahrensschritt [C]: (XVI) + (III) → (I) erfolgt im Allgemeinen in einem Temperaturbereich von -78°C bis +120°C, bevorzugt im Bereich von -78°C bis +60°C, insbesondere bei 0°C bis +50°C. Als Abgangsgruppe X werden beispielsweise Halogene eingesetzt, also das entsprechende Säurechlorid oder -bromid, oder es werden die entsprechenden Säureanhydride eingesetzt. Bevorzugt ist das entsprechende Säurechlorid. Als Lösemittel bevorzugt ist Pyridin oder Tetrahydrofuran. Gegebenenfalls wird als Base 4-Dimethylaminopyridin (4-DMAP) oder Triethylamin zugegeben.

Im Verfahrensschritt [A.1.1]: (IV) + (V) → (I) und Verfahrensschritt [A.2.1]: (II) + (V) → (IX) erfolgt eine Umsetzung von Phthalsäureanhydriden mit primären Aminen zu den entsprechenden Phthalimiden. Als Lösemittel bevorzugt sind Essigsäure oder Dioxan. Die Umsetzung erfolgt im Allgemeinen in einem Temperaturbereich von -78°C bis +150°C, bevorzugt von 0°C bis +120°C, insbesondere bei der Rückflusstemperatur des Lösungsmittels.

Im Verfahrensschritt [A.1.2]: (IV) + (VI) → (VII) → (I) und Verfahrensschritt [A.2.2]: (II) + (VI) → (VIII) → (IX) erfolgt zunächst eine Umsetzung von Phthalsäureanhydriden mit Aminosäuren zu den entsprechenden Phthalimiden (VII) bzw. (IX). Als Lösemittel bevorzugt sind Dioxan oder Essigsäure. Die Umsetzung erfolgt im Allgemeinen in einem Temperaturbereich von -78°C bis +150°C, bevorzugt von 0°C bis +120°C, insbesondere bei der Rückflusstemperatur des Lösungsmittels.

Anschließend erfolgt in einer zweiten Stufe eine Derivatisierung der Säurefunktion der ursprünglichen Aminosäure durch Umsetzung mit Alkoholen bzw. Aminen zu den entsprechenden Estern bzw. Amiden. Bevorzugte Ausführungsformen sind beispielsweise die Reaktion in Dichlormethan oder Dimethylformamid als Lösungsmittel bei Raumtemperatur in Gegenwart von N'-(3-Dimethylaminopropyl)-N-ethylcarbodiimid Hydrochlorid (EDC), 1-Hydroxy-1*H*-benzotriazol Hydrat (HOBT) und einer Base wie Diisopropylethylamin (DIEA) oder Triethylamin.

Im zweiten Verfahrensschritt [B.1]: (XI) + (XII) → (I) und ersten Verfahrensschritt [B.2]: (X) + (XII) → (IX) erfolgt eine Derivatisierung von Phthalimiden in einer nukleophilen Substitutionsreaktion mit Verbindungen der Formel (XII). Die Hydroxygruppe in Verbindungen der Formel (XII) wird hierbei durch Reagenzien wie Diethylazodicarboxylat (DEAD)/PPh₃ aktiviert (Mitsunobu-Reaktion). Als Lösemittel bevorzugt ist Tetrahydrofuran. Die Umsetzung erfolgt im Allgemeinen in einem Temperaturbereich von -78°C bis +120°C, bevorzugt bei 0°C bis Raumtemperatur.

Die Alkylierung im ersten Verfahrensschritt [C]: (XIV) + (V) → (XV) erfolgt im Allgemeinen in einem Temperaturbereich von -78°C bis +120°C, bevorzugt bei +50°C bis +80°C. Als Abgangsgruppe Z in Verbindungen der Formel (XI) kommen beispielsweise in Frage: Halogen, Tosylat oder Mesylat, bevorzugt ist Brom. Als Lösemittel bevorzugt ist Dimethylformamid, als zusätzliche Base wird beispielsweise Triethylamin eingesetzt

Im zweiten Verfahrensschritt [C]: (XV)→ (XVI) wird eine aromatische Nitrogruppe in das entsprechende Amin überführt. Als Lösemittel bevorzugt sind Methanol, Ethanol, Tetrahydrofuran oder Essigester oder Gemische dieser Lösungsmittel. Die Umsetzung erfolgt im Allgemeinen in einem Temperaturbereich von -78°C bis +120°C, bevorzugt bei Raumtemperatur. Geeignet sind herkömmliche Hydrierverfahren, bevorzugt ist die Hydrierung mit Wasserstoff bei Atmosphärendruck in Gegenwart eines Katalysators wie beispielsweise Palladium auf Kohle [Pd(C); 10 Gew.%].

Die Verbindungen der Formeln (II), (III), (V), (VI), (X), (XII) und (XIV) sind kommerziell erhältlich, literaturbekannt oder nach üblichen literaturbekannten Methoden herstellbar.

Die erfindungsgemäßen Verbindungen der Formel (I) zeigen ein nicht vorhersehbares, wertvolles pharmakologisches Wirkspektrum und sind daher insbesondere zur Prävention und/oder Behandlung von Erkrankungen geeignet.

Die erfindungsgemäßen Verbindungen der Formel (I) sind auch in Kombination mit einem oder mehreren anderen Wirkstoffen zur Prävention und/oder Behandlung von Erkrankungen geeignet. Geeignete Kombinationswirkstoffe sind insbesondere Plättchenaggregationshemmer, Antikoagulantien, Fibrinolytika, Lipidsenker, Koronartherapeutika und/oder Vasodilatatoren.

Die erfindungsgemäßen Verbindungen der Formel (I) wirken insbesondere als Antikoagulantien und können daher bevorzugt eingesetzt werden in Arzneimitteln zur Prävention und/oder Behandlung von thromboembolischen Erkrankungen. Zu den "thromboembolischen Erkrankungen" im Sinne der vorliegenden Erfindung zählen insbesondere schwerwiegende Erkrankungen wie Herzinfarkt, Angina Pectoris (eingeschlossen instabile Angina), Reokklusionen und Restenosen nach einer Angioplastie oder aortokoronarem Bypass, Hirnschlag, transitorische ischämische Attacken, periphere arterielle Verschlusskrankheiten, Lungenembolien oder tiefe venöse Thrombosen.

Darüber hinaus sind die erfindungsgemäßen Verbindungen der Formel (I) gleichermaßen zur Behandlung der disseminierten intravasalen Gerinnung (DIC) geeignet.

Schließlich kommen die erfindungsgemäßen Verbindungen der Formel (I) ebenso für die Prävention und/oder Behandlung von Atherosklerose und Arthritis in Betracht, darüber hinaus ebenso für die Prävention und/oder Behandlung der Alzheimer'schen Erkrankung und von Krebs.

Weiterhin umfasst die vorliegende Erfindung auch ein Verfahren zur Verhinderung der Blutkoagulation in vitro, insbesondere bei Blutkonserven oder biologischen Proben, die Faktor Xa enthalten, das dadurch gekennzeichnet ist, dass Verbindungen der Formel (I) zugegeben werden.

Die erfindungsgemäßen Verbindungen der Formel (I) wirken insbesondere als selektive Inhibitoren des Blutgerinnungsfaktors Xa und hemmen nicht oder erst bei deutlich höheren Konzentrationen auch andere Serinproteasen wie Thrombin, Plasmin oder Trypsin.

Als "selektiv" werden im Rahmen der vorliegenden Erfindung solche Inhibitoren des Blutgerinnungsfaktors Xa bezeichnet, bei denen die IC₅₀-Werte für die Faktor Xa-Inhibierung gegenüber den IC₅₀-Werten für die Inhibierung anderer Serinproteasen, insbesondere Thrombin, Plasmin und Trypsin, um das 100-fachc vorzugsweise um das 500-fache, insbesondere um das 1.000-fache, kleiner sind, wobei bezüglich der Testmethoden für die Selektivität Bezug genommen wird auf die im folgenden beschriebenen Testmethoden der Beispiele A-1) a.1) und a.2).

Für die Applikation der erfindungsgemäßen Verbindungen kommen alle üblichen Applikationsformen in Betracht. Vorzugsweise erfolgt die Applikation oral, lingual, sublingual, bukkal, rektal, lokal wie beispielsweise bei Implantaten oder Stents, oder parenteral (d.h. unter Umgehung des Intestinaltraktes, also intravenös, intraarteriell, intrakardial, intrakutan, subkutan, transdermal, intraperitoneal oder intramuskulär). Insbesondere geeignet sind die orale und intravenöse Applikation. Ganz besonders bevorzugt ist die orale Applikation, worin ein weiterer Vorteil gegenüber der aus dem Stand der Technik bekannten Therapie von thromboembolischen Erkrankungen liegt.

Die neuen Wirkstoffe der Formel (I) können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,1 bis 95 Gew.-%, bevorzugt in 0,5 bis 90 Gew.-%, insbesondere von 1 bis 85 Gew.-%, der Gesamtmischung vorhanden sein, d.h. in Mengen, die ausreichend sind, um den angegebenen Dosierungsspielraum zu erreichen.

Trotzdem kann es gegebenenfalls erforderlich sein, von den zuvor genannten Mengen abzuweichen, und zwar in Abhängigkeit vom Körpergewicht bzw. von der Art des Applikationsweges, vom individuellen Verhalten gegenüber dem Medikament, von der Art der Formulierung und von dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Im Allgemeinen hat es sich als vorteilhaft erwiesen, bei intravenöser Applikation Mengen von etwa 0,001 bis 10 mg/kg, vorzugsweise etwa 0,01 bis 10 mg/kg, insbesondere etwa 0,1 bis 8 mg/kg Körpergewicht, zur Erzielung wirksamer Ergebnisse zu verabreichen.

Im Allgemeinen hat es sich als vorteilhaft erwiesen, bei oraler Applikation Mengen von etwa 0,01 bis 50 mg/kg, vorzugsweise etwa 0,1 bis 10 mg/kg, insbesondere etwa 0,5 bis 8 mg/kg Körpergewicht, zur Erzielung wirksamer Ergebnisse zu verabreichen.

Trotzdem kann es gegebenenfalls erforderlich sein, von den zuvor genannten Mengen bei intravenöser bzw. oraler Applikation abzuweichen, und zwar in Abhängigkeit vom Körpergewicht bzw. von der Art des Applikationsweges, vom individuellen Verhalten gegenüber dem Medikament, von der Art der Formulierung und von dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese über den Tag zu verteilen, und zwar entweder in mehreren Einzelgaben oder als Dauerinfusion.

Die erfindungsgemäßen Verbindungen der Formel (I) zeichnen sich gegenüber herkömmlichen Präparaten zur Behandlung von thromboembolischen Erkrankungen insbesondere dadurch aus, dass durch die selektive Hemmung des Faktors Xa eine größere therapeutische Breite erreicht wird. Dies bedeutet für den Patienten ein geringeres Blutungsrisiko und für den behandelnden Arzt eine bessere Einstellbarkeit des Patienten. Außerdem erfolgt - durch den Mechanismus bedingt - ein schneller Wirkeintritt. Vor allem aber erlauben die erfindungsgemäßen Verbindungen eine orale Applikationsform, worin ein weiterer Vorteil der Therapie mit den erfindungsgemäßen Verbindungen liegt.

Die vorliegende Erfindung wird an den folgenden Beispielen veranschaulicht.

### A Bewertung der physiologischen Wirksamkeit

### 1. Allgemeine Testmethoden

Die besonders vorteilhaften biologischen Eigenschaften der erfindungsgemäßen Verbindungen können durch folgende Methoden festgestellt werden.

### a) Testbeschreibung (in vitro)

### a.1) Messung der Faktor Xa-Hemmung

Die enzymatische Aktivität von humanem Faktor Xa (FXa) wurde über die Umsetzung eines für den FXa-spezifischen chromogenen Substrats gemessen. Dabei spaltet der Faktor Xa aus dem chromogenen Substrat p-Nitroanilin ab. Die Bestimmungen wurden wie folgt in Mihotiterplatten durchgeführt.

Die Prüfsubstanzen wurden in unterschiedlichen Konzentrationen in DMSO gelöst und für 10 Minuten mit humanem FXa (0,5 nmol/l gelöst in 50 mmol/l Tris-Puffer [C,C,C-Tris(hydroxymethyl)-aminomethan], 150 mmol/l NaCl, 0,1 % BSA (bovine serum albumine), pH = 8,3) bei 25°C inkubiert. Als Kontrolle dient reines DMSO. Anschließend wurde das chromogene Substrat (150 µmol/l Pefachrome® FXa von der Firma Pentapharm) hinzugefügt. Nach 20 Minuten Inkubationsdauer bei 25°C wurde die Extinktion bei 405 nm bestimmt Die Extinktionen der Testansätze mit Prüfsubstanz wurden mit den Kontrollansätzen ohne Prüfsubstanz verglichen und daraus die IC₅₀-Werte berechnet

### a.2) Bestimmung der Selektivität

Zum Nachweis der selektiven FXa-Inhibition wurden die Prüfsubstanzen auf ihre Hemmung anderer humaner Serinproteasen wie Thrombin, Trypsin, Plasmin hin untersucht. Zur Bestimmung der enzymatischen Aktivität von Thrombin (75 mU/ml), Trypsin (500 mU/ml) und Plasmin (3,2 nmol/l) wurden diese Enzyme in Tris-Puffer (100 mmol/l, 20 mmol/l CaCl₂, pH = 8,0) gelöst und für 10 Minuten mit Prüfsubstanz oder Lösungsmittel inkubiert. Anschließend wurde durch Zugabe der entsprechenden spezifischen chromogenen Substrate (Chromozym Thrombin® von der Firma Boehringer Mannheim, Chromozym Trypsin® von der Firma Boehringer Mannheim, Chromozym Plasmin® von der Firma Boehringer Mannheim) die enzymatische Reaktion gestartet und die Extinktion nach 20 Minuten bei 405 nm bestimmt Alle Bestimmungen wurden bei 37°C durchgeführt. Die Extinktionen der Testansätze mit Prüfsubstanz wurden mit den Kontrollproben ohne Prüfsubstanz verglichen und daraus die IC₅₀-Werte berechnet.

### a.3) Bestimmung der antikoagulatorischen Wirkung

Die antikoagulatorische Wirkung der Prüfsubstanzen wurde in vitro in Humanplasma bestimmt. Dazu wurde Humanblut unter Verwendung einer 0,11 molaren Natriumcitrat-Lösung als Vorlage in einem Mischungsverhältnis Natriumcitrat/Blut 1/9 abgenommen. Das Blut wurde unmittelbar nach der Abnahme gut gemischt und 10 Minuten bei ca. 2000 g zentrifugiert. Der Überstand wurde abpipettiert. Die Prothrombinzeit (PT, Synonyme: Thromboplastinzeit, Quick-Test) wurde in Gegenwart variierender Konzentrationen an Prüfsubstanz oder dem entsprechenden Lösungsmittel mit einem handelsüblichen Testkit (Neoplastin® von der Firma Boehringer Mannheim) bestimmt. Die Testverbindungen wurden 10 Minuten bei 37°C mit dem Plasma inkubiert. Anschließend wurde durch Zugabe von Thromboplastin die Gerinnung ausgelöst und der Zeitpunkt des Gerinnungseintritts bestimmt. Es wurde die Konzentration an Prüfsubstanz ermittelt, die eine Verdoppelung der Prothrombinzeit bewirkt.

### b) Bestimmung der antithrombotischen Wirkung (in vivo)

### b.1) Arteriovenöses Shunt-Modell (Ratte)

Nüchterne männliche Ratten (Stamm: HSD CPB:WU) mit einem Gewicht von 200 bis 250 g wurden mit einer Rompun/ Ketavet Lösung narkotisiert (12 mg/kg/50 mg/kg). Die Thrombusbildung wurde in einem artetiovenösen Shunt in Anlehnung an die von Christopher N. Berry et al., Br. J. Pharmacol. (1994), 113, 1209 bis 1214 beschriebene Methode ausgelöst. Dazu wurden die linke Vena jugularis und die rechte Arteria carotis freipräpariert. Ein extracorporaler Shunt wurde mittels eines 10 cm langen Polyethylenschlauchs (PE 60) zwischen den beiden Gefäßen gelegt. Dieser Polyethylenschlauch war in der Mitte in einen weiteren 3 cm langen Polyethylenschlauch (PE 160), der zur Erzeugung einer thrombogenen Oberfläche einen aufgerauhten und zu einer Schlinge gelegten Nylonfaden enthielt, eingebunden. Der extrakorporale Kreislauf wurde 15 Minuten lang aufrechterhalten. Dann wurde der Shunt entfernt und der Nylonfaden mit dem Thrombus sofort gewogen. Das Leergewicht des Nylonfadens war vor Versuchsbeginn ermittelt worden. Die Prüfsubstanzen wurden vor Anlegung des extrakorporalen Kreislaufs entweder intravenös über die Schwanzvene oder oral mittels Schlundsonde wachen Tieren verabreicht.

### b.2) Arterielles Thrombose-Modell (Ratte)

Männliche nüchterne Ratten (Stamm: HSD CPB: WU) wurden wie oben beschrieben narkotisiert. Die Ratten waren im Mittel etwa 200 g schwer. Die linke Arteria carotis wurde freipräpariert (ca. 2 cm). Die Bildung eines arteriellen Thrombus wurde durch eine mechanische Gefäßverletzung in Anlehnung an die von K. Meng et al., Naunyn-Schmiedeberg's Arch. Pharmacol. (1977), 301, 115-119 beschriebene Methode induziert. Dazu wurde die freipräparierte Arteria carotis vom Blutfluss abgeklemmt, für 2 Minuten in einer Metallrinne auf -12°C abgekühlt und zur Standardisierung der Thrombengröße gleichzeitig mit einem Gewicht von 200 g komprimiert. Anschließend wurde der Blutfluss durch einen um die Arteria carotis distal von dem verletzten Gefäßabschnitt gelegten Clip zusätzlich reduziert. Die proximale Klemme wurde entfernt, die Wunde verschlossen und nach 4 Stunden wieder geöffnet, um den verletzten Gefäßabschnitt zu entnehmen. Der Gefäßabschnitt wurde longitudinal geöffnet und der Thrombus von dem verletzten Gefäßabschnitt entfernt. Das Feuchtgewicht der Thromben wurde sofort ermittelt. Die Prüfsubstanzen wurden zu Versuchsbeginn entweder intravenös über die Schwanzvene oder oral mittels Schlundsonde wachen Tieren verabreicht

### b.3) Venöses Thrombose-Modell (Ratte)

Männliche nüchterne Ratten (Stamm: HSD CPB: WU) wurden wie oben beschrieben narkotisiert. Die Ratten waren im Mittel etwa 200 g schwer. Die linke Vena jugularis wurde freipräpariert (ca. 2 cm). Die Bildung eines venösen Thrombus wurde durch eine mechanische Gefäßverletzung in Anlehnung an die von K. Meng et al., Naunyn-Schmiedeberg's Arch. Pharmacol. (1977), 301, 115-119 beschriebene Methode induziert. Dazu wurde die Vena jugularis vom Blutfluss abgeklemmt, für 2 Minuten in einer Metallrinne auf -12°C abgekühlt und zur Standardisierung der Thrombengröße gleichzeitig mit einem Gewicht von 200 g komprimiert. Der Blutfluss wurde wieder eröffnet und die Wunde verschlossen. Nach 4 Stunden wurde die Wunde wieder geöffnet, um die Thromben von den verletzten Gefäßabschnitten zu entfernen. Das Feuchtgewicht der Thromben wurde sofort ermittelt. Die Prüfsubstanzen wurden zu Versuchsbeginn entweder intravenös über die Schwanzvene oder oral mittels Schlundsonde wachen Tieren verabreicht

### B Herstellungbeispiele

In den Beispielen werden folgendeAbkürzungen verwendet:
- DIEA: *= N,N-*Diisopropylethylamin
- DMAP: = 4-*N,N-*Dimethylaminopyridin
- DMF: = Dimethylformamid
- EDCI: = *N'*-(3-Dimethylaminopropyl)-*N*-ethylcarbodiimid x HCl
- EtOH: = Ethanol
- HOBt: = 1-Hydroxy-1H-benzotriazol x H₂O
- rt: = Retentionszeit
- RT: = Raumtemperatur
- TFA: = Trifluoressigsäure
- THF: = Tetrahydrofuran
- Tol: = Toluol

### HPLC-Parameter:

**Methode 1:** Säule: Kromasil C18, L-R Temperatur: 30°C, Fluss = 0.75 mlmin⁻¹, Eluent: A = 0.01 M HClO₄, B = CH₃CN, Gradient: → 0.5 min 98%A → 4.5 min 10%A → 6.5 min 10%A
**Methode 2:** Säule: Kromasil C18 60*2, L-R Temperatur: 30°C, Fluss = 0.75 mlmin⁻¹, Eluent: A = 0.01 M H₃PO₄, B = CH₃CN, Gradient: → 0.5 min 90%A → 4.5 min 10%A → 6.5 min 10%A
**Methode 3:** Säule: Kromasil C18 60*2, L-R Temperatur: 30°C, Fluss = 0.75 mlmin⁻¹, Eluent: A = 0.005 M HClO₄, B = CH₃CN, Gradient: → 0.5 min 98%A → 4.5 min 10%A → 6.5 min 10%A
**Methode 4:** Säule: Symmetry C18 2.1x150 mm, Säulenofen: 50°C, Fluss = 0.6 mlmin⁻¹, Eluent: A = 0.6 g 30%ige HCl/l Wasser, B = CH₃CN, Gradient: 0.0 min 90%A → 4.0 min 10%A → 9 min 10%A
**Methode 5:** Instrument Micromass Quattro LCZ
   Säule Symmetry C18, 50 mm x 2.1 mm, 3.5 µm, Temperatur: 40°C, Fluss = 0.5 mlmin⁻¹, Eluent A = CH₃CN + 0.1% Ameisensäure, Eluent B = Wasser + 0.1% Ameisensäure, Gradient: 0.0 min 10% A → 4 min 90% A → 6 min 90% A
**Methode 6:** Instrument Micromass Platform LCZ
   Säule Symmetry C18, 50 mm x 2.1 mm, 3.5 µm, Temperatur: 40°C, Fluss = 0.5 mlmin⁻¹, Eluent A = CH₃CN + 0.1% Ameisensäure, Eluent B = Wasser + 0.1% Ameisensäure, Gradient: 0.0 min 10% A → 4 min 90% A → 6 min 90% A
**Methode 7:** Instrument Micromass Quattro LCZ
   Säule Symmetry C18, 50 mm x 2.1 mm, 3.5 µm, Temperatur: 40°C, Fluss = 0.5 mlmin⁻¹, Eluent A = CH₃CN + 0.1% Ameisensäure, Eluent B = Wasser + 0.1% Ameisensäure, Gradient: 0.0 min 5% A → 1 min 5% A → 5 min 90% A → 6 min 90% A

### Synthesebeispiele

### Beispiel 1

### 5-Chloro-N-(1,3-dioxo-1,3-dihydro-2-benzofuran-4-yl)-2-thiophencarboxamid

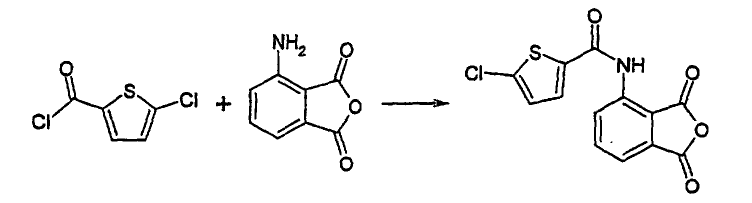

Zur Herstellung des benötigten Säurechlorids werden 27.77 g 2-Chlor-5-thiophencarbonsäure (0.171 mol) in 50 ml Thionylchlorid (0.685 mol) suspendiert und 90 min bei 90°C gerührt (nach 15 min entsteht eine klare Lösung). Die resultierende Lösung wird eingedampft, zweimal mit Toluol azeotropiert, in 60 ml Toluol gelöst und innerhalb von 40 min bei 20°C zu einer Lösung von 17.98 g (110.2 mmol) 3-Aminophthalsäureanhydrid in 200 ml Pyridin getropft. Man rührt über Nacht bei RT, verdampft das Pyridin im Vakuum und verrührt den Rückstand zweimal mit Toluol und dampft wieder ein. Nach Zugabe von 1 1 Essigester und 0.5 1 KH₂PO₄-Puffer (pH=4-5) wird kräftig geschüttelt, abgesaugt, mit Essigester gewaschen, gut abgepresst und im Vakuum über P₂O₅ getrocknet. Die Mutterlauge wird dreimal mit Essigester extrahiert, eingedampft, der Rückstand mit Essigester verrührt und abgesaugt. Man erhält so insgesamt 19.77 g (58.3 % d.Th.) der Zielverbindung mit einem Smp. von 195°C.

### Beispiel 2

### 2-(4-{[(5-Chloro-2-thienyl)carbonyl]amino}-1,3-dioxo-1,3-dihydro-2H-isoindol-2-yl)essigsäure

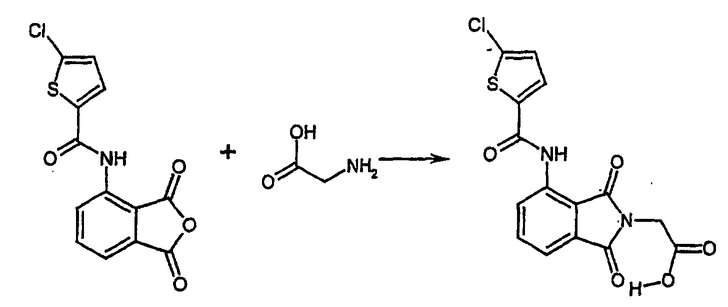

8.7 g (28.27 mmol) 5-Chloro-*N*-(1,3-dioxo-1,3-dihydro-2-benzofuran-4-yl)-2-thiophencarboxamid und 2.12 g (28.2 mmol) Glycin werden über Nacht in 250 ml Dioxan gekocht. Dann gibt man nochmals 6.3 g (83.9 mmol) Glycin hinzu und kocht weitere 22 Stunden. Nach dem Abkühlen wird die Lösung von Glycin abfiltriert und das Filtrat im Vakuum eingedampft. Man erhält 10.2 g (98.9 % d. Th.) der Zielverbindung mit einem Schmelzpunkt von 247 °C.

Analog wurde ausgehend von β-Alanin dargestellt

### Beispiel 3

**3-(4-{[(5-Chloro-2-thienyl)carbonyl]amino}-1,3-dioxo-1,3-dihydro-2*H*-isoindol-2-yl)propansäure** in 99 % Ausbeute mit einem Smp. von 194°C und einem Rf (SiO₂, EtOAc) = 0.63.

### Beispiel 4

**5-Chloro-*N*-(1,3-dioxo-2-{2-oxo-2-[4-(4-pyridinyl)piperazino]ethyl}-2,3-dihydro-1*H*-isoindol-4-yl}-2-thiophencarboxamid**

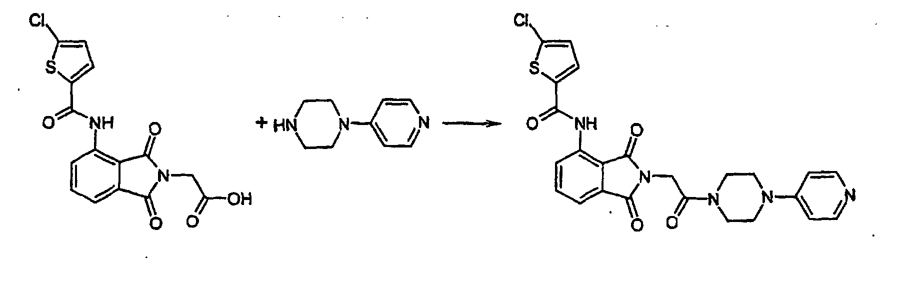

Man löst 0.43 g (1.18 mmol) 2-(4-{[(5-Chloro-2-thienyl)carbonyl]amino}-1,3-dioxo-1,3-dihydro-2*H*-isoindol-2-yl)essigsäure in 15 ml DMF, gibt 0.2 g (1.3 Äquivalente) 1-Hydroxy-1*H*-benzotriazol Hydrat (HOBT) und 0.238 g N'-(3-Dimethylaminopropyl)-N-ethylcarbodiimid (EDCI) (1.3 Äquivalente) hinzu und tropft innerhalb 15 min bei Raumtemperatur 0.305 g (0.411 ml; 2 Äquivalente) Düsopropylethylamin (DIEA) hinzu. Man rührt über Nacht bei Raumtemperatur, wobei die Suspension in eine klare Lösung übergeht Anschließend wird das Reaktionsgemisch mit Wasser versetzt, die wässrige Phase mit Essigester dreimal extrahiert, die vereinigten organischen Phasen getrocknet, im Vakuum eingedampft und der Rückstand dreimal mit Toluol coevaporiert. Der Rückstand wird mit Toluol aufgeschlämmt, filtriert und mit Toluol gewaschen. Nach Trocknung erhält man 0.458 g (76.4 % d. Th.) der Zielverbindung mit einem Smp. von 207°C.

### Analog wurde dargestellt:

### Beispiel 5

**5-Chloro-*N*-(1,3-dioxo-2-{3-oxo-3-[4-(4-pyridinyl)piperazino]propyl}-2,3-dihydro-1*H*-isoindol-4-yl)-2-thiophencarboxamid**

Smp. 167°C; Rf(SiO₂, Toluol/Ethanol =1:1) 0.2.

Analog wurden ferner die in der folgenden Tabelle aufgeführten Verbindungen aus den entsprechenden substituierten Aminderivaten dargestellt, welche wiederum z.B. auf den in WO97/03072, US 4968704 beschriebenen Wegen bzw. durch Umsetzung von Halogenpyridinen oder Halogenpyrimidinen mit Diaminen erhältlich sind.

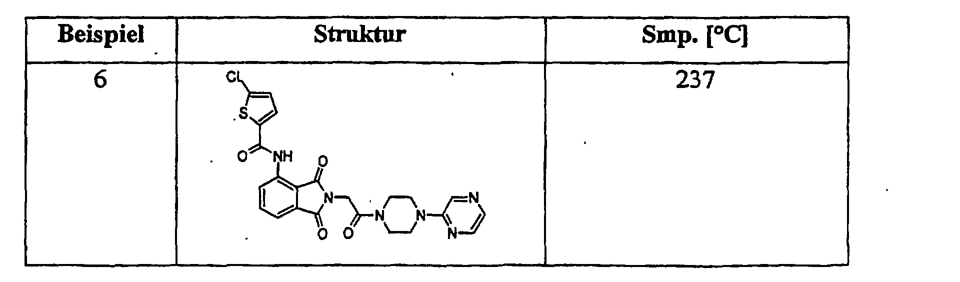

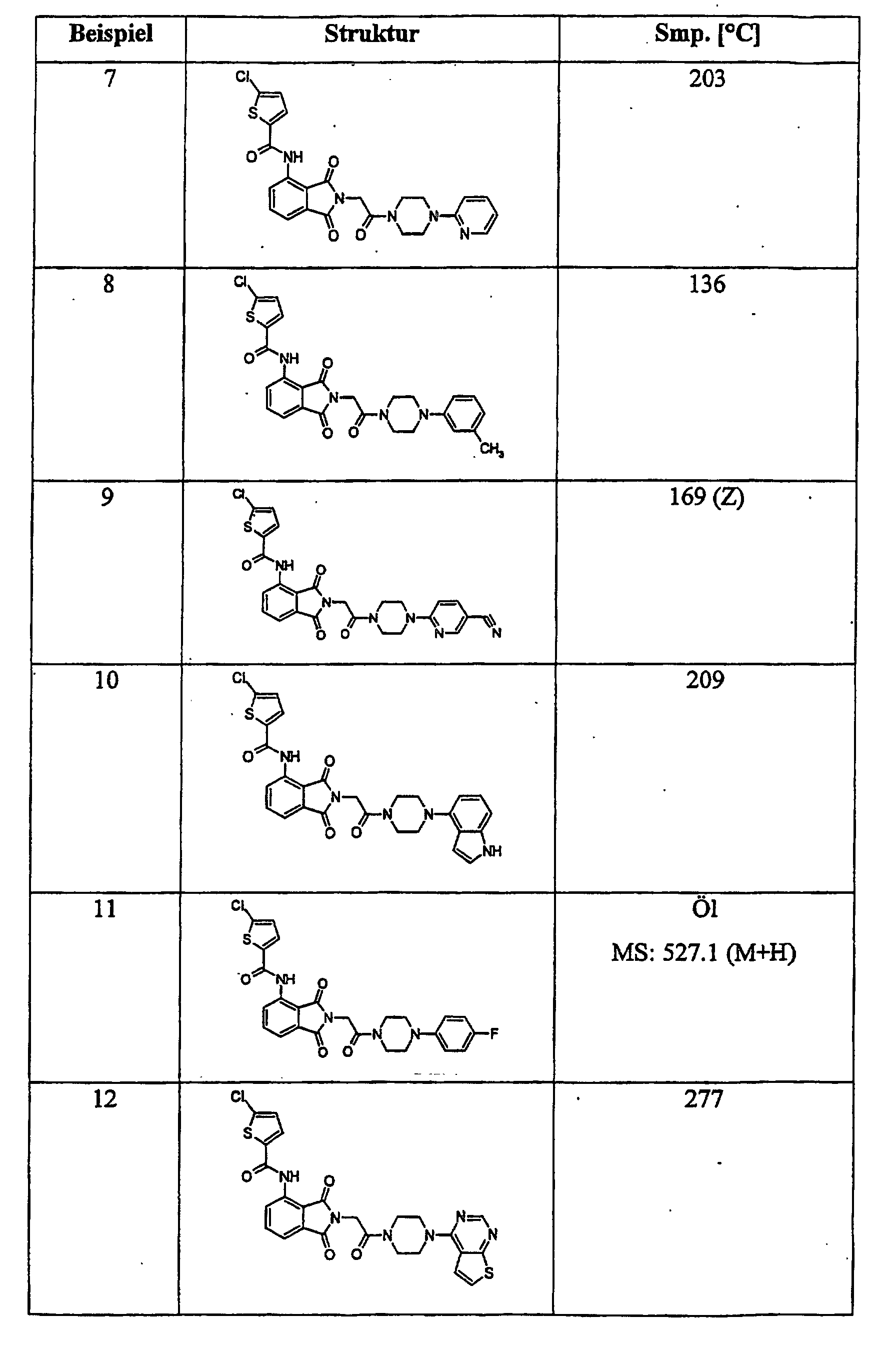

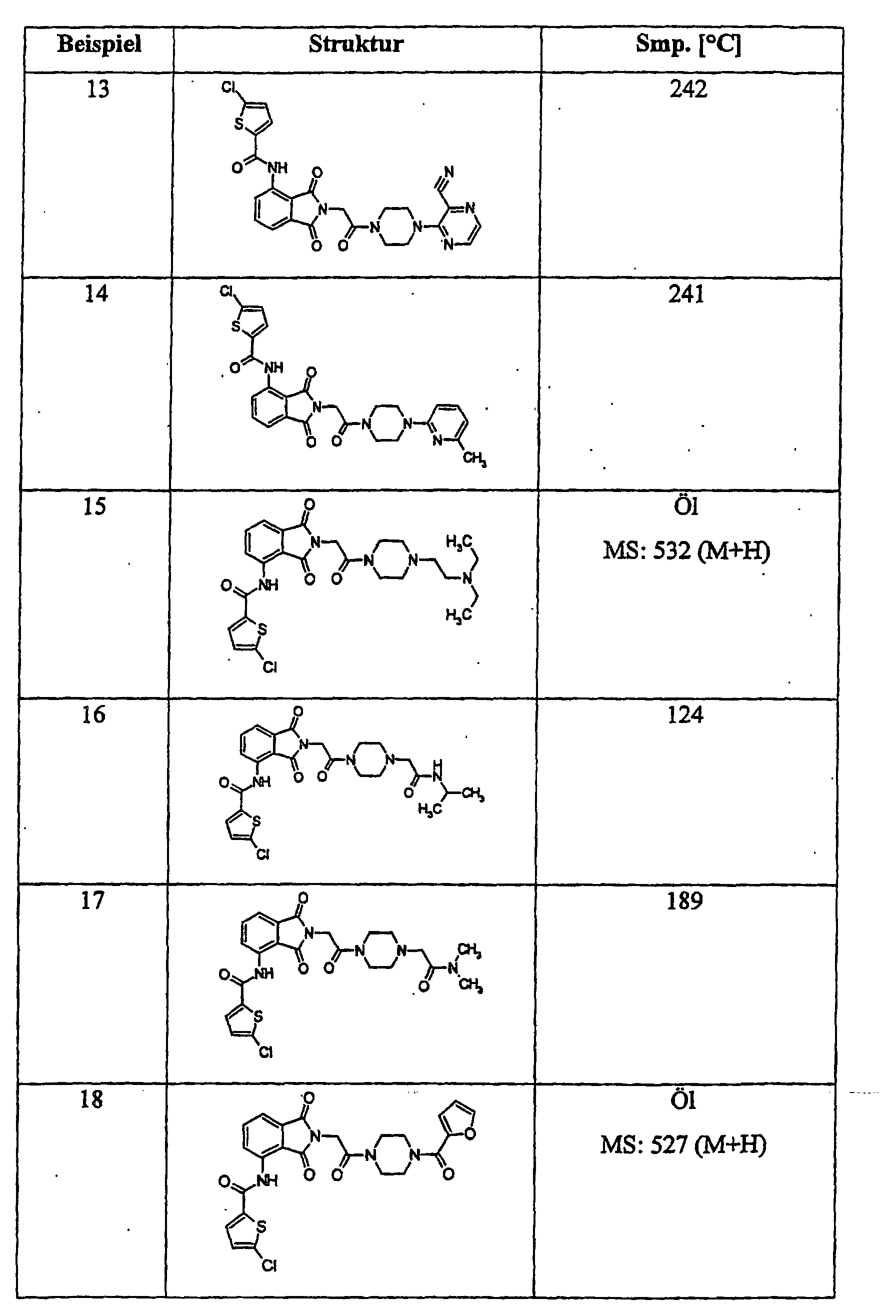

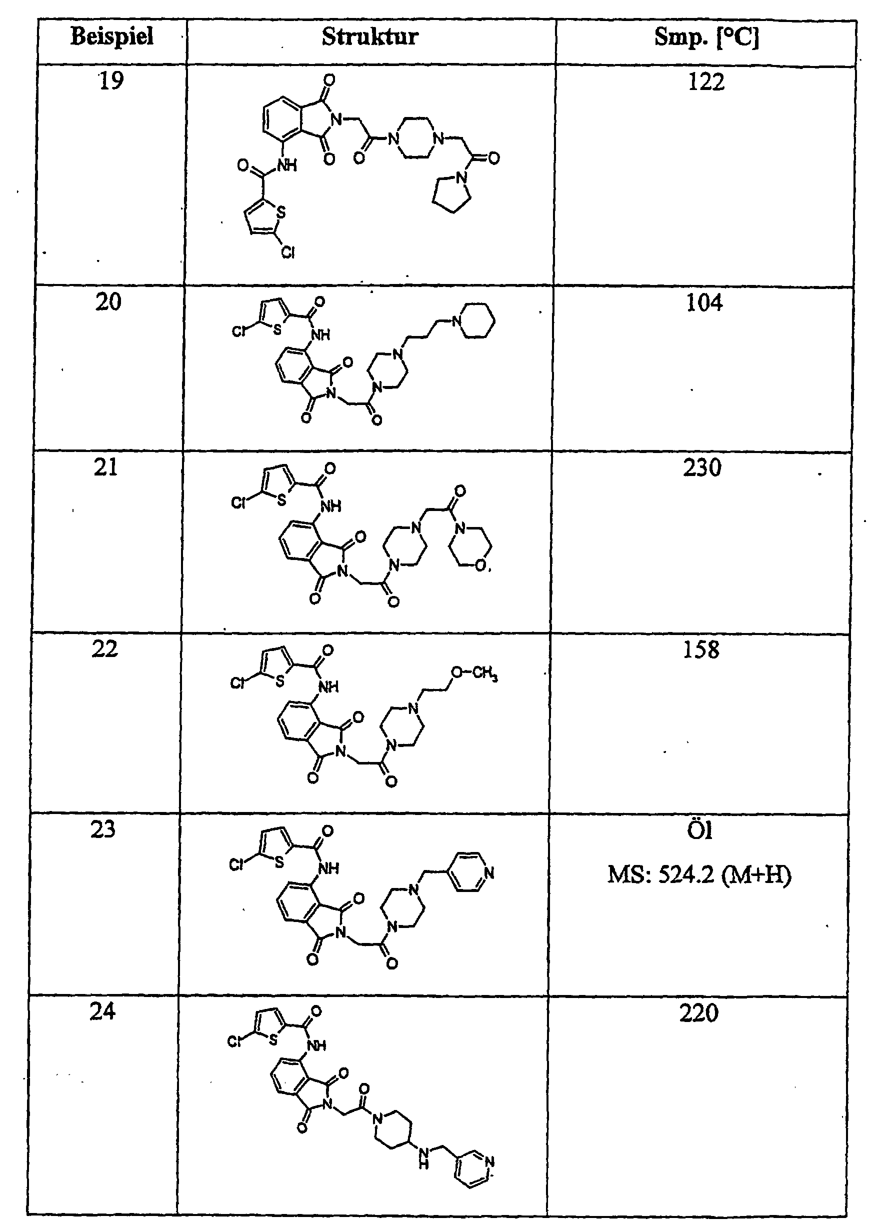

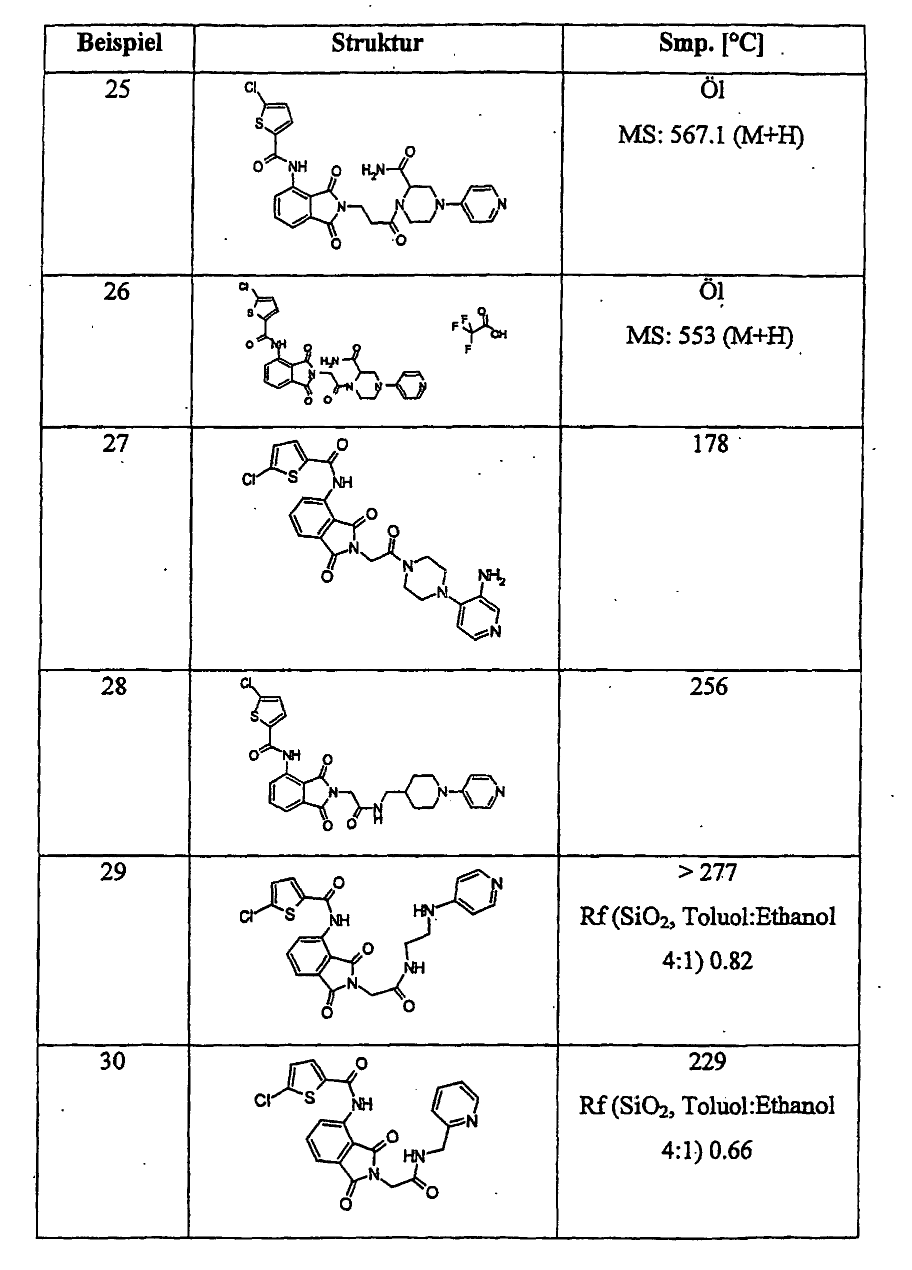

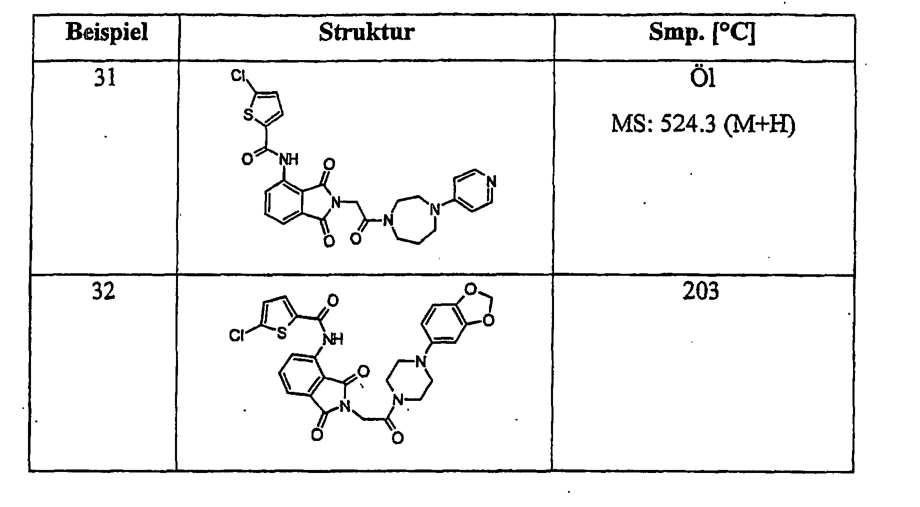

Folgende Ester können ebenfalls auf analoge Weise mittels EDCI, HOBt, DIEA erhalten werden:

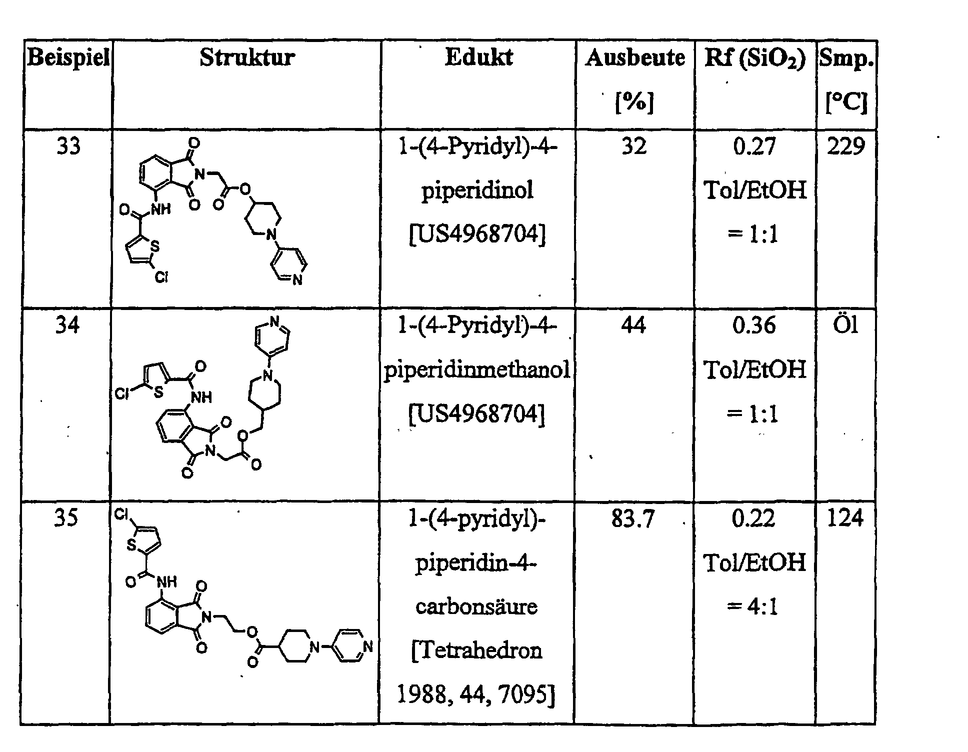

### Beispiel 36

Analog erhält man 5-Chloro-*N*-(2-{2-[4-(hydroxymethyl)piperidino]-2-oxoethyl}-1,3-dioxo-2,3-dihydro-1*H-*isoindol-4-yl)-2-thiophencarboxamid

Smp. 198°C, Rf (SiO₂, Toluol/Essigester = 1:1): 0.79.

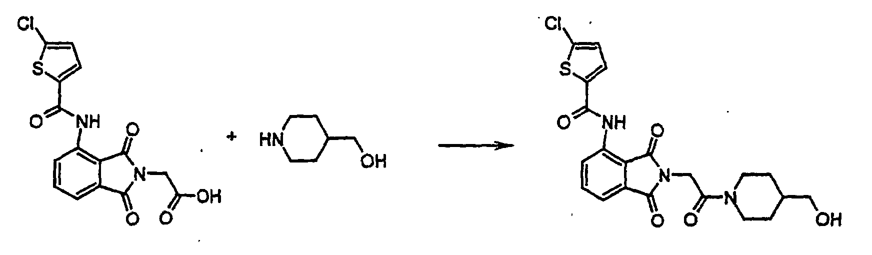

### Beispiel 37

### 2-(4-Amino-1,3-dioxo-1,3-dihydro-2H-isoindol-2-yl)-N-[2-(1H-imidazol-4-yl)ethyl]acetamid

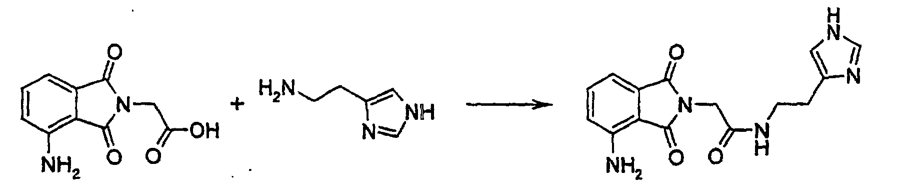

981 mg (4.455 mmol) (4-Amino-1,3-dioxo-1,3-dihydro-2*H*-isoindol-2-yl)essigsäure (Caswell, Atkinson; *J. Org. Chem*.; *29*; **1964;** S. 3151) werden in 50 ml Dichlormethan suspendiert. 902 mg (4.9 mmol) Histamin Dihydrochlorid, 1.025 g (5.35 mmol) N'-(3-Dimethylaminopropyl)-*N*-ethylcarbodiimid Hydrochlorid, 722 mg (5.35 mmol) 1-Hydroxy-1*H*-benzotriazol und 3.1 ml (22.3 mmol) Triethylamin werden hinzugegeben. Das Reaktionsgemisch wird über Nacht bei RT gerührt und anschließend eingeengt. Der Rückstand wird mit Wasser verrührt und filtriert. Der so erhaltene Feststoff wird in Dichlormethan/Methanol gelöst, filtriert, und das Filtrat wird eingeengt, wobei 539 mg (39 % d. Th.) des Produkts erhalten werden.
MS = 314 (M+H), LC (Methode 4): rt = 1.19 min.

### Beispiel 38

### tert.-Butyl-4-(2-{[(4-amino-1,3-dioxo-1,3-dihydro-2H-isoindol-2-yl)acetyl]-amino}ethyl)-1H-imidazol-1-carboxylat

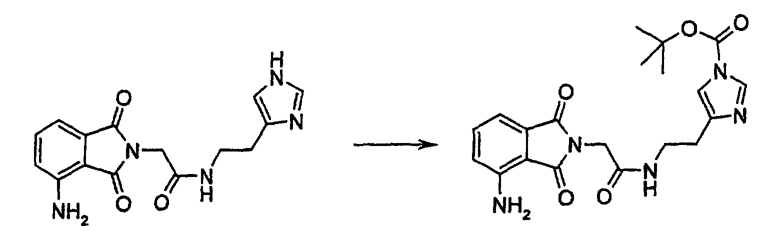

490 mg (1.56 mmol) 2-(4-Amino-1,3-dioxo-1,3-dihydro-2*H*-isoindol-2-yl)-*N*-[2-(1*H*-imidazol-4-yl)ethyl]acetamid werden in 20 ml DMF gelöst und mit 0.65 ml (4.69 mmol) Triethylamin, 19 mg (0.16 mmol) 4-DMAP und 375.5 mg (1.72 mmol) Di-tert-butyl-dicarbonat versetzt. Das Reaktionsgemisch wird über Nacht bei RT gerührt, mit Essigsäureethylester verdünnt und mit gesättigter wässriger NaHCO₃-Lösung gewaschen. Die organische Phase wird über MgSO₄ getrocknet, filtriert und eingeengt. Es werden 642 mg (99 % d. Th.) des Produkts erhalten.
MS = 414 (M+H), 314 (M+H - tert.-BuCO₂).

### Beispiel 39

### tert.-Butyl-4-[(4-amino-1,3-dioxo-1,3-dihydro-2H-isoindol-2-yl)acetyl]-1-piperazincarboxylat

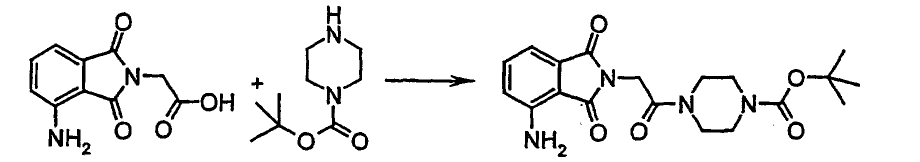

2 g (9.08 mmol) (4-Amino-1,3-dioxo-1,3-dihydro-2*H*-isoindol-2-yl)essigsäure, 1.692 g (9.08 mmol) tert.-Butyl-1-piperazincarboxylat, 1.916 g (10 mmol) N'-(3-Dimethylaminopropyl)-*N*-ethylcarbodiimid Hydrochlorid, 1.35 g (10 mmol) 1-Hydroxy-1*H*-benzotriazol und 3.8 ml (27 mmol) Triethylamin werden in 200 ml Dichlormethan über Nacht bei RT gerührt. Dann wird das Reaktionsgemisch mit ges. wässr.

NaHCO₃-Lsg. gewaschen, über MgSO₄ getrocknet, filtriert und eingeengt. Das Rohprodukt (3.05 g) kann ohne weitere Reinigung in der nächsten Stufe eingesetzt werden oder ggf. zur Reinigung an Kieselgel chromatographiert werden (CH₂Cl₂/EtOH =1/0 bis 2/1 + 0.1 % konz. wässrige Ammoniaklösung).
MS = 389 (M+H), LC (Methode 6): rt = 3.57 min.

### Analog wurden dargestellt:

### Beispiel 40

Aus 3-(4-Amino-1,3-dioxo-1,3-dihydro-2*H*-isoindol-2-yl)propansäure (Caswell, Yang; *J. Chem Eng. Data*; *13*; **1968;** S. 291) und tert.-Butyl-1-piperazincarboxylat:

### tert.-Butyl-4-[3-(4-amino-1,3-dioxo-1,3-dihydro-2H-isoindol-2-yl)propanoyl]-1-piperazincarboxylat

MS = 403 (M+H), LC (Methode 6): rt = 3.58 min.

### Beispiel 41

Aus 3-(4-Amino-1,3-dioxo-1,3-dihydro-2*H*-isoindol-2-yl)propansäure und Morpholin:

### 4-Amino-2-[3-(4-morpholinyl)-3-oxopropyl]-1H-isoindole-1,3(2H)-dion

MS = 304 (M+H), LC (Methode 6): rt = 2.57 min.

### Beispiel 42

### 5-Chloro-N-{1,3-dioxo-2-[2-oxo-2-(1-piperazinyl)ethyl]-2,3-dihydro-1H-isoindol-4-yl)-2-thiophencarboxamid

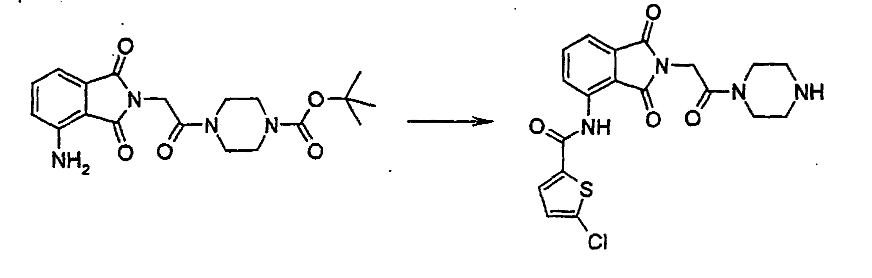

2.29 g (5.26 mmol) tert.-Butyl-4-[(4-amino-1,3-dioxo-1,3-dihydro-2*H*-isoindol-2-yl)acetyl]-1-piperazincarboxylat werden in 20 ml Pyridin gelöst und mit 1.143 g (6.31 mmol) 5-Chlor-2-thiophencarbonsäurechlorid und 67 mg (0.55 mmol) 4-DMAP versetzt. Das Reaktionsgemisch wird für 2 h auf 50°C erwärmt, dann werden weitere 500 µl 5-Chlor-2-thiophencarbonsäurechlorid zugegeben, und es wird für 3 h bei 50°C gerührt. Dann wird das Reaktionsgemisch eingeengt, der Rückstand in Methylenchlorid aufgenommen, mit Wasser und ges. Natriumhydrogencarbonat-Lösung gewaschen, über Magnesiumsulfat getrocknet, filtriert und eingeengt. Das Rohprodukt kann durch Chromatographie an Kieselgel gereinigt werden (CH₂Cl₂/EtOH/konz. wässrige Ammoniaklösung).

Das Produkt der Acylierung wird in 150 ml Methylenchlorid gelöst und bei 0°C langsam mit einem Gemisch von 10 ml Trifluoressigsäure und 40 ml Methylenchlorid versetzt. Das Reaktionsgemisch wird langsam auf Raumtemperatur erwärmt und über Nacht gerührt. Dann wird das Reaktionsgemisch eingeengt, und das Rohprodukt wird an Kieselgel chromatographiert (CH₂Cl₂/EtOH/konz. wässrige Ammoniaklösung = 20/1/0.05). Es werden 1.17 g (51 % d. Th.) des Produkts erhalten.
MS = 433 (M+H), LC (Methode 4): rt = 3.01 min.

Analog wurden durch Umsetzung mit 5-Chlor-2-thiophencarbonsäurechlorid und gegebenenfalls anschliessende Abspaltung der tert.-Butoxycarbonylschutzgruppe folgende Verbindungen erhalten:

### Beispiel 43

Aus tert-Butyl-4-(2-{[(4-amino-1,3-dioxo-1,3-dihydro-2*H*-isoindol-2-yl)acetyl]amino}ethyl)-1*H*-imidazol-1-carboxylat:

### 5-Chloro-N-[2-(2-{[2-(1H-imidazol-4-yl)ethyl]amino}-2-oxoethyl)-1,3-dioxo-2,3-dihydro-1H-isoindol-4-yl]-2-thiophencarboxamid

MS = 458 (M+H), LC (Methode 6): rt = 2.86 min.

### Beispiel 44

Aus tert.-Butyl-4-[3-(4-amino-1,3-dioxo-1,3-dihydro-2*H*-isoindol-2-yl)propanoyl]-1-piperazincarboxylat:

### 5-Chloro-N-{1,3-dioxo-2-[3-oxo-3-(1-piperazinyl)propyl]-2,3-dihydro-1H-isoindol-4-yl}-2-thiophencarboxamid

MS = 447 (M+H), LC (Methode 4): rt = 3.06 min.

### Beispiel 45

Aus 4-Amino-2-[3-(4-morpholinyl)-3-oxopropyl]-1*H*-isoindole-1,3(2*H*)-dion:

### 5-Chloro-N-{2-[3-(4-morpholinyl)-3-ozopropyl]-1,3-dioxo-2,3-dihydro-1H-isoindol-4-yl}-2-thiophencarboxamid

MS = 448 (M+H), RT (Methode 5) = 4.11 min.

### Beispiel 46

### N-{2-[3-(4-Acetyl-1-piperazinyl)-3-oxopropyl]-1,3-dioxo-2,3-dihydro-1H-isoindol-4-yl}-5-chloro-2-thiophencarboxamid

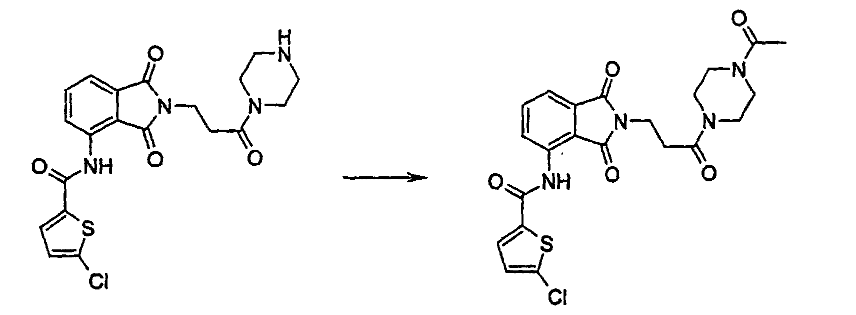

90 mg (0.2 mmol) 5-Chloro-*N*-{1,3-dioxo-2-[3-oxo-3-(1-piperazinyl)propyl]-2,3-dihydro-1*H*-isoindol-4-yl}-2-thiophencarboxamid und 41 mg (0.4 mmol) Triethylamin werden in 10 ml THF gelöst und mit 20.6 mg (0.2 mmol) Essigsäureanhydrid versetzt. Das Reaktionsgemisch wird 5 h bei Raumtemperatur gerührt, mit Methylenchlorid verdünnt, mit Wasser gewaschen, über Magnesiumsulfat getrocknet, filtriert und eingeengt. Es werden 81 mg (82 % d. Th.) des Produkts erhalten. MS = 489 (M+H), RT (Methode 6) = 3.82 min.

### Beispiel 47

### 5-Chloro-N-(1,3-dioxo-2-{2-[4-(4-pyridinyl)piperazino]ethyl}-2,3-dihydro-1H-isoindol-4-yl)-2-thiophencarboxamid

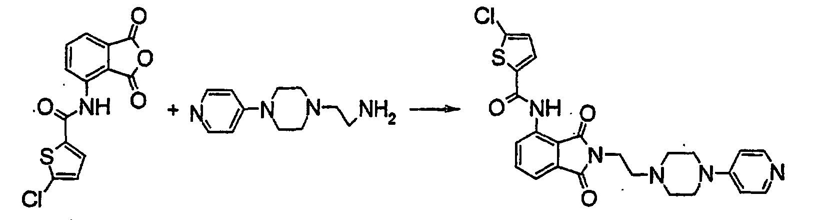

310 mg (1 mmol) 5-Chloro-*N*-(1,3-dioxo-1,3-dihydro-2-benzofuran-4-yl)-2-thiophencarboxamid und 0.64 g (1 mmol) 2-[4-(4-Pyridinyl)piperazino]ethylamin (erhältlich durch Erhitzen von 4-Chlorpyridin mit Aminoethylpiperazin oder gemäß Ger. Off. 2024350) werden über Nacht in 40 ml Eisessig gekocht. Nach Zugabe von 340 mg 5-Chloro-*N*-(1,3-dioxo-1,3-dihydro-2-benzofuran-4-yl)-2-thiophencarboxamid wird nochmals 24 h gekocht, anschließend im Vakuum eingedampft, mit Wasser versetzt, die wässrige Phase viermal mit Essigester extrahiert, die vereinigten organischenPhasen mit Na₂SO₄ getrocknet, mit Kieselgel versetzt und im Vakuum eingedampft. Nach Chromatographie auf Kieselgel mit einem Toluol/Essigester -> Toluol/Ethanol-Gradienten erhält man 140 mg (28.2 % d. Th.) der Zielverbindung mit einem Smp. von 168°C und Rf(SiO₂, Toluol/Ethanol = 1:1) = 0.28.

### Beispiel 48

### 5-Chloro-N-{2-[2-(1-methyl-2-pyrrolidinyl)ethyl}-1,3-dioxo-2,3-dihydro-1H-isoindol-4-yl}-2-thiophencarboxamid

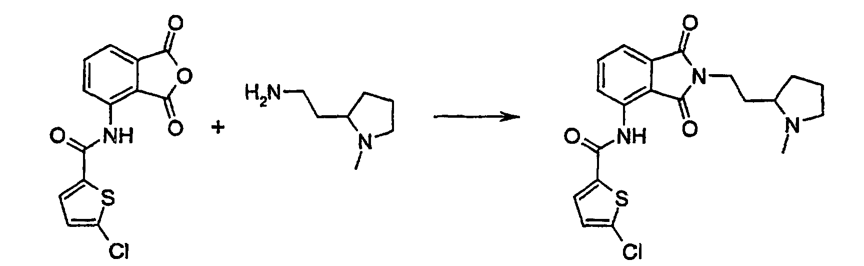

50 mg (0.16 mmol) 5-Chloro-*N*-(1,3-dioxo-1,3-dihydro-2-benzofuran-4-yl)-2-thiophencarboxamid und 20.8 mg (0.16 mmol) 2-(1-Methyl-2-pyrrolidinyl)ethylamin werden in 2 ml Dioxan gelöst und 24 h zum Rückfluss erhitzt. Entfernen des Lösemittels im Vakuum ergibt 65 mg (97 % d. Th.) des gewünschten Produkts.
MS = 418 (M+H); rt (Methode 5) = 2.36 min.
Alternativ kann die Reaktion auch in Essigsäure als Lösemittel bei Rückflusstemperatur durchgeführt werden. Das Produkt kann gegebenenfalls durch Chromatographie an Kieselgel (CH₂Cl₂/EtOH/konz. wässr. NH₃-Lsg. Gemische) gereinigt werden.

Analog wurden durch Umsetzung des Anhydrids mit den entsprechenden primären Aminen folgende Verbindungen erhalten (2-[4-(2-Pyrimidinyl)-1-piperazinyl]-propylamin und 2-[4-(4-Pyridinyl)-1-piperazinyl]propylamin können analog zu He, Woodruff, Brodbeck; *Bioorg. Med Chem. Lett.* **1997;** 7; *18*; S. 2399-2402 dargestellt werden):

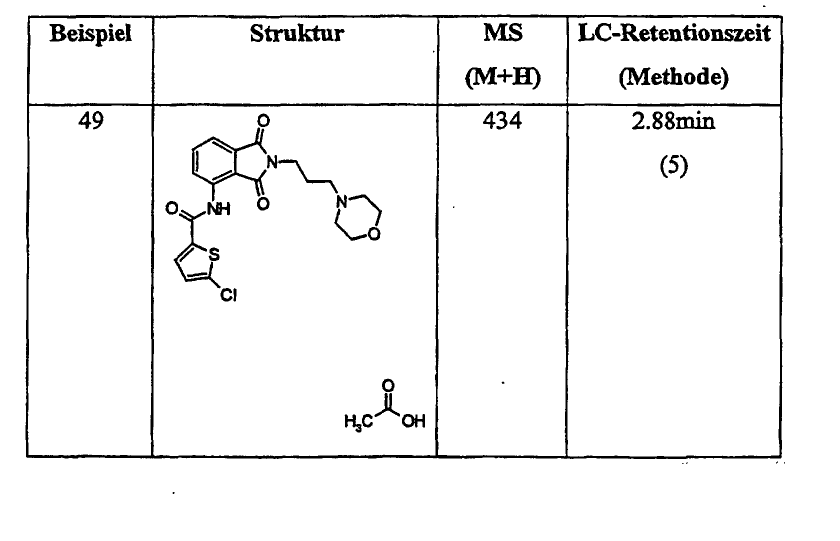

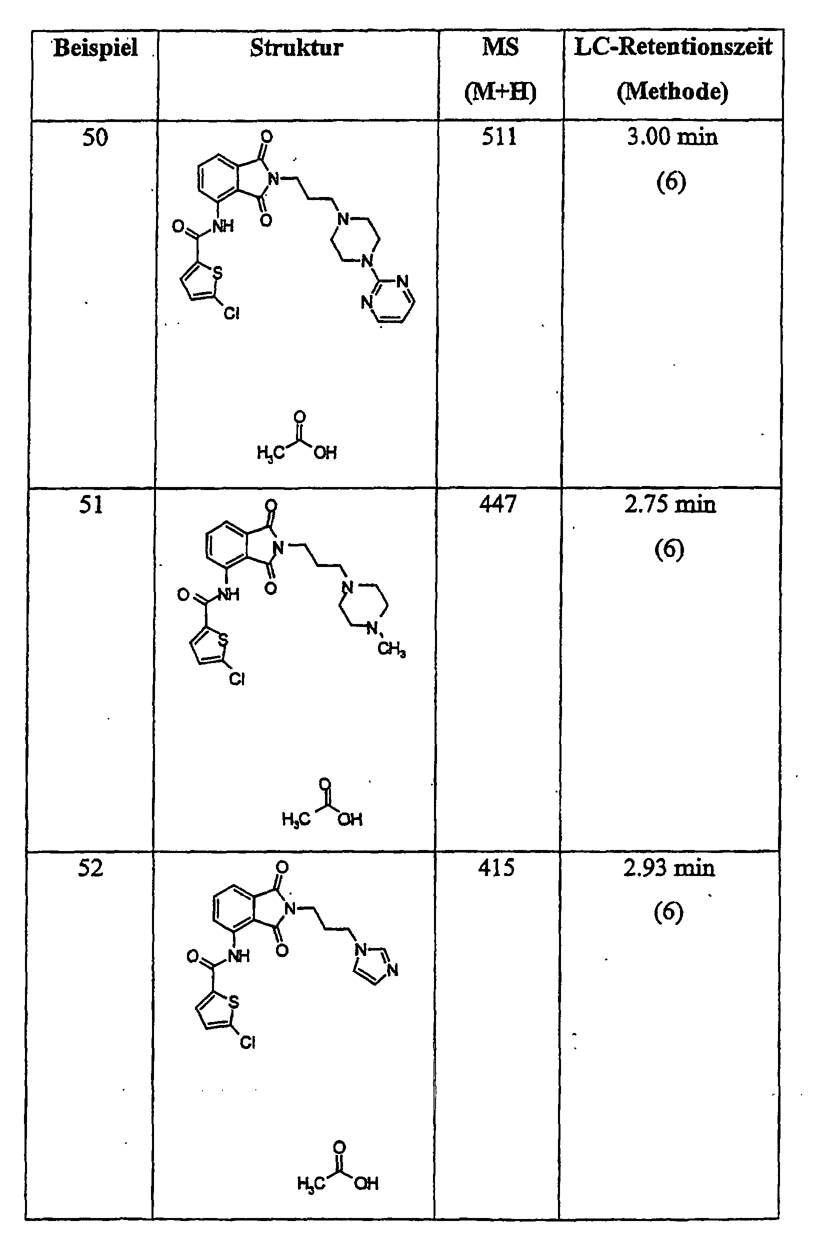

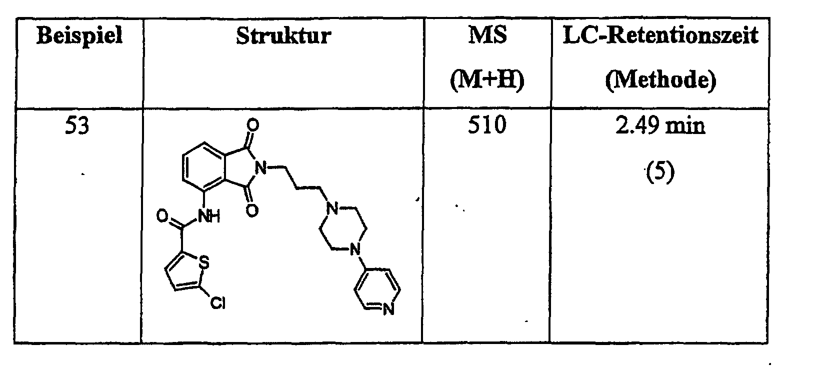

analog wurden erhalten:

### Beispiel 54

### tert.-Butyl-4-(4-{[(5-chloro-2-thienyl)carbonyl]amino}-1,3-dioxo-1,3-dihydro-2H-isoindol-2-yl)butylcarbamat

MS = 478 (M+H), LC (Methode 4): rt = 5.74 min.

### Beispiel 55

### tert.-Butyl-6-(4-{[(5-chloro-2-thienyl)carbonyl]amino}-1,3-dioxo-1,3-dihydro-2H-isoindol-2-yl)hexylcarbamat

MS = 506 (M+H), LC (Methode 4): rt = 6.32 min.

### Beispiel 56

### N-[2-(4-Aminobutyl)-1,3-dioxo-2,3-dihydro-1H-isoindol-4-yl]-5-chloro-2-thiophencarboxamid Trifluoroacetat

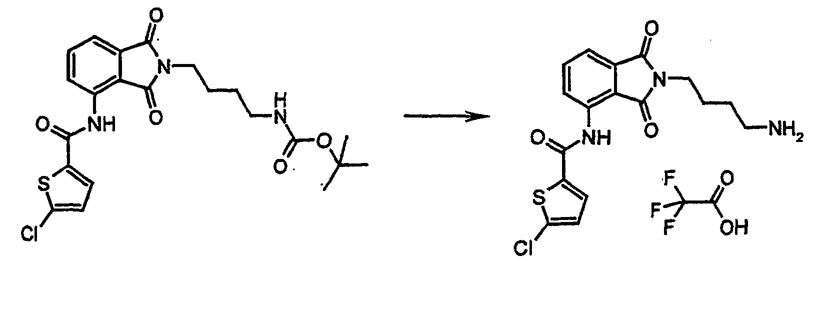

3.53 g (7.39 mmol) tert.-Butyl-4-(4-{[(5-chloro-2-thienyl)carbonyl]amino}-1,3-dioxo-1,3-dihydro-2*H*-isoindol-2-yl)butylcarbamat werden in 150 ml Dichlormethan gelöst und bei 0°C mit einer Lösung von 10 ml Trifluoressigsäure in 50 ml Dichlormethan versetzt. Das Reaktionsgemisch wird langsam auf RT erwärmt und über Nacht gerührt. Dann wird das Reaktionsgemisch eingeengt und der ölige Rückstand über Nacht bei 5°C gelagert. Der nun teilweise kristalline Rückstand wird mit Dichlormethan und wenig Ethanol versetzt und filtriert. Es werden 3.44 g (95 % d. Th.) des gewünschten Produkts als Feststoff erhalten.
MS = 378 (M+H), LC (Methode 4): rt = 3.01 min.

### Beispiel 57

Analog wurde durch Umsetzung von tert.-Butyl-6-(4-{[(5-chloro-2-thienyl)-carbonyl]amino}-1,3-dioxo-1,3-dihydro-2*H*-isoindol-2-yl)hexylcarbamat mit Trifluoressigsäure ***N*-[2-(6-Aminohexyl)-1,3-dioxo-2,3-dihydro-1*H*-isoindol-4-yl]-5-chloro-2-thiophencarboxamid Trifluoroacetat** erhalten.
MS = 406 (M+H), LC (Methode 4): rt = 3.23 min.

### Beispiel 58

### N-[4-(4-{[(5-Chloro-2-thienyl)carbonyl]amino}-1,3-dioxo-1,3-dihydro-2H-isoindol-2-yl)butyl]isonicotinamid

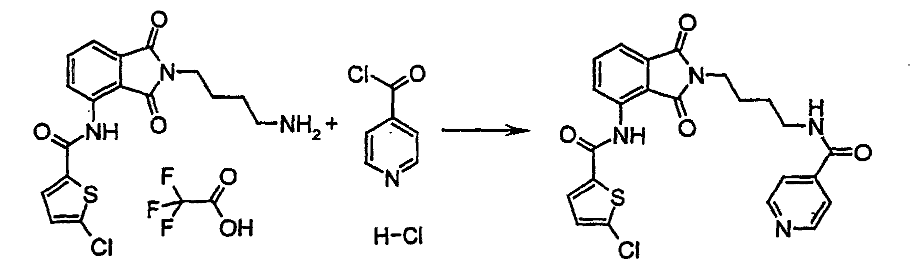

40 mg (0.08 mmol) *N*-[2-(4-Aminobutyl)-1,3-dioxo-2,3-dihydro-1*H*-isoindol-4-yl]-5-chloro-2-thiophencarboxamid Trifluoroacetat werden in 2 ml THF mit 25 mg (0.24 mmol) Triethylamin und einer katalytischen Menge 4-Dimethylaminopyridin versetzt. Dann werden bei 0°C 17.4 mg (0.10 mmol) Isonicotinsäurechlorid Hydrochlorid zugegeben. Das Reaktionsgemisch wird auf RT erwärmt, 3 h bei RT gerührt und dann für 20 h zum Rückfluss erhitzt. Nach Abkühlen auf RT wird das Reaktionsgemisch mit Dichlormethan verdünnt und mit wässr. ges. NaHCO₃-Lsg. gewaschen. Die organische Phase wird über MgSO₄ getrocknet, filtriert und eingeengt. Das Rohprodukt wird über Kieselgel chromatographiert (CH₂Cl₂/EtOH = 50/1). Es werden 18.5 mg (47 % d. Th.) des gewünschten Produkts erhalten.
MS = 483 (M+H), LC (Methode 4): rt = 3.75 min.

### Beispiel 59

### tert-Butyl 4-hydroxytetrahydro-1(2H)-pyridincarboxylat

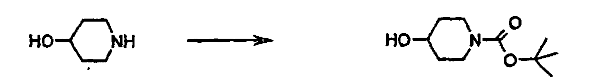

Zu einer eisgekühlten Lösung von 5 g (49.4 mmol) 4-Hydroxypiperidin in 50 ml 1,4-Dioxan werden 12.11 g (54.4 mmol) Di-*tert*.-butyl-dicarbonat (Boc-Anhydrid) und 54.3 ml 1N Natriumhydroxid-Lösung (über 20 min) gegeben. Nach 15 min wird die Eiskühlung entfernt und die Mischung 2 h bei Raumtemperatur gerührt. Es wird eingeengt und die auf pH ca. 6 eingestellte wässrige Phase mit Dichlormethan extrahiert. Die organische Phase wird über Magnesiumsulfat getrocknet, eingeengt und am Hochvakuum getrocknet Ausbeute: 10.12 g (99 % der Theorie); MS (DCI, NH₄): m/z (%) = 202 (M+H, 82), 102 (100); LC-MS (Methode 4): rt (%) = 3.11 (100).

### Beispiel 60

Auf analoge Weise lässt sich ausgehend von 4-Hydroxyethylpiperidin ***tert*-Butyl-4-(2-hydroxyethyl)tetrahydro-1(2*H*)-pyridincarboxylat darstellen.**
MS (DCI, NH₄): 230 (M+H, 70), 130 (100); HPLC (Methode 2): rt (%) = 3.72 (88).

### Beispiel 61

### 5-Chlor-N-(1,3-dioxo-2,3-dihydro-1H-isoindol-4-yl)-2-thiophencarboxamid

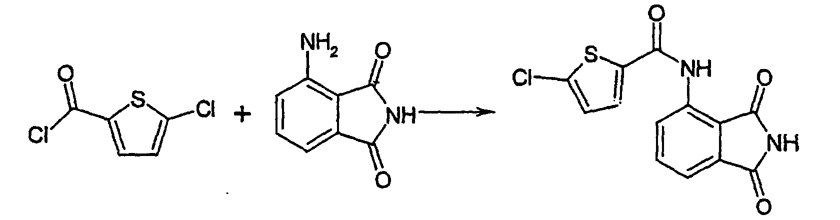

Man löst 7.8 g (43.7 mmol) 3-Aminophthalsäureimid bei 0°C unter Rühren in 200 ml Pyridin. Anschließend gibt man innerhalb von 5 min 5-Chlor-2-thiophencarbonsäurechlorid (erhalten durch Kochen von 5-Chlor-2-thiophencarbonsäure in SOCl₂) hinzu. Man rührt bei RT unter DC-Kontrolle nach, wobei aus der zunächst klaren Lösung ein Brei wird. Man gibt nochmals 200 ml THF hinzu und rührt über Nacht bei RT.

Anschließend wird der Ansatz in 500 ml Wasser gegeben und mit Essigester extrahiert. In der wässrigen Phase verbleiben noch größere Mengen an schwerlöslichen Kristallen, die abgesaugt werden. Man löst die Kristalle in 200 ml THF und trocknet mit MgSO₄. Die einrotierte Lösung wird nochmals mit Ether verrieben und abgesaugt. Es werden 3.5 g (26.4 % d. Th.) der gewünschten Verbindung erhalten.

### Beispiel 62

### 5-Chloro-N-(1,3-dioxo-2-{[1-(4-pyridinyl)-4-piperidinyl]methyl}-2,3-dihydro-1H-isoindol-4-yl)-2-thiophencarboxamid

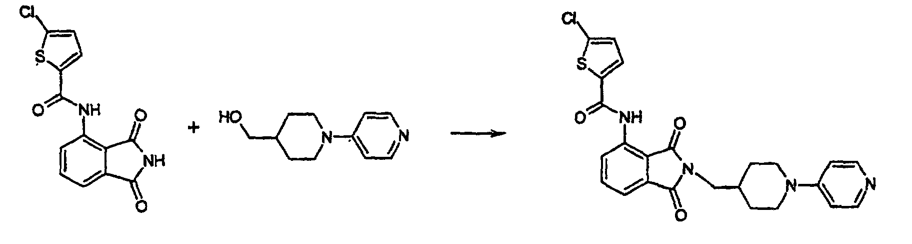

1-(4-Pyridyl)-4-piperidinmethanol (US 4968704) (211.5 mg, 1.1 mmol), 5-Chloro-*N*-(1,3-dioxo-2,3-dihydro-1*H*-isoindol-4-yl)-2-thiophencarboxamid (306.7 mg, 1 mmol), Triphenylphosphin (1049 mg, 4 mmol) werden in 40 ml THF vorgelegt und mit Diethylazodicarboxylat (DEAD) (696.6 mg, 4 mmol, 0.63 ml) versetzt Man rührt über Nacht bei Raumtemperatur, dampft den Ansatz ein, löst den Rückstand in Ethanol, versetzt mit Kieselgel, dampft nochmals ein und trägt den Rückstand auf eine Kieselgelsäule auf. Nach Elution mit Toluol/Essigester und Toluol/Ethanol (25:1)-Gemischen erhält man eine Hauptfraktion mit Rf(SiO₂, Toluol/Ethanol = 4:1) = 0.19. Sie wird eingedampft, mit Ethanol behandelt, und die entstandenen Kristalle (112 mg, 23.3 % d. Th.) werden abgesaugt. Smp.: 123 °C (Zers.).
Analog erhält man:

### Beispiel 63

### 5-Chloro-N-{1,3-dioxo-2-[1-(4-pyridinyl)-4-piperidinyl)-2,3-dihydro-1H-isoindol-4-yl}-2-thiophencarboxamid

Smp. 214°C, Rf(SiO₂, Toluol-Ethanol 1:1): 0.4.

### Beispiel 64

### 5-Chloro-N-[1,3-dioxo-2-(4-piperidinylmethyl)-2,3-dihydro-1H-isoindol-4-yl]-2-thiophencarboxamid

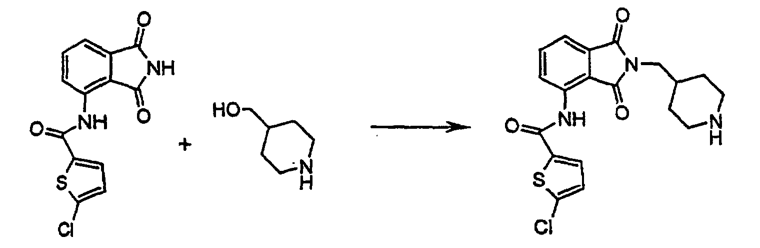

Zu einer Suspension von 670 mg (2.18 mmol) 5-Chloro-*N*-(1,3-dioxo-2,3-dihydro-1*H*-isoindol-4-yl)-2-thiophencarboxamid, 314 mg (2.73 mmol) 4-Hydroxymethylpiperidin und 716 mg (2.73 mmol) Triphenylphosphin in 8.7 ml absolutem THF werden bei Raumtemperatur tropfenweise 0.43 ml (2.73 mmol) Diethylazodicarboxylat (DEAD) gegeben. Nach 2 h bei Raumtemperatur wird die Mischung eingeengt, der Rückstand durch Chromatographie an Silicagel (Gradient: von Dichlormethan-Methanol 95:5 zu Dichlormethan-Methanol-Triethylamin 9:1:0.1) getrennt. Ausbeute: 430 mg (41.5 % der Theorie); MS (ESI): m/z (%) = 404 (M+H, 60),145 (100); LC-MS (Methode 4): rt (%) = 3.02 (84).

### Beispiel 65

### tert-Butyl-4-(4-{[(5-chloro-2-thienyl)carbonyl]amino}-1,3-dioxo-1,3-dihydro-2H-isoindol-2-yl)tetrahydro-1(2H)-pyridincarboxylat

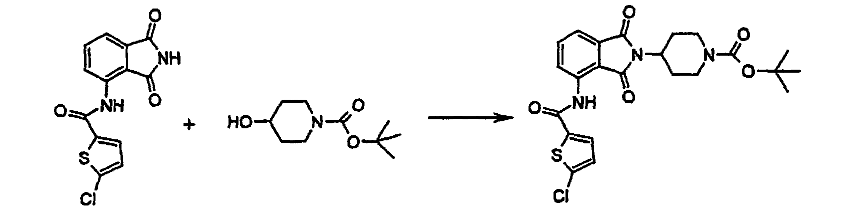

Zu einer Suspension von 150 mg (0.49 mmol) 5-Chloro-*N*-(1,3-dioxo-2,3-dihydro-1*H*-isoindol-4-yl)-2-thiophencarboxamid, 148 mg (0.73 mmol) *tert*-Butyl-4-hydroxytetrahydro-1(2*H*)-pyridincarboxylat und 199 mg (0.76 mmol) Triphenylphosphin in 1.95 ml absolutem THF werden tropfenweise bei Raumtemperatur 0.12 ml (0.76 mmol) Diethylazodicarboxylat (DEAD) gegeben. Nach 2 h bei Raumtemperatur wird die Mischung eingeengt und der Rückstand durch Chromatographie an Silicagel (Dichlormethan/Methanol-Gemisch) getrennt und das Produkt isoliert. Ausbeute 184 mg (76.8 % der Theorie); MS (LC-MS): m/z (%) = 279 (M+H, 100); LC-MS (Methode 4): rt (%) = 3.88 (98.9).

### Beispiel 66

### 5-Chloro-N-[1,3-dioxo-2-(4-piperidinyl)-2,3-dihydro-1H-isoindol-4-yl]-2-thiophencarboxamid

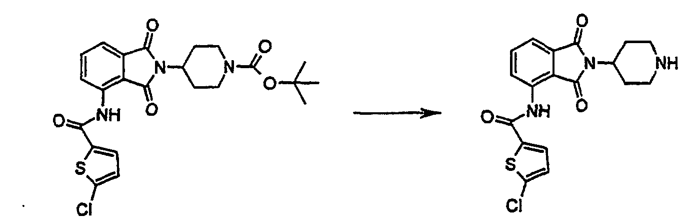

Zu einer eisgekühlten Mischung von 1.82 g (3.71 mmol) *tert*-Butyl-4-(4-{[(5-chloro-2-thienyl)carbonyl]amino}-1,3-dioxo-1,3-dihydro-2*H*-isoindol-2-yl)tetrahydro-1(2*H*)-pyridincarboxylat in 50 ml Chloroform werden 50 ml einer Mischung von TFA und Wasser (9:1) getropft. Die Eiskühlung wird entfernt, und die Mischung wird 1.5 h bei Raumtemperatur gerührt, bevor eingeengt wird. Der Rückstand wird in Dichlormethan/Methanol aufgenommen und mit ges. Natriumhydrogencarbonat-Lösung gewaschen, über Magnesiumsulfat getrocknet und konzentriert. Das Rohprodukt kann durch Kristallisation mit Ether weiter aufgereinigt werden. Ausbeute: 1.185 g (78 % der Theorie); MS (DCI, NH₄): m/z (%) = 390 (M+H, 100).

### Beispiel 67

Auf analoge Weise wurde **5-Chloro-*N*-{1,3-dioxo-2-[2-(4-piperidinyl)ethyl]-2,3-dihydro-1*H*-isoindol-4-yl}-2-thiophencarboxamid** ausgehend von *tert*-Butyl-4-[2-(4-{[(5-chloro-2-thienyl)carbonyl]amino}-1,3-dioxo-1,3-dihydro-2*H*-isoindol-2-yl)ethyl]tetrahydro-1(2*H*})-pyridincarboxylat erhalten.
MS (ESI): m/z (%) = 418 (M+H, 100); HPLC (Methode 1): rt (%) = 3.82 (78).

### Beispiel 68

### 5-Chloro-N-{2-[1-(cyanomethyl)-4-piperidinyl]-1,3-dioxo-2,3-dihydro-1H-isoindol-4-yl}-2-thiophencarboxamid

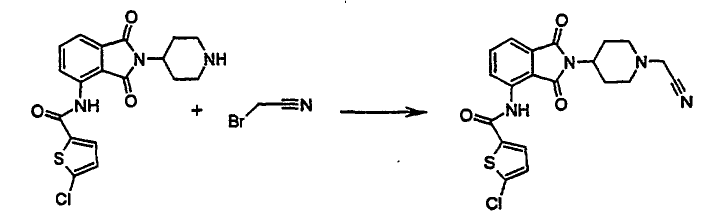

Eine Mischung aus 100 mg (0.26 mmol) 5-Chloro-*N*-[1,3-dioxo-2-(4-piperidinyl)-2,3-dihydro-1*H*-isoindol-4-yl]-2-thiophencarboxamid, 0.022 ml (0.31 mmol) α-Bromacetonitril und 0.072 ml (0.51 mmol) Triethylamin werden in 2.5 ml DMF bei Raumtemperatur über Nacht gerührt. Es wird eingeengt, der Rückstand mit Dichlormethan aufgenommen, mit Natriumhydrogencarbonat-Lösung gewaschen, über Magnesiumsulfat getrocknet und konzentriert. Das Produkt wird mit Ether kristallisiert, abgesaugt und am Hochvakuum getrocknet Alternativ kann eine Reinigung durch Chromatographie an Silicagel (Dichlormethan/Methanol-Gemische) erfolgen.
Ausbeute: 92.2 mg (83.8 % der Theorie); MS (DCI, NH₄): m/z (%) = 429 (M+H, 100); HPLC (Methode 1): rt (%) = 4.51 (100).

Auf analoge Weise können ausgehend von den entsprechenden Amin-Derivaten und α-Bromacetonitril folgende Verbindungen dargestellt werden:

### Beispiel 69

### 5-Chloro-N-(2-{[1-(cyanomethyl)-4-piperidinyl]methyl}-1,3-dioxo-2,3-dihydro-1H-isoindol-4-yl)-2-thiophencarboxamid

MS (ESI): m/z (%) = 443 (M+H, 12), 416 (100); HPLC (Methode 1): rt (%) = 4.41 (97).

### Beispiel 70

### 5-Chloro-N-(2-{2-[1-(cyanomethyl)-4-piperidinyl]ethyl}-1,3-dioxo-2,3-dihydro-1H-isoindol-4-yl)-2-thiophencarboxamid

MS (DCI, NH₄): m/z (%) = 457 (M+H, 100); HPLC (Methode 1): rt (%) = 4.49 (95).

### Beispiel 71

### 4-[2-(4-{[(5-Chloro-2-thienyl)carbonyl]amino}-1,3-dioxo-1,3-dihydro-2H-isoindol-2-yl)ethyl]-1,1-dimethylpiperidiniumjodid

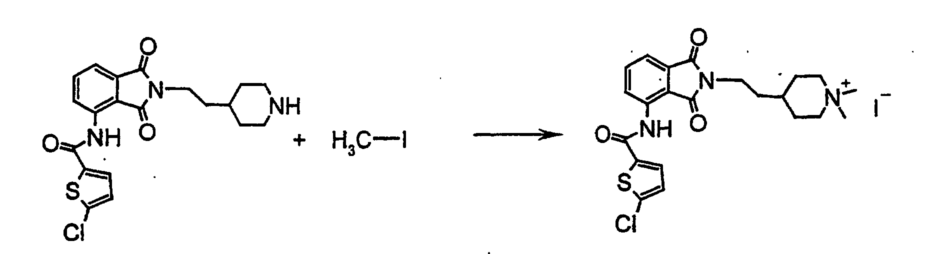

50 mg (0.12 mmol) 5-Chloro-*N*-{1,3-dioxo-2-[2-(4-piperidinyl)ethyl]-2,3-dihydro-1*H*-isoindol-4-yl}-2-thiophencarboxamid werden in 2 ml 1,2-Dichlorethan gelöst, mit 0.025 ml *N*,*N-*Diisopropylethylamin versetzt und mit einem Überschuss Methyljodid behandelt. Die Mischung wird über Nacht bei Raumtemperatur gerührt. Der Rückstand wird mit Wasser und Dichlormethan versetzt. Der unlösliche Feststoff wird abfiltriert und am Hochvakuum getrocknet.
Ausbeute: 51.5 mg (74.8 % der Theorie); LC-MS (Methode 1): rt (%) = 3.02 (84), m/z (%) = 446 (M⁺-I, 100).

### Beispiel 72

### 5-Chloro-N-{2-[2-(1-ethyl-4-piperidinyl)ethyl]-1,3-dioxo-2,3-dihydro-1H-isoindol-4-yl}-2-thiophencarboxamid

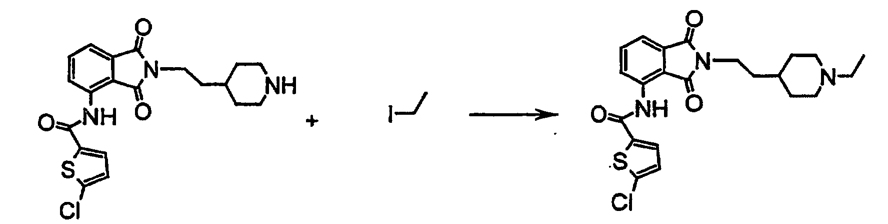

50.1 mg (0.12 mmol) 5-Chloro-*N*-{1,3-dioxo-2-[2-(4-piperidinyl)ethyl]-2,3-dihydro-1*H*-isoindol-4-yl}-2-thiophencarboxamid werden in 1 ml 1,4-Dioxan gelöst und mit 25 µl (0.144 mmol) *N*,*N*-Diisopropylethylamin und 10 µl (0.12 mmol) Jodethan versetzt. Die Mischung wird über Nacht bei Raumtemperatur gerührt und nach Einengen an Silicagel chromatographiert (Dichlormethan/Methanol 97:3).
Ausbeute: 5.6 mg (10.5 % der Theorie); LC-MS (Methode 1): rt (%) = 3.09 (95); m/z (%) = 446 (M+H, 100).

### Beispiel 73

### N-{2-[2-(1-Acetyl-4-piperidinyl)ethyl]-1,3-dioxo-2,3-dihydro-1H-isoindol-4-yl}-5-chloro-2-thiophencarboxamid

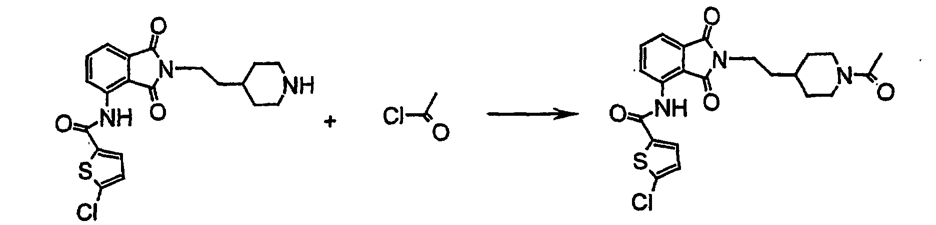

Zu einer Lösung von 50 mg (0.12 mmol) 5-Chloro-*N*-{1,3-dioxo-2-[2-(4-piperidinyl)ethyl]-2,3-dihydro-1*H*-isoindol-4-yl}-2-thiophencarboxamid und 0.1 ml Pyridin in 1 ml absolutem Dichlormethan werden bei Raumtemperatur 17 µl (0.24 mmol) Acetylchlorid getropft. Nach 1 h bei Raumtemperatur wird die Mischung mit einigen Tropfen Wasser versetzt und mit Dichlormethan verdünnt. Es wird mit gesättigter Natriumhydrogencarbonat-Lösung gewaschen, über Magnesiumsulfat getrocknet und eingeengt. Das Rohprodukt wird durch Chromatographie an Silicagel (Dichlormethan/Methanol 98:2) gereinigt.
Ausbeute: 41.8 mg (76 % der Theorie); MS (ESI): m/z (%) = 482 (M+Na, 18), 460 (M+H, 45); HPLC (Methode 1): rt (%) = 4.92 (94).

### Beispiel 74

### 4-[2-(4-{[(5-Chloro-2-thienyl)carbonyl]amino}-1,3-dioxo-1,3-dihydro-2H-isoindol-2-yl)ethyl]-1-piperidincarboxamid

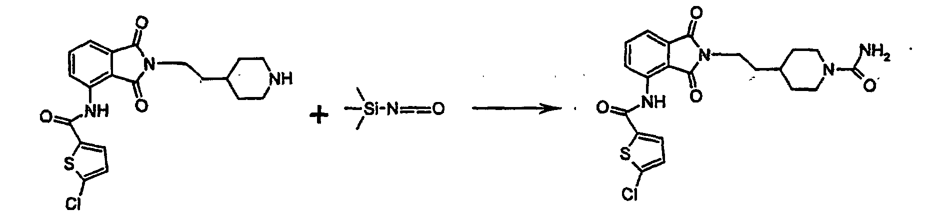

Zu einer Suspension von 50 mg (0.12 mmol) 5-Chloro-*N*-{1,3-dioxo-2-[2-(4-piperidinyl)ethyl]-2,3-dihydro-1*H*-isoindol-4-yl}-2-thiophencarboxamid in 2.5 ml absolutem Dichlormethan werden bei Raumtemperatur 0.18 ml Trimethylsilylisocyanat getropft. Es wird 48 h bei Raumtemperatur gerührt, bevor nach Einengen an Silicagel (Dichlormethan/Methanol 97:3) chromatographiert wird.
Ausbeute: 31.1 mg (56.4 % der Theorie); MS (ESI): m/z (%) = 483 (M+Na, 8), 461 (M+H, 15), 444 (45); HPLC (Methode 1): rt (%) = 4.60 (85).

### Beispiel 75

### 5-Chloro-N-{1,3-dioxo-2-[1-(2-pyridinyl)-4-piperidinyl]-2,3-dihydro-1H-isoindol-4-yl}-2-thiophencarboxamid

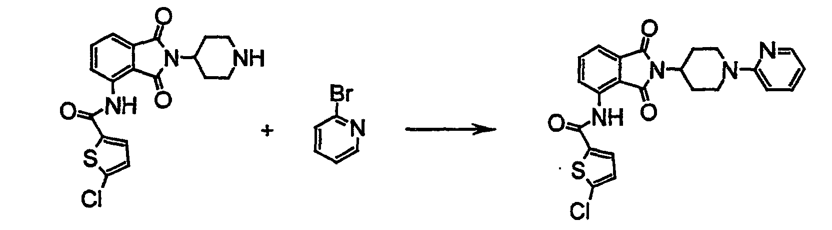

100 mg (0.257 mmol) 5-Chloro-*N*-[1,3-dioxo-2-(4-piperidinyl)-2,3-dihydro-1*H*-isoindol-4-yl]-2-thiophencarboxamid und 0.05 ml (0.513 mmol) 2-Brompyridin werden in 0.5 ml Pyridin 48 h bei 110°C gerührt. Das Gemisch wird eingeengt und am Hochvakuum getrocknet Mittels präparativer RP-HPLC wird das Produkt isoliert.
Ausbeute: 15 mg (12.5 % der Theorie); MS (DCI, NH₄): m/z (%) = 467 (M+H, 100); HPLC (Methode 1): rt (%) = 4.6 (100).

### Beispiel 76

Auf analoge Weise lässt sich **5-Chloro-*N*-(1,3-dioxo-2-{2-(1-(2-pyridinyl)-4-piperidinyl]ethyl}-2,3-dihydro-1*H*-isoindol-4-yl)-2-thiophencarboxamid** ausgehend von 5-Chloro-*N*-{1,3-dioxo-2-[2-(4-piperidinyl)ethyl]-2,3-dihydro-1*H*-isoindol-4-yl}-2-thiophencarboxamid und 2-Brompyridin darstellen.
MS (ESI): m/z (%) = 914 (M+H, 15); HPLC (Methode 1): rt (%) = 4.79 (93).

### Beispiel 77

### tert-Butyl-4-[2-(4-nitro-1-oxo-1,3-dihydro-2H-isoindol-2-yl)ethyl]-1-piperidincarboxylat

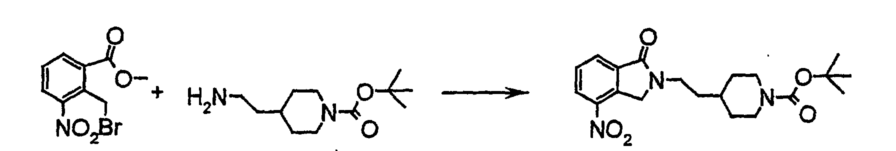

2.7 g (9.81 mmol) 2-(Bromomethyl)-3-nitrobenzoesäuremethylester (*J*. *Org. Chem*., **1999,** 9731-9734) und 2.25 g (9.81 mmol) *tert-*Butyl-4-(2-aminoethyl)-1-piperidincarboxylat (*J*. *Med*. *Chem*., **1997,** 1779-1788) werden in 45 ml absolutem DMF gelöst, mit 2.05 ml Triethylamin versetzt und 2 h bei 70°C gerührt. Nach Abkühlen wird das Reaktionsgemisch mit Wasser versetzt. Die organische Phase wird in Dichlormethan aufgenommen, über Magnesiumsulfat getrocknet und eingeengt. Das Rohprodukt wird durch Chromatographie an Silicagel gereinigt.
Ausbeute 1.96 g (51.3 % der Theorie); MS (DCI, NH₄): m/z (%) = 390 (M+H, 10), 290 (100); HPLC (Methode 2): rt (%) = 4.57 (100).

### Beispiel 78

### tert-Butyl-4-[2-(4-amino-1-oxo-1,3-dihydro-2H-isoindol-2-yl)ethyl]-1-piperidincarboxylat

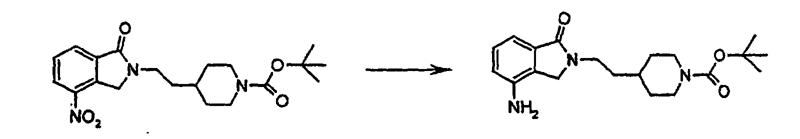

Zu einer Mischung von 2.1 g (5.4 mmol) *tert-*Butyl-4-[2-(4-nitro-1-oxo-1,3-dihydro-2*H-*isoindol-2-yl)ethyl]-1-piperidincarboxylat in 25 ml Methanol werden 450 mg Pd/C (10%) gegeben. Die Mischung wird 2 h bei Normaldruck unter Wasserstoffatmosphäre gerührt und nach Filtration über Celite eingeengt. Das Produkt wird durch Säulenfiltration an Silicagel (Dichlormethan/Methanol 9:1) gereinigt.
Ausbeute: 1.72 g (88.7 % der Theorie); MS (DCI, NH₄): m/z (%) = 377 (M+NH₄, 100); HPLC (Methode 1): rt (%) = 3.79 (100).

### Beispiel 79

### tert-Butyl 4-[2-(4-{[(5-chloro-2-thienyl)carbonyl]amino}-1-oxo-1,3-dihydro-2H-isoindol-2-yl)ethyl]-1-piperidincarboxylat

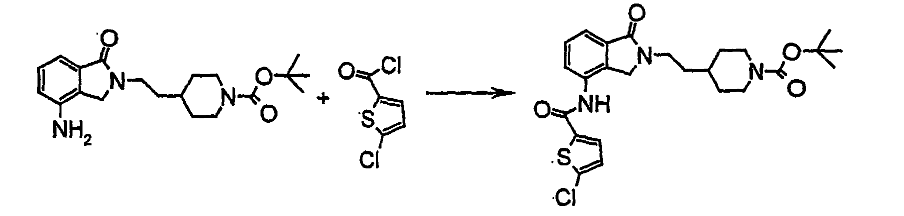

Zu einer Suspension von 1.48 g (4.12 mmol) *tert*-Butyl-4-[2-(4-amino-1-oxo-1,3-dihydro-2*H*-isoindol-2-yl)ethyl]-1-piperidincarboxylat, 1.7 ml (20.6 mmol) Pyridin und 25 mg (0.206 mmol) 4-DMAP in 21 ml absolutem THF werden bei 0°C 0.97 g (5.35 mmol) 5-Chlor-thiophen-2-carbonsäurechlorid getropft. Die Mischung wird langsam auf Raumtemperatur erwärmt und über Nacht gerührt. Nach Konzentrieren wird die Reaktionsmischung durch Chromatographie an Silicagel (Dichlormethan/Methanol 97:3) getrennt.
Ausbeute:1.95 g (94 % der Theorie); MS (ESI): m/z (%) = 504 (M+H, 15), 447 (28), 404 (100); HPLC (Methode 3): rt (%) = 4.98 (95).

### Beispiel 80

### 5-Chloro-N-(1-oxo-2-{2-oxo-2-[4-(4-pyridinyl)piperazino]ethyl}-2,3-dihydro-1H-isoindol-4-yl)-2-thiophencarboxamid und von 5-Chloro-N-(3-oxo-2-{2-oxo-2-[4-(4-pyridinyl)piperazino]ethyl}-2,3-dihydro-1H-isoindol-4-yl)-2-thiophencarboxamid

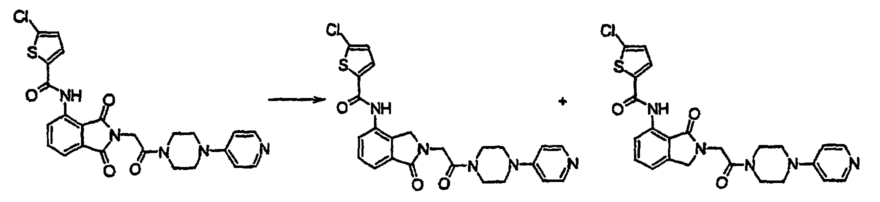

Zu einer Lösung von 1.5 g (2.94 mmol) 5-Chloro-*N*-(1,3-dioxo-2-(2-oxo-2-[4-(4-pyridinyl)piperazino]ethyl}-2,3-dihydro-1*H*-isoindol-4-yl)-2-thiophencarboxamid in 60 ml Methanol gibt man 134 mg (3.53 mmol) Natriumborhydrid und rührt 2 Stunden bei Raumtemperatur. Die erhaltene Lösung von **5-Chloro-*N*-(3-hydroxy-2-{2-oxo-2-[4-(4-pyridinyl)piperazino]ethyl}-2,3-dihydro-1*H*-isoindol-4-yl)-2-thiophencarboxamid** [¹H-NMR: 3.6-3.9 (m, 8H), 4.22 (d, 1H), 4.68 (d, 1H), 6.12 (s, 1H), 7.2 (d, 2H), 7.3 (d, 1H), 7.55 (d, 1H), 7.6 (t, 1H), 7.7 (d, 1H), 7,88 (d, 1H), 8.28 (d, 2H), 10.3 (s, 1H), 13.5 (s, 1H)] und **5-Chloro-*N*-(1-hydroxy-2-{2-oxo-2-[4-(4-pyridinyl)piperazino]ethyl}-2,3-dihydro-1*H*-isoindol-4-yl)-2-thiophencarboxamid** [¹H-NMR: 3.6-3.9 (m, 8H), 4.22 (d, 1H), 4.7 (d, 1H), 5.85 (s, 1H), 7.2 (d, 2H), 7.38 (d, 1H), 7.61 (d, 1H), 7.68 (t, 1H), 7.63 (t, 1H), 8.25-8.32 (m, 3H), 11.0 (s, 1H), 13.41 (s, 1H)] wird mit 2N HCl versetzt und mit Methylenchlorid extrahiert. Die organische Phase wird im Vakuum eingedampft (0.35 g) und die in der wässrigen und organischen Phase unlöslichen Kristalle absaugt (0.5 g). Zu 0.5 g dieser Mischung in 10 ml Methylenchlorid gibt man 1.336 g Trifluoressigsäure, gefolgt von 0.341 g (2.93 mmol) Triethylsilan, und rührt über Nacht bei Raumtemperatur. Anschließend dampft man den Ansatz im Vakuum komplett ein und chromatographiert den Rückstand auf RP8-Kieselgel mit einer 4:1 bis 1:1 Mischung von Wasser und Acetonitril.
Man erhält die beiden Isomere, wobei die zuerst eluierende Verbindung das Trifluoracetat des **1-Oxo-Isomers** darstellt: ¹H-NMR (400 MHz, *d*^{*6*}-DMSO): δ = 3.6-3.9 (m, 8H), 4.50 (s, 2H), 4.55 (s, 2H), 7.21 (d, 2H), 7.30 (d, 1H), 7.50-7.70 (m, 3H), 7.90 (d, 1H), 8.30 (d, 2H), 10.52 (s, 1H), 13.50 (breites s, 1H).
**3-Oxo-Isomer** (Trifluoracetat): ¹H-NMR (400 MHz, *d*⁶-DMSO): δ = 3.6-3.9 (m, 8H), 4.57 (s, 2H), 4.59 (s, 2H), 7.21 (d, 2H), 7.31 (m, 2H), 7.6 (d, 1H), 7.63 (t, 1H), 8.28 (d, 1H), 8.3 (d, 2H), 11.23 (s, 1H), 13.58 (breites s, 1H).

Analog erhält man aus 5-Chloro-*N*-(1,3-dioxo-2-{2-[4-(4-pyridinyl)piperazino]-ethyl}-2,3-dihydro-1*H*-isoindol-4-yl)-2-thiophencarboxamid durch NaBH₄-Reduktion und gegebenenfalls anschließende Triethylsilan/TFA-Reduktion die TFA-Salze folgender Verbindungen, wobei zuerst Triethylsilan und dann TFA hinzugegeben wurde:

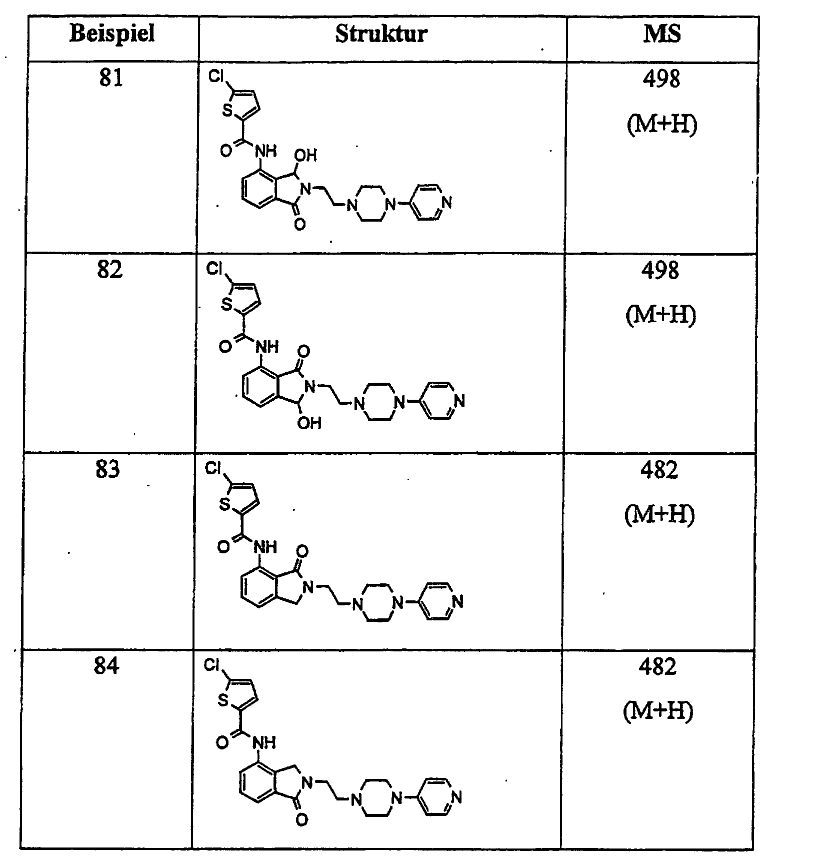

Analog erhält man aus 5-Chloro-*N*-(1,3-dioxo-2-{3-oxo-3-[4-(4-pyridinyl)piperazino]propyl}-2,3-dihydro-1*H*-isoindol-4-yl)-2-thiophencarboxamid durch NaBH₄-Reduktion und gegebenenfalls anschließende Triethylsilan/TFA-Reduktion die TFA-Salze folgender Verbindungen, wobei zuerst Triethylsilan und dann TFA hinzugegeben wurde:

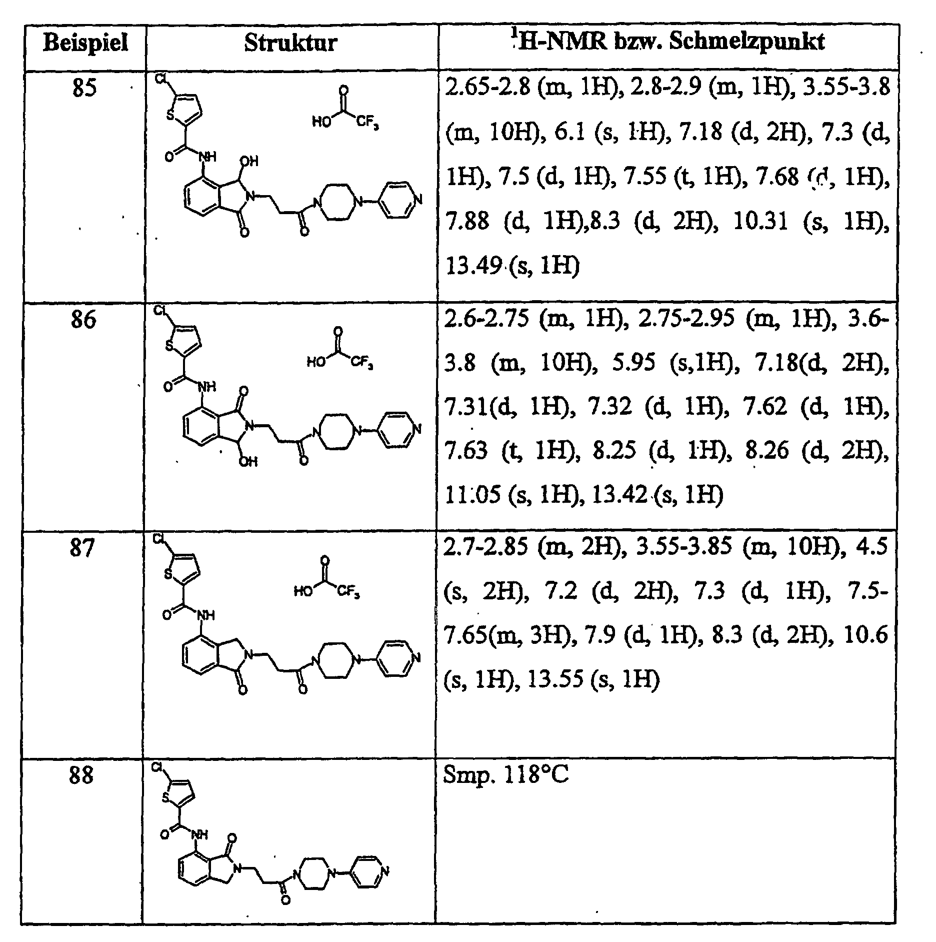

Durch Reduktion von 5-Chloro-*N*-(2-{2-[4-(hydroxymethyl)piperidino]-2-oxoethyl}-1,3-dioxo-2,3-dihydro-1*H*-isoindol-4-yl)-2-thiophencarboxamid mit NaBH₄ und gegebenenfalls anschließend mit Triethylsilan/TFA erhält man folgende Verbindungen:

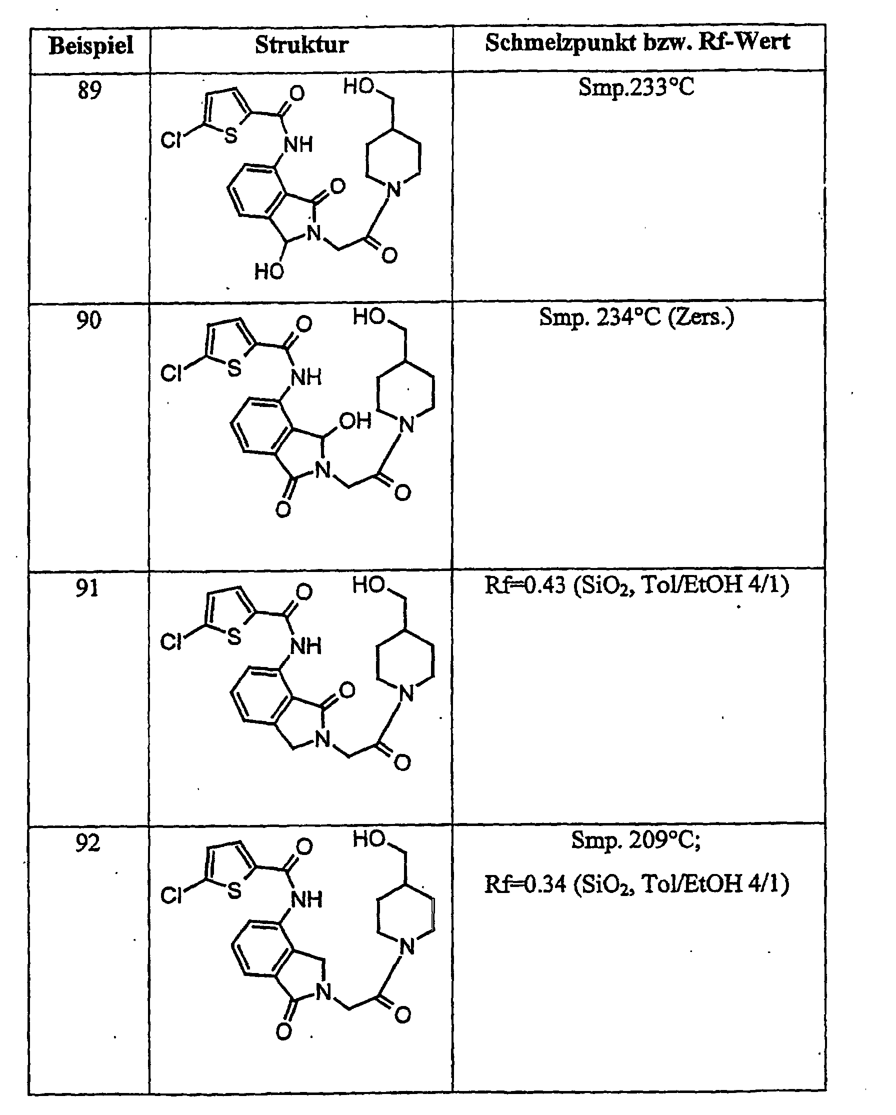

Aus 5-Chloro-*N*-(1,3-dioxo-2-{[1-(4-pyridinyl)-4-piperidinyl]methyl}-2,3-dihydro-1*H*-isoindol-4-yl)-2-thiophencarboxamid erhält man durch Reduktion mit NaBH₄ und anschließend gegebenenfalls mit Triethylsilan/TFA die folgenden Verbindungen:

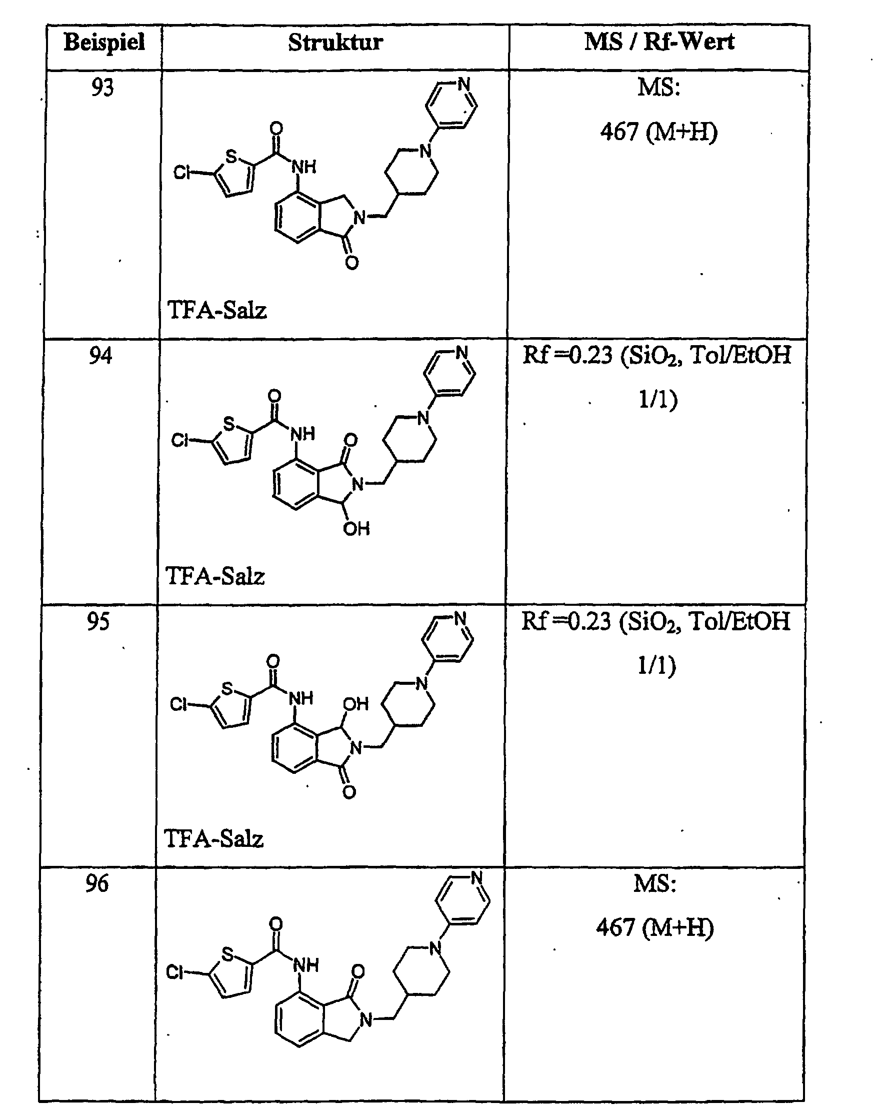

### Beispiel 97

### 5-Chloro-N-{3-oxo-2-[3-oxo-3-(1-piperazinyl)propyl]-2,3-dihydro-1H-isoindol-4-yl}-2-thiophencarboxamid und 5-Chloro-N-{1-oxo-2-[3-oxo-3-(1-piperazinyl)-propyl]-2,3-dihydro-1H-isoindol-4-yl}-2-thiophencarboxamid

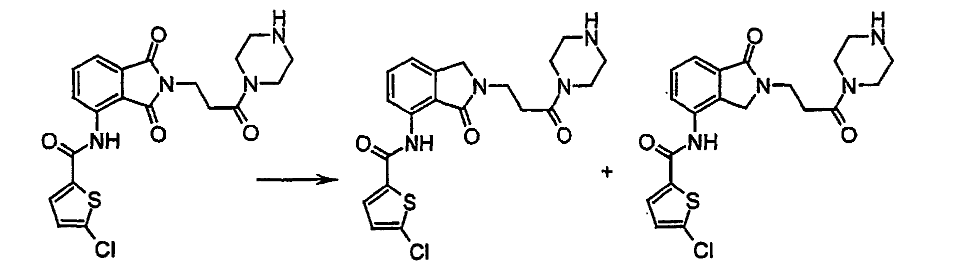

511 mg (1.14 mmol) 5-Chloro-*N*-{1,3-dioxo-2-[3-oxo-3-(1-piperazinyl)propyl]-2,3-dihydro-1*H*-isoindol-4-yl}-2-thiophencarboxamid werden in 30 ml Methanol suspendiert und mit 216 mg (5.72 mmol) Natriumborhydrid versetzt. Das Reaktionsgemisch wird 4 h bei Raumtemperatur gerührt, dann vorsichtig mit 2 N Salzsäure angesäuert, 20 min gerührt, mit 2 N Natronlauge basisch gestellt und dreimal mit Methylenchlorid extrahiert. Die vereinigten organischen Phasen werden über Magnesiumsulfat getrocknet, filtriert und eingeengt.

Der Rückstand wird in 20 ml Methylenchlorid gelöst und mit 1.07 g (9.36 mmol) Trifluoressigsäure und 181 mg (1.56 mmol) Triethylsilan versetzt. Das Reaktionsgemisch wird über Nacht bei Raumtemperatur gerührt und anschließend eingeengt. Der Rückstand wird an Kieselgel chromatographiert (CH₂Cl₂/EtOH/konz. wässr. Ammoniaklsg = 20/1/0.01 bis 5/1/0.01).
Es werden 170 mg des **3-Oxo-Isomers** (MS = 433 (M+H), rt (Methode 5) = 2.86 min) und 168 mg des **1-Oxo-Isomers** (MS = 433 (M+H), rt (Methode 5) = 2.47 min) erhalten.

### Beispiel 98

### 5-Chloro-N-{3-hydroxy-2-[2-(1-methyl-2-pyrrolidinyl)ethyl]-1-oxo-2,3-dihydro-1H-isoindol-4-yl}-2-thiophencarboxamid und 5-Chloro-N-{1-hydroxy-2-[2-(1-methyl-2-pyrrolidinyl)ethyl]-3-oxo-2,3-dihydro-1H-isoindol-4-yl}-2-thiophencarboxamid

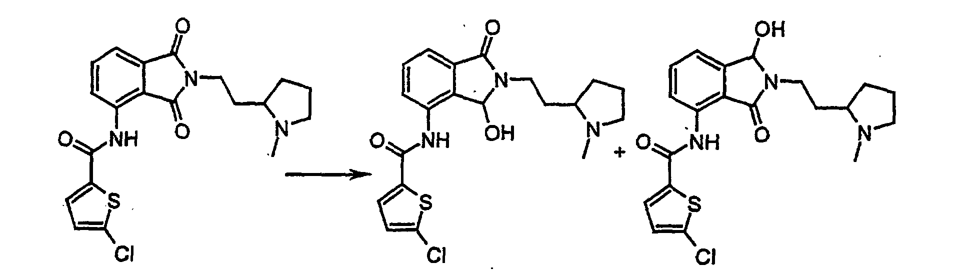

553 mg (1.32 mmol) 5-Chloro-*N*-{2-[2-(1-methyl-2-pyrrolidinyl)ethyl]-2,3-dioxo-2,3-dihydro-1*H*-isoindol-4-yl}-2-thiophencarboxamid werden in 20 ml Methanol gelöst und mit 250 mg (6.62 mmol) Natriumborhydrid versetzt. Nach 4 h Rühren bei RT wird das Reaktionsgemisch mit 2 N Salzsäure vorsichtig angesäuert, 20 min intensiv gerührt, mit 2 N Natronlauge basisch gestellt und dreimal mit Dichlormethan extrahiert. Die vereinigten organischen Phasen werden über MgSO₄ getrocknet, filtriert und eingeengt.
Es werden 545 mg (97 % d. Th.) des Produktes als Isomerengemisch erhalten.
MS = 420 (M+H), LC (Methode 6): rt = 2.41 min und 2.75 min.

### Beispiel 99

### 5-Chloro-N-{2-[2-(1-methyl-2-pyrrolidinyl)ethyl]-1-oxo-2,3-dihydro-1H-isoindol-4-yl}-2-thiophencarboxamid und 5-Chloro-N-{2-[2-(1-methyl-2-pyrrolidinyl)ethyl]-3-oxo-2,3-dihydro-1H-isoindol-4-yl}-2-thiophencarboxamid

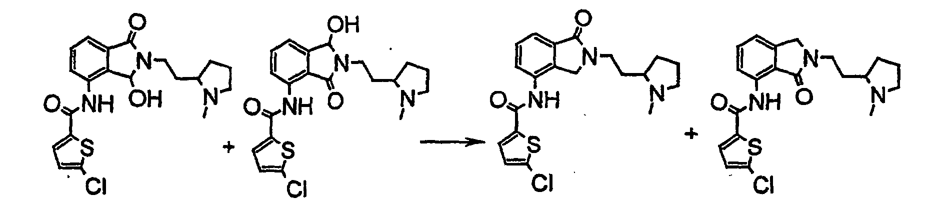

530 mg (1.26 mmol) eines Isomerengemisches von 5-Chloro-*N*-{3-hydroxy-2-[2-(1-methyl-2-pyrrolidinyl)ethyl]-1-oxo-2,3-dihydro-1*H*-isoindol-4-yl}-2-thiophencarboxamid und 5-Chloro-*N*-{1-hydroxy-2-[2-(1-methyl-2-pyrrolidinyl)ethyl]-3-oxo-2,3-dihydro-1*H*-isoindol-4-yl}-2-thiophencarboxamid werden in 15 ml Dichlormethan gelöst. Es werden 17.27 g (15.15 mmol) Trifluoressigsäure und 293.5 mg (2.52 mmol) Triethylsilan zugegeben. Nach Rühren über Nacht bei RT wird das Reaktionsgemisch mit Dichlormethan verdünnt und mit 2 N Natronlauge gewaschen. Die organische Phase wird über MgSO₄ getrocknet, filtriert und eingeengt. Das Rohprodukt wird über Kieselgel chromatographiert (CH₂Cl₂/EtOH/konz. wässrige Ammoniaklösung = 50/1/0.01 bis 20/1/0.01).
Zuerst wird das **3-Oxo-Isomer** eluiert. Es werden 196 mg (38 % d. Th.) dieses Produkts erhalten; MS = 404 (M+H), LC (Methode 5): rt = 2.90.
Vom **1-Oxo-Isomer** werden 248 mg (49 % d. Th.) erhalten; MS = 404 (M+H), LC (Methode 5): rt = 2.49 min.

### Beispiel 100

### tert-Butyl-4-[2-(4-{[(5-chloro-2-thienyl)carbonyl]amino}-1-methoxy-3-oxo-1,3-dihydro-2H-isoindol-2-yl)ethyl]-1-piperidincarboxylat und tert-Butyl-4-[2-(4-{[(5-chloro-2-thienyl)carbonyl]amino}-3-methoxy-1-oxo-1,3-dihydro-2H-isoindol-2-yl)ethyl]-1-piperidincarboxylat

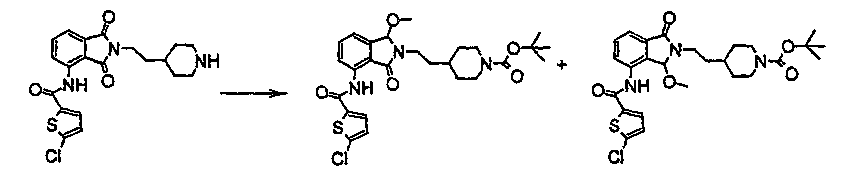

1.05 g (2.51 mmol) 5-Chloro-*N*-{1,3-dioxo-2-[2-(4-piperidinyl)ethyl]-2,3-dihydro-1*H*-isoindol-4-yl}-2-thiophencarboxamid werden in 15 ml Dichlormethan und 15 ml Methanol gelöst, auf 0°C abgekühlt und portionsweise mit 143 mg (3.77 mmol) Natriumborhydrid versetzt. Die Mischung wird über Nacht bei Raumtemperatur gerührt, bevor mit 1N Salzsäure-Lösung leicht angesäuert und mit Dichlormethan extrahiert wird. Die organische Phase wird über Magnesiumsulfat getrocknet und eingeengt. Die wässrige Phase wird eingedampft. Beide Rückstande werden vereinigt, in 15 ml 1,4-Dioxan aufgenommen und bei 0°C mit 0.82 g (3.77 mmol) Di*tert*-butyldicarbonat und 15 ml 1N Natriumhydroxid-Lösung versetzt. Die Eiskühlung wird nach 15 min entfernt und die Mischung weitere 3.5 h bei Raumtemperatur gerührt, bevor nach Verdünnen mit Wasser mit Dichlormethan extrahiert wird. Der organische Extrakt wird über Magnesiumsulfat getrocknet und eingeengt.

Das Produktgemisch wird durch Chromatographie an Silicagel (Dichlormethan/Methanol 98:2) getrennt.
Ausbeute: 400 mg (29.8 % der Theorie) an **1-Methoxy-3-oxo Isomer**; LC-MS: m/z (%) = 551 (M+NH₄⁺, 30), 534 (M+H, 75), 434 (100); HPLC (Methode 1): rt (%) = 5.56 (100).
Ausbeute: 560 mg (41.7 % der Theorie) an **3-Methoxy-1-oxo Isomer** LC-MS: m/z (%) = 551 (M+NH₄⁺, 20), 502 (85), 402 (100); HPLC (Methode 6): rt (%) = 4.84 (79).

### Beispiel 101

### 5-Chloro-N-{3-oxo-2-[2-(4-piperidinyl)ethyl]-2,3-dihydro-1H-isoindol-4-yl}-2-thiophencarboxamid

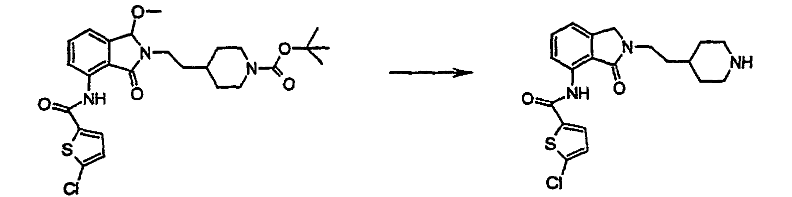

Zu einer Lösung von 378 mg (0.704 mmol) *tert*-Butyl-4-[2-(4-{[(5-chloro-2-thienyl)carbonyl]amino}-1-methoxy-3-oxo-1,3-dihydro-2*H*-isoindol-2-yl)ethyl]-1-piperidin-carboxylat in 12 ml Dichlormethan und 0.65 ml Trifluoressigsäure werden 0.23 ml (1.4 mmol), Triethylsilan gegeben. Die Mischung wird über Nacht gerührt, eingeengt und in Dichlormethan aufgenommen. Die Lösung wird mit 1N Natriumhydroxid-Lösung gewaschen, getrocknet und eingeengt. Der Rückstand wird mit Diethylether kristallisiert. Der erhaltene Feststoff wird durch Chromatographie an Silicagel (Gradient von Dichlormethan/Methanol 95:5 bis Dichlormethan/Methanol/Triethylamin 9:1: 0.1) weiter aufgereinigt. Ausbeute 144 mg (50.5 % der Theorie). MS (DCI, NH₄): m/z (%) = 404 (M+H, 100); HPLC (Methode 3): rt (%) = 4.19 (91).

### Beispiel 102

Auf analoge Weise lässt sich **5-Chloro-*N*-{1-oxo-2-[2-(4-piperidinyl)ethyl]-2,3-dihydro-1*H*-isoindol-4-yl}-2-thiophencarboxamid,** ausgehend von *tert*-Butyl 4-[2-(4-{[(5-chloro-2-thienyl)carbonyl]amino}-3-methoxy-1-oxo-1,3-dihydro-2*H*-isoindol-2-yl)ethyl]-1-piperidincarboxylat, darstellen.
MS (ESI): m/z (%) = 404 (M+H, 86), 305 (100); HPLC (Methode 2): rt (%) = 3.93 (96).

### Darstellung von 5-Chloro-N-{2-(2-hydroxy-3-(1-piperazinyl)propyl]-1,3-dioxo-2,3-dihydro-1H-isoindol-4-yl}-2-thiophencarboxamid-Derivaten aus 5-Chloro-N-(1,3-dioxo-2,3-dihydro-1H-isoindol-4-yl)-2-thiophencarboxamid

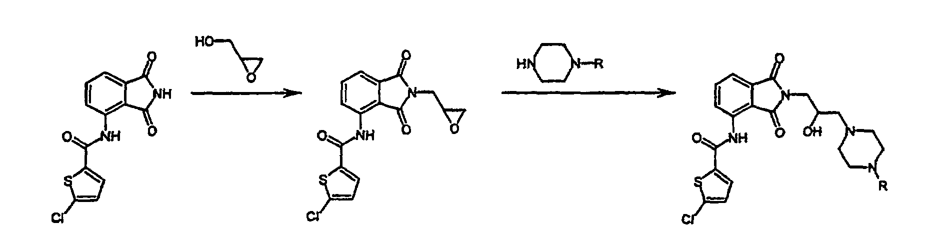

### Beispiel 103

### 5-Chloro-N-[2-(2-oxiranylmethyl)-1,3-dioxo-2,3-dihydro-1H-isoindol-4-yl]-2-thiophencarboxamid

Zu einer Lösung von 5-Chloro-*N*-(1,3-dioxo-2,3-dihydro-1*H*-isoindol-4-yl)-2-thiophencarboxamid, (*R*/*S*)-2,3-Epoxy-1-propanol (1.1 eq.) und Triphenylphosphin (1.1 eq.) in Tetrahydrofuran (0.1 mol/l) wird unter Argon bei Raumtemperatur tropfenweise Azodicarbonsäurediethylester (1.1 eq.) gegeben. Die Reaktionsmischung wird 16-20 h gerührt und mit Wasser versetzt. Nach Zugabe von Dichlormethan und Phasentrennung wird die wässrige Phase mit Dichlormethan extrahiert, und die vereinigten organischen Phasen werden getrocknet (Natriumsulfat), filtriert und im Vakuum eingeengt. Das gewünschte Produkt wird mittels Flash-Chromatographie (Cyclohexan-Ethylacetat-Gemische) gereinigt.
MS (DCI, NH₃): m/z (%) = 380 ([M+NH₄]⁺, 100), Cl-Muster; HPLC (Methode 1): rt = 4.69 min.

Zu einer Lösung des Epoxids und des substituierten Piperazins (1.2 eq.) in Tetrahydrofuran (0.1 mol/l) wird unter Argon bei Raumtemperatur Triethylamin (1 eq.) gegeben. Das Reaktionsgemisch wird für 3d refluxiert und anschließend im Vakuum eingeengt. Das gewünschte Produkt wird mittels Flash-Chromatographie (Dichlormethan-Methanol-Gemische) gereinigt.

Folgende Verbindungen wurden analog dargestellt:

### Beispiel 104

### 5-Chloro-N-(2-{2-hydroxy-3-[4-(4-pyridinyl)-1-piperazinyl]propyl}-1,3-dioxo-2,3-dihydro-1H-isoindol-4-yl)-2-thiophencarboxamid

MS (ESI): m/z (%) = 526 ([M+H]⁺, 100), Cl-Muster; HPLC (Methode 1): rt = 3.68 min.

### Beispiel 105

### 5-Chloro-N-(2-{2-hydroxy-3-[4-(2-methoxyphenyl)-1-piperazinyl]propyl}-1,3-dioxo-2,3-dihydro-1H-isoindol-4-yl)-2-thiophencarboxamid

MS (ESI): m/z (%) = 555 ([M+H]⁺, 100), Cl-Muster; HPLC (Methode 2): rt = 3.97 min.

### Beispiel 106

### 5-Chloro-N-(2-{3-[4-(2-fluorophenyl)-1-piperazinyl]-2-hydroxypropyl}-1,3-dioxo-2,3-dihydro-1H-isoindol-4-yl)-2-thiophencarboxamid

MS (ESI): m/z (%) = 543 ([M+H]⁺, 100), Cl-Muster; HPLC (Methode 2): rt = 4.00 min.

### Beispiel 107

### 5-Chloro-N-(2-{3-[4-(4-fluorophenyl)-1-piperazinyl]-2-hydroxypropyl}-1,3-dioxo-2,3-dihydro-1H-isoindol-4-yl)-2-thiophencarboxamid

MS (ESI): m/z (%) = 543 ([M+H]⁺, 100), Cl-Muster; HPLC (Methode 2): rt = 4.01 min.

### Beispiel 108

### 5-Chloro-N-(2-{2-hydroxy-3-[4-(2-pyridinyl)-1-piperazinyl]propyl}-1,3-dioxo-2,3-dihydro-1H-isoindol-4-yl)-2-thiophencarboxamid

MS (ESI): m/z (%) = 526 ([M+H]⁺, 100), Cl-Muster; HPLC (Methode 2): rt = 3.36 min.

### Beispiel 109

### 5-Chloro-N-(2-{2-hydroxy-3-[4-(2-nitrophenyl)-1-piperazinyl]propyl}-1,3-dioxo-2,3-dihydro-1H-isoindol-4-yl)-2-thiophencarboxamid

MS (ESI): m/z (%) = 570 ([M+H]⁺, 100), Cl-Muster; HPLC (Methode 2): rt = 4.00 min.

### Beispiel 110

### 5-Chloro-N-(2-{3-[4-(4-chlorophenyl)-1-piperazinyl]-2-hydroxypropyl}-1,3-dioxo-2,3-dihydro-1H-isoindol-4-yl)-2-thiophencarboxamid

MS (ESI): m/z (%) = 559 ([M+H]⁺, 100), Cl₂-Muster; HPLC (Methode 2): rt = 4.05 min.

### Beispiel 111

### 5-Chloro-N-(2-{2-hydroxy-3-[4-(3-methylphenyl)-1-piperazinyl]propyl}-1,3-dioxo-2,3-dihydro-1H-isoindol-4-yl)-2-thiophencarboxamid

MS (ESI): m/z (%) = 539 ([M+H]⁺, 100), Cl-Muster; HPLC (Methode 2): rt = 3.97 min.

### Beispiel 112

### 5-Chloro-N-{2-[3-(4-cyclohexyl-1-piperazinyl)-2-hydroxypropyl]-1,3-dioxo-2,3-dihydro-1H-isoindol-4-yl}-2-thiophencarboxamid

MS (ESI): m/z (%) = 531 ([M+H]⁺, 100), Cl-Muster; HPLC (Methode 2): rt = 3.53 min.

### Beispiel 113

### 5-Chloro-N-(2-{2-hydroxy-3-[4-(2-pyrimidinyl)-1-piperazinyl]propyl}-1,3-dioxo-2,3-dihydro-1H-isoindol-4-yl)-2-thiophencarboxamid

MS (ESI): m/z (%) = 527 ([M+H]⁺, 100), Cl-Muster; HPLC (Methode 2): rt = 3.66 min.

### Beispiel 114

### 5-Chloro-N-(2-{3-[4-(2,4-difluorophenyl)-1-piperazinyl]-2-hydroxypropyl}-1,3-dioxo-2,3-dihydro-1H-isoindol-4-yl)-2-thiophencarboxamid

MS (ESI): m/z (%) = 561 ([M+H]⁺, 100), Cl-Muster; HPLC (Methode 2): rt = 4.01 min.

### Beispiel 115

### 5-Chloro-N-{2-[2-hydroxy-3-(4-phenyl-1-piperazinyl)propyl]-1,3-dioxo-2,3-dihydro-1H-isoindol-4-yl}-2-thiophencarboxamid

MS (ESI): m/z (%) = 525 ([M+H]⁺, 100), Cl-Muster; HPLC (Methode 2): rt = 3.92 min.

### Beispiel 116

### 5-Chloro-N-[2-(2-hydroxy-3-{4-[3-(trifluoromethyl)phenyl]-1-piperazinyl}propyl)-1,3-dioxo-2,3-dihydro-1H-isoindol-4-yl]-2-thiophencarboxamid

MS (ESI): m/z (%) = 593 ([M+H]⁺, 100), Cl-Muster; HPLC (Methode 2): rt = 4.33 min.

### Beispiel 117

### 5-Chloro-N-(2-{2-hydroxy-3-[4-(4-methoxyphenyl)-1-piperazinyl]propyl}-1,3-dioxo-2,3-dihydro-1H-isoindol-4-yl)-2-thiophencarboxamid

MS (ESI): m/z (%) = 555 ([M+H]⁺, 100), Cl-Muster; HPLC (Methode 2): rt = 4.05 min.

### Beispiel 118

### N-{2-[3-(1,4'-Bipiperidin-1'-yl)-2-hydroxypropyl]-1,3-dioxo-2,3-dihydro-1Hisoindol-4-yl}-5-chloro-2-thiophencarboxamid

MS (ESI): m/z (%) = 531 ([M+H]⁺, 100), Cl-Muster; HPLC (Methode 4): rt = 2.56 min.

Die beiden folgenden Verbindungen lassen sich durch Reduktion der entsprechenden Phthalimide mit i) NaBH4, ii) TFA, Et₃SiH darstellen:

### Beispiel 119

### 5-Chloro-N-(2-{2-hydroxy-3-[4-(4-pyridinyl)-1-piperazinyl]propyl}-1-oxo-2,3-dihydro-1H-isoindol-4-yl)-2-thiophencarboxamid

MS (ESI): m/z (%) = 512 ([M+H]⁺, 100), Cl-Muster; HPLC (Methode 4): rt = 2.16 min.

### Beispiel 120

### 5-Chloro-N-(2-{2-hydroxy-3-[4-(4-pyridinyl)-1-piperazinyl]propyl}-3-oxo-2,3-dihydro-1H-isoindol-4-yl)-2-thiophencarboxamid

MS (ESI): m/z (%) = 512 {[M+H]⁺, 100), Cl-Muster; HPLC (Methode 4): rt = 2.50 min.

## Patentansprüche

1. Verbindungen der Formel (I) 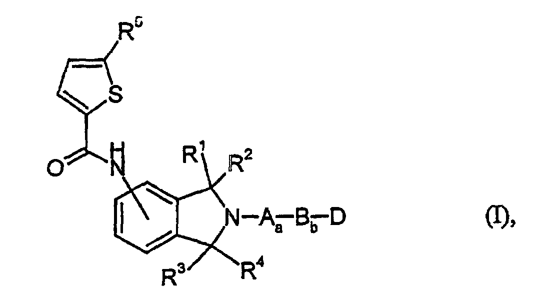 worin
R¹ und R² gemeinsam für O stehen und
R³ und R⁴ gemeinsam für O stehen,
oder
R¹ Wasserstoff, Hydroxy oder (C₁-C₄)-Alkoxy bedeutet,
R² Wasserstoff bedeutet und
R³ und R⁴ gemeinsam für O stehen,
oder
R¹ und R² gemeinsam für O stehen,
R³ Wasserstoff, Hydroxy oder (C₁-C₄)-Alkoxy bedeutet und
R⁴ Wasserstoff bedeutet,
R⁵ Halogen, Trifluormethyl oder Methyl bedeutet,
A (C₁-C₄)-Alkandiyl bedeutet, das durch Hydroxy oder (C₁-C₄)-Alkoxy substituiert sein kann,
a 0 oder 1 bedeutet,
B für eine Gruppe 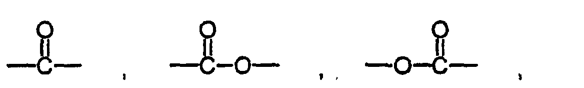 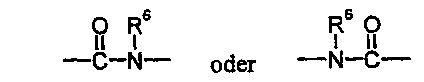 steht,
worin
R⁶ Wasserstoff oder (C₁-C₄)-Alkyl bedeutet,
b 0 oder 1 bedeutet,
D 5- bis 7-gliedriges Heterocyclyl,
das ein- oder zweifach, unabhängig voneinander, durch Hydroxy, Carbamoyl, (C₁-C₄)-Alkanoyl, ein über eine Carbonylgruppe verknüpftes (C₃-C₇)-Cycloalkyl, (C₃-C₇)-Cycloalkyl, 5- bis 10-gliedriges Heterocyclyl, (C₁-C₄)-Alkoxycarbonyl, (C₁-C₆)-Alkyl,
welches seinerseits durch Hydroxy, Cyano, (C₁-C₄)-Alkoxy, Mono- oder Di-(C₁-C₆)-alkylamino, Mono- oder Di-(C₁-C₆)-alkylaminocarbonyl, 5- bis 10-gliedriges Heterocyclyl, 5- oder 6-gliedriges Heterocyclylcarbonyl oder 5- bis 10-gliedriges Heteroaryl substituiert sein kann, (C₆-C₁₀)-Aryl,
welches seinerseits durch Halogen, Trifluormethyl, Nitro, (C₁-C₄)-Alkyl oder (C₁-C₄)-Alkoxy substituiert sein kann, 5- bis 10-gliedriges Heteroaryl,
welches seinerseits durch Cyano, Amino oder (C₁-C₄)-Alkyl substituiert sein kann,
oder 5- bis 10-gliedriges Heteroarylcarbonyl substituiert sein kann,
(C₁-C₆)-Alkyl,
das durch Cyano, Amino, Mono- oder Di-(C₁-C₆)-alkylamino, Amidino, 5- bis 10-gliedriges Heteroaryl, gegebenenfalls durch Halogen substituiertes 5- bis 10-gliedriges Heteroarylamino, gegebenenfalls durch 5- bis 10-gliedriges Heteroaryl substituiertes 5- bis 10-gliedriges Heterocyclyl oder (C₁-C₄)-Alkanoylamino substituiert sein kann,
oder
5- bis 10-gliedriges Heteroaryl,
das durch Halogen oder (C₁-C₄)-Alkyl substituiert sein kann, bedeutet,
sowie deren Salze, Hydrate, Hydrate der Salze und Solvate.

2. Verbindungen nach Anspruch 1,
worin
der Thiophencarbonsäuresubsrituent in ortho-Position zur Anknüpfungsstelle des annelierten Heterocyclus mit dem Phenylring verknüpft ist,
R¹ und R² gemeinsam für O stehen und
R³ und R⁴ gemeinsam für O stehen,
oder
R¹ Wasserstoff, Hydroxy, Methoxy oder Ethoxy bedeutet,
R² Wasserstoff bedeutet und
R³ und R⁴ gemeinsam für 0 stehen,
oder
R¹ und R² gemeinsam für O stehen,
R³ Wasserstoff, Hydroxy, Methoxy oder Ethoxy bedeutet und
R⁴ Wasserstoff bedeutet,
R⁵ Halogen oder Trifluormethyl bedeutet,
A (C₁-C₄)-Alkandiyl bedeutet, das durch Hydroxy substituiert sein kann,
a 0 oder 1 bedeutet,
B für eine Gruppe 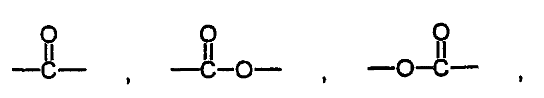 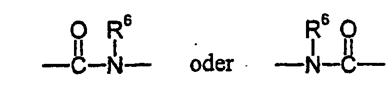 steht,
worin
R⁶ Wasserstoff bedeutet,
b 0 oder 1 bedeutet,
D 5- bis 7-gliedriges Heterocyclyl,
das ein- oder zweifach, unabhängig voneinander, durch Hydroxy, Carbamoyl, Acetyl, Cyclopropanoyl, (C₃-C₆)-Cycloalkyl, 5- bis 10-gliedriges Heterocyclyl, (C₁-C₃)-Alkyl,
welches seinerseits durch Hydroxy, Methoxy, Mono- oder Dimethylamino, Mono- oder Di-(C₁-C₃)-alkylaminocarbonyl, 5-oder 6-gliedriges Heterocyclyl, 5- oder 6-gliedriges Heterocyclylcarbonyl oder 5- oder 6-gliedriges Heteroaryl substituiert sein kann,
Phenyl,
welches seinerseits durch Fluor, Chlor, Trifluormethyl, Methyl oder Methoxy substituiert sein kann,
oder 5- oder 6-gliedriges Heteroaryl substituiert sein kann, welches seinerseits durch Cyano, Amino oder Methyl substituiert sein kann,
(C₁-C₆)-Alkyl,
das durch Amino, Mono- oder Di-methylamino, Amidino, 5- oder 6-gliedriges Heteroaryl, 5- oder 6-gliedriges Heteroarylamino, gegebenenfalls durch 5- oder 6-gliedriges Heteroaryl substituiertes 5- oder 6-gliedriges Heterocyclyl oder (C₁-C₄)-Alkanoylamino substituiert sein kann,
oder
5- bis 9-gliedriges Heteroaryl bedeutet,
sowie deren Salze, Hydrate, Hydrate der Salze und Solvate.

3. Verbindungen nach Anspruch 1,
worin
der Thiophencarbonsäuresubstituent in ortho-Position zur Anknüpfungsstelle des annelierten Heterocyclus mit dem Phenylring verknüpft ist,
R¹ und R² gemeinsam für O stehen und
R³ und R⁴ gemeinsam für O stehen,
oder
R¹ Wasserstoff, Hydroxy oder Methoxy bedeutet,
R² Wasserstoff bedeutet und
R³ und R⁴ gemeinsam für O stehen,
oder
R¹ und R² gemeinsam für O stehen,
R³ Wasserstoff, Hydroxy oder Methoxy bedeutet und
R⁴ Wasserstoff bedeutet,
R⁵ Chlor oder Brom bedeutet,
A Methandiyl, Ethandiyl oder Propan-1,3-diyl bedeutet, die durch Hydroxy substituiert sein können,
a 0 oder 1 bedeutet,
B für eine Gruppe 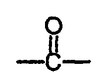 steht,
b 0 oder 1 bedeutet,
D Pyrolidin, Piperidin oder Piperazin,
die ein- oder zweifach, unabhängig voneinander, durch Methyl, Ethyl, n-Propyl oder Isopropyl,
welche ihrerseits durch Hydroxy oder Pyridyl substituiert sein können,
oder Pyridyl substituiert sein können,
welches seinerseits durch Amino oder Methyl substituiert sein kann,
oder
(C₁-C₃)-Alkyl bedeutet,
das durch Amidino, gegebenenfalls durch Pyridyl substituiertes Piperidinyl oder Acetylamino substituiert sein kann,
sowie deren Salze, Hydrate, Hydrate der Salze und Solvate.

4. Verfahren zur Herstellung von Verbindungen der Formel (1), wie in Anspruch 1 definiert, **dadurch gekennzeichnet, dass** man entweder
[A.1] Verbindungen der Formel (VII) 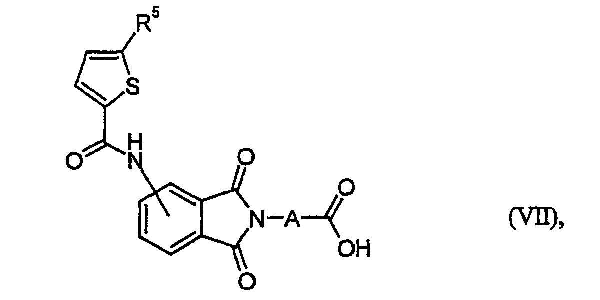 worin A und R⁵ die in Anspruch 1 angegebene Bedeutung besitzen,
durch Reaktion mit Aminen oder Alkoholen
zu Verbindungen der Formel (I) umsetzt
oder
[A.2] Verbindungen der Formel (IX) 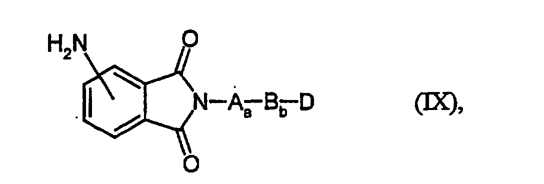 worin a, b, A, B und D die in Anspruch 1 angegebene Bedeutung besitzen,
mit Verbindungen der Formel (III) 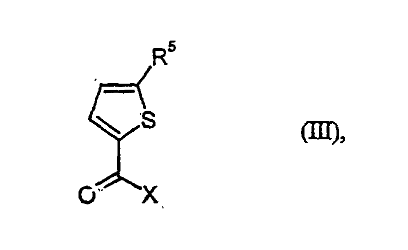 worin R⁵ die in Anspruch 1 angegebene Bedeutung hat und X für eine Abgangsgruppe steht,
zu Verbindungen der Formel (I) umsetzt
oder
[B.1] Verbindungen der Formel (XI) 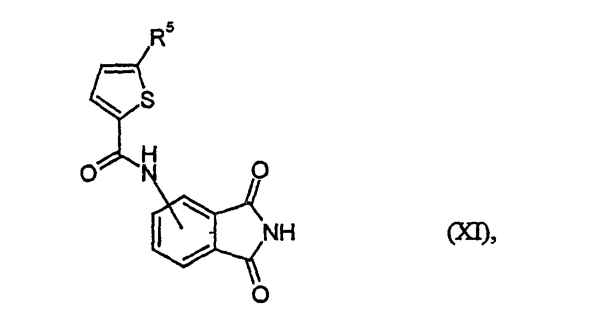 worin R⁵ die in Anspruch 1 angegebene Bedeutung besitzt,
mit Verbindungen der Formel (XII)
HO-Aₐ-B_{b}-D (XII),
worin A, a, B, b und D die in Anspruch 1 angegebene Bedeutung besitzen,
zu Verbindungen der Formel (I) umsetzt
oder
[C] Verbindungen der Formel (XVI) 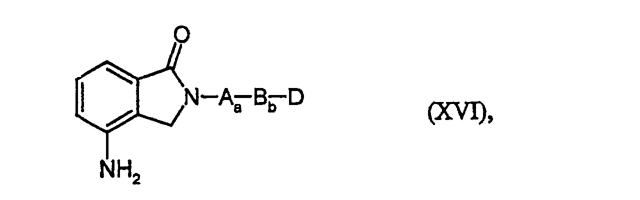 worin A, a, B, b und D die in Anspruch 1 angegebene Bedeutung besitzen, mit Verbindungen der Formel (III) zu Verbindungen der Formel (I) umsetzt,
wobei sich bei den so erhaltenen Verbindungen der Formel (I) gegebenenfalls weitere Derivatisierungen, die nach üblichen Methoden durchgeführt werden können, anschließen können.

5. Verbindungen der Formel (I), wie in Anspruch 1 definiert, zur Prävention und/oder Behandlung von Erkrankungen.

6. Arzneimittel enthaltend mindestens eine Verbindung der Formel (I), wie in Anspruch 1 definiert, und mindestens einen weiteren Hilfsstoff.

7. Arzneimittel enthaltend mindestens eine Verbindung der Formel (I), wie in Anspruch 1 definiert, und mindestens einen weiteren Wirkstoff.

8. Verwendung von Verbindungen der Formel (I), wie in Anspruch 1 definiert, zur Herstellung eines Arzneimittels zur Prävention und/oder Behandlung von thromboembolischen Erkrankungen, insbesondere Heizinfarkt, Angina Pectoris (eingeschlossen instabile Angina), Reokklusionen und Restenosen nach einer Angioplastie oder aortokoronarem Bypass, Hirnschlag, transitorische ischämische Attacken, periphere arterielle Verschlusskrankheiten, Lungenembolien oder tiefe venöse Thrombosen.

9. Verwendung von Verbindungen der Formel (I), wie in Anspruch 1 definiert, zur Herstellung eines Arzneimittels zur Prävention und/oder Behandlung der disseminierten intravasalen Gerinnung (DIC).

10. Verwendung von Verbindungen der Formel (I), wie in Anspruch 1 definiert, zur Herstellung eines Arzneimittels zur Prävention und/oder Behandlung von Erkrankungen wie Atherosklerose, Arthritis, Alzheimer'sche Erkrankung oder Krebs.

11. Verfahren zur Verhinderung der Blutkoagulation in vitro, insbesondere bei Blutkonserven oder biologischen Proben, die Faktor Xa enthalten, **dadurch gekennzeichnet, dass** Verbindungen der Formel (I), wie in Anspruch 1 definiert, zugegeben werden.

## Claims

1. Compounds of the formula (I) 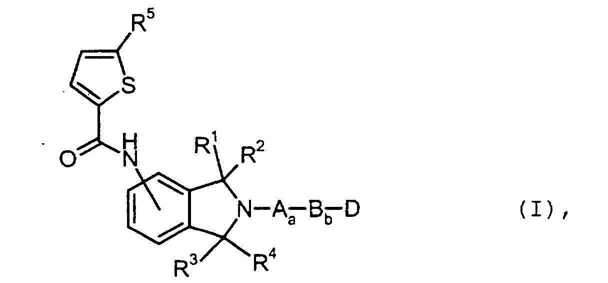 in which
R¹ and R² together represent O and
R³ and R⁴ together represent O,
or
R¹ represents hydrogen, hydroxy or (C₁-C₄)-alkoxy,
R² represents hydrogen and
R³ and R⁴ together represent O,
or
R¹ and R² together represent O,
R³ represents hydrogen, hydroxy or (C₁-C₄)-alkoxy and
R⁴ represents hydrogen,
R⁵ represents halogen, trifluoromethyl or methyl,
A represents (C₁-C₄)-alkanediyl which may be substituted by hydroxy or (C₁-C₄)-alkoxy,
a represents 0 or 1,
B represents a group 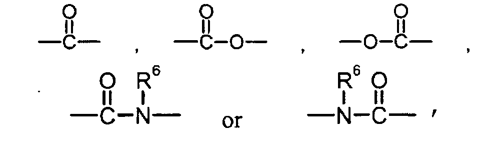 in which
R⁶ represents hydrogen or (C₁-C₄)-alkyl,
b represents 0 or 1,
D represents a 5- to 7-membered heterocyclyl, which may be mono- or disubstituted, independently of one another, by hydroxy, carbamoyl, (C₁-C₄)-alkanoyl, (C₃-C₇)-cycloalkyl which is attached via a carbonyl group, (C₃-C₇) -cycloalkyl, 5- to 10-membered heterocyclyl, (C₁-C₄) -alkoxycarbonyl, (C₁-C₆)-alkyl,
which for its part may be substituted by hydroxy, cyano, (C₁-C₄)-alkoxy, mono- or di-(C₁-C₆)-alkylamino, mono- or di-(C₁-C₆)-alkylaminocarbonyl, 5- to 10-membered heterocyclyl, 5- or 6-membered heterocyclylcarbonyl or 5- to 10-membered heteroaryl,
(C₆-C₁₀) -aryl,
which for its part may be substituted by halogen, trifluoromethyl, nitro, (C₁-C₄)-alkyl or (C₁-C₄)-alkoxy,
5- to 10-membered heteroaryl,
which for its part may be substituted by cyano, amino or (C₁-C₄)-alkyl,
or 5- to 10-membered heteroarylcarbonyl,
(C₁-C₆)-alkyl,
which may be substituted by cyano, amino, mono- or di-(C₁-C₆)-alkylamino, amidino, 5- to 10-membered heteroaryl, by 5- to 10-membered heteroarylamino which is optionally substituted by halogen, by 5- to 10-membered heterocyclyl which is optionally substituted by 5- to 10-membered heteroaryl, or by (C₁-C₄)-alkanoylamino,
or
5- to 10-membered heteroaryl,
which may be substituted by halogen or (C₁-C₄)-alkyl,
and its salts, hydrates, hydrates of the salts and solvates.

2. Compounds according to Claim 1,
in which
the thiophenecarboxylic acid substituent is attached to the phenyl ring in the ortho-position to the point of attachment of the fused heterocycle,
R¹ and R² together represent O and
R³ and R⁴ together represent O,
or
R¹ represents hydrogen, hydroxy, methoxy or ethoxy,
R² represents hydrogen and
R³ and R⁴ together represent O,
or
R¹ and R² together represent O,
R³ represents hydrogen, hydroxy, methoxy or ethoxy and
R⁴ represents hydrogen,
R⁵ represents halogen or trifluoromethyl,
A represents (C₁-C₄)-alkanediyl, which may be substituted by hydroxy,
a represents 0 or 1,
B represents a group 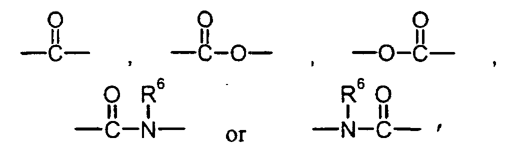
in which
R⁶ represents hydrogen,
b represents 0 or 1,
D represents a 5- to 7-membered heterocyclyl, which may be mono- or disubstituted, independently of one another, by hydroxy, carbamoyl, acetyl, cyclopropanoyl, (C₃-C₆)-cycloalkyl, 5- to 10-membered heterocyclyl, (C₁-C₃)-alkyl,
which for its part may be substituted by hydroxy, methoxy, mono- or dimethylamino, mono- or di-(C₁-C₃)-alkylaminocarbonyl, 5- or 6-membered heterocyclyl, 5- or 6-membered heterocyclylcarbonyl or 5- or 6-membered heteroaryl, phenyl,
which for its part may be substituted by fluorine, chlorine, trifluoromethyl, methyl or methoxy,
or 5- or 6-membered heteroaryl,
which for its part may be substituted by cyano, amino or methyl,
(C₁-C₆)-alkyl,
which may be substituted by amino, mono- or dimethylamino, amidino, 5- or 6-membered heteroaryl, 5- or 6-membered heteroarylamino, by 5- or 6-membered heterocyclyl which is optionally substituted by 5- or 6-membered heteroaryl, or by (C₁-C₄)-alkanoylamino,
or
represents 5- to 9-membered heteroaryl,
and its salts, hydrates, hydrates of the salts and solvates.

3. Compounds according to Claim 1,
in which
the thiophenecarboxylic acid substituent is attached to the phenyl ring in the ortho-position to the point of attachment of the fused heterocycle,
R¹ and R² together represent O and
R³ and R⁴ together represent O,
or
R¹ represents hydrogen, hydroxy or methoxy
R² represents hydrogen and
R³ and R⁴ together represent O,
or
R¹and R² together represent O,
R³ represents hydrogen, hydroxy or methoxy and
R⁴ represents hydrogen,
R⁵ represents chlorine or bromine,
A represents methanediyl, ethanediyl or propane-1,3-diyl, which radicals may be substituted by hydroxy,
a represents 0 or 1,
B represents a group 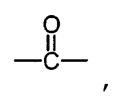
b represents 0 or 1,
D represents pyrolidine, piperidine or piperazine,
which may be mono- or disubstituted, independently of one another, by methyl, ethyl, n-propyl or isopropyl,
which for their part may be substituted by hydroxy or pyridyl,
or pyridyl,
which for its part may be substituted by amino or methyl,
or
(C₁-C₃)-alkyl,
which may be substituted by amidino, optionally pyridyl-substituted piperidinyl or acetylamino,
and its salts, hydrates, hydrates of the salts and solvates.

4. Process for preparing compounds of the formula (1) as defined in Claim 1, **characterized in that**
either
[A.1] compounds of the formula (VII) 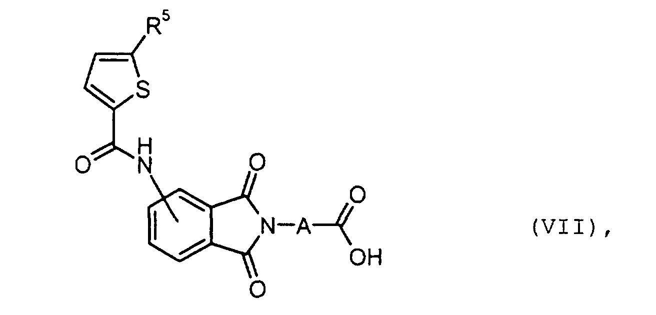 in which A and R⁵ are as defined in Claim 1,
are converted, by reaction with amines or alcohols,
into compounds of the formula (I)
or
[A.2] compounds of the formula (IX) 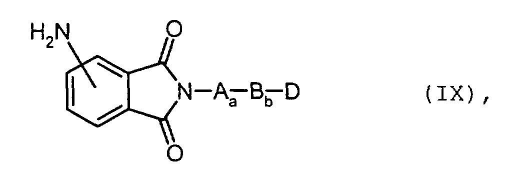 in which a, b, A, B and D are as defined in Claim 1,
are converted with compounds of the formula (III) 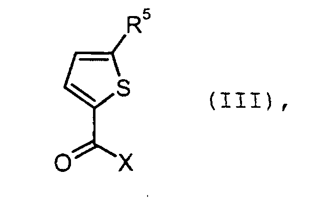 in which R⁵ is as defined in Claim 1 and X represents a leaving group,
into compounds of the formula (I)
or
[B.1] compounds of the formula (XI) 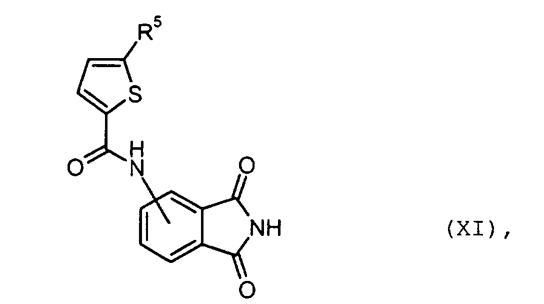 in which R⁵ is as defined in Claim 1,
are converted with compounds of the formula (XII)
HO-Aₐ-B_{b}-D (XII),
in which A, a, B, b and D are as defined in Claim 1,
into compounds of the formula (I)
or
[C] compounds of the formula (XVI) 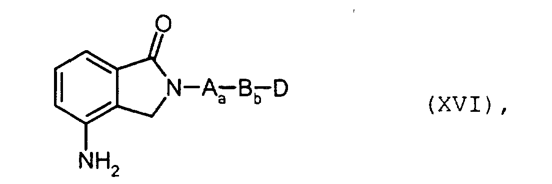 in which A, a, B, b and D are as defined in Claim 1,
are converted with compounds of the formula (III) into compounds of the formula (I),
where the resulting compounds of the formula (I) may, if appropriate, subsequently be subjected to further derivatizations which can be carried out by customary methods.

5. Compounds of the formula (I) as defined in Claim 1 for the prevention and/or treatment of disorders.

6. Medicament, comprising at least one compound of the formula (I) as defined in Claim 1 and at least one further auxiliary.

7. Medicament, comprising at least one compound of the formula (I) as defined in Claim 1 and at least one further active compound.

8. Use of compounds of the formula (I) as defined in Claim 1 for preparing a medicament for the prevention and/or treatment of thromboembolic disorders, in particular myocardial infarction, angina pectoris (including unstable angina), reocclusions and restenoses after angioplasty or aortocoronary bypass, stroke, transitory ischaemic attacks, peripheral arterial occlusive diseases, pulmonary embolisms or deep venous thromboses.

9. Use of compounds of the formula (I) as defined in Claim 1 for preparing a medicament for the prevention and/or treatment of disseminated intravascular coagulation (DIC).

10. Use of compounds of the formula (I) as defined in Claim 1 for preparing a medicament for the prevention and/or treatment of disorders such as atherosclerosis, arthritis, Alzheimer's disease or cancer.

11. Method for preventing the coagulation of blood in vitro, in particular in the case of banked blood or biological samples containing factor Xa, **characterized in that** compounds of the formula (I) as defined in Claim 1 are added.

## Revendications

1. Composés de formule (I) 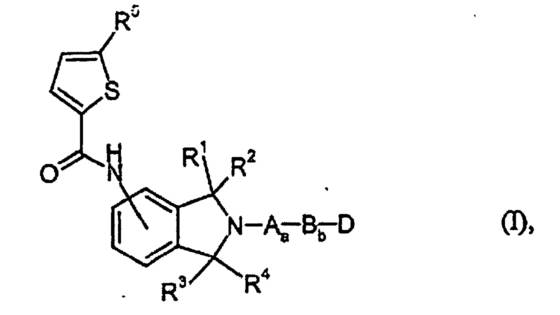 dans laquelle
R¹ et R² forment ensemble O et
R³ et R⁴ forment ensemble O,
ou bien
R¹ représente l'hydrogène, un groupe hydroxy ou un reste alkoxy en C₁ à C₄,
R² représente l'hydrogène et
R³ et R⁴ forment ensemble O,
ou bien
R¹ et R² forment ensemble O,
R³ représente l'hydrogène, un groupe hydroxy ou un reste alkoxy en C₁ à C₄ et
R⁴ représente l'hydrogène,
R⁵ représente un halogène, un reste trifluorométhyle ou méthyle,
A est un reste alcanediyle en C₁ à C₄, qui peut être substitué par un radical hydroxy ou alkoxy en C₁ à C₄,
a a la valeur 0 ou 1,
B représente un groupe 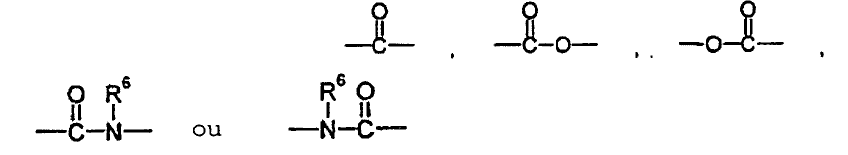 dans lequel
R⁶ représente l'hydrogène ou un reste alkyle en C₁ à C₄,
b a la valeur 0 ou 1,
D est un reste hétérocyclyle pentagonal à heptagonal,
qui peut être substitué une ou deux fois, indépendamment l'une de l'autre, par un groupe hydroxy, carbamoyle, alcanoyle en C₁ à C₄, cycloalkyle en C₃ à C₇ lié par l'intermédiaire d'un groupe carbonyle, cycloalkyle en C₃ à C₇, hétérocyclyle pentagonal à décagonal, (alkoxy en C₁ à C₄)carbonyle, alkyle en C₁ à C₆,
qui peut lui-même être substitué par un radical hydroxy, cyano, alkoxy en C₁ à C₄, mono- ou di(alkyle en C₁ à C₆)amino, mono- ou di(alkyle en C₁ à C₆)aminocarbonyle, hétérocyclyle pentagonal à décagonal, hétérocyclylcarbonyle pentagonal ou hexagonal ou hétéroaryle pentagonal à décagonal, aryle en C₆ à C₁₀,
qui peut lui-même être substitué par un radical halogéno, trifluorométhyle, nitro, alkyle en C₁ à C₄ ou alkoxy en C₁ à C₄,
hétéroaryle pentagonal à décagonal,
qui peut lui-même être substitué par un radical cyano, amino ou alkyle en C₁ à C₄,
ou hétéroarylcarbonyle pentagonal à décagonal, alkyle en C₁ à C₆,
qui peut être substitué par un radical cyano, amino, mono- ou di(alkyle en C₁ à C₆)amino, amidino, hétéroaryle pentagonal à décagonal, hétéroarylamino pentagonal à décagonal éventuellement substitué par un halogène, hétérocyclyle pentagonal à décagonal éventuellement substitué par un radical hétéroaryle pentagonal à décagonal, ou alcanoylamino en C₁ à C₄,
ou bien
hétéroaryle pentagonal à décagonal,
qui peut être substitué par un radical halogéno ou alkyle en C₁ à C₄,
ainsi que leurs sels, leurs hydrates, les hydrates de leurs sels et leurs produits de solvatation.

2. Composés suivant la revendication 1,
dans lesquels
le substituant acide thiophènecarboxylique est lié au noyau phényle en ortho par rapport à la position de liaison de l'hétérocycle condensé,
R¹ et R² forment ensemble O et
R³ et R⁴ forment ensemble O,
ou bien
R¹ représente l'hydrogène, un groupe hydroxy, méthoxy ou éthoxy,
R² représente l'hydrogène et
R³ et R⁴ forment ensemble O,
ou bien
R¹ et R² forment ensemble O,
R³ représente l'hydrogène, un groupe hydroxy, méthoxy ou éthoxy et
R⁴ représente l'hydrogène,
R⁵ est un halogène ou un groupe trifluorométhyle,
A est un reste alcanediyle en C₁ à C₄ qui peut être substitué par un radical hydroxy,
a a la valeur 0 ou 1,
B représente un groupe 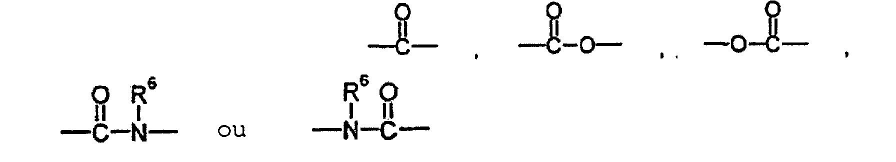
dans lequel
R⁶ représente l'hydrogène,
b a la valeur 0 ou 1,
D est un reste hétérocyclyle pentagonal à heptagonal, qui peut être substitué une ou deux fois indépendantes l'une de l'autre par un radical hydroxy, carbamoyle, acétyle, cyclopropanoyle, cycloalkyle en C₃ à C₆, hétérocyclyle pentagonal à décagonal, alkyle en C₁ à C₃,
qui peut lui-même être substitué par un radical hydroxy, méthoxy, mono- ou diméthylamino, mono- ou di(alkyle en C₁ à C₃)aminocarbonyle, hétérocyclyle pentagonal ou hexagonal, hétérocyclylcarbonyle pentagonal ou hexagonal ou hétéroaryle pentagonal ou hexagonal,
phényle,
qui peut lui-même être substitué par un radical fluoro, chloro, trifluorométhyle, méthyle ou méthoxy,
ou hétéroaryle pentagonal ou hexagonal,
qui peut lui-même être substitué par un radical cyano, amino ou méthyle,
alkyle en C₁ à C₆,
qui peut être substitué par un radical amino, mono- ou diméthylamino, amidino, hétéroaryle pentagonal ou hexagonal, hétéroarylamino pentagonal ou hexagonal, hétérocyclyle pentagonal ou hexagonal éventuellement substitué par un radical hétéroaryle pentagonal ou hexagonal, ou alcanoylamino en C₁ à C₄,
ou bien
un reste hétéroaryle pentagonal à nonagonal,
ainsi que leurs sels, leurs hydrates, les hydrates de leurs sels et leurs produits de solvatation.

3. Composés suivant la revendication 1,
dans lesquels
le substituant acide thiophènecarboxylique est lié au noyau phényle en ortho par rapport à la position de liaison de l'hétérocycle condensé,
R¹ et R² forment ensemble O et
R³ et R⁴ forment ensemble O,
ou bien
R¹ représente l'hydrogène, un groupe hydroxy ou méthoxy,
R² représente l'hydrogène et
R₃ et R₄ forment ensemble O,
ou bien
R¹ et R² forment ensemble O et
R³ représente l'hydrogène, un groupe hydroxy ou méthoxy
et
R⁴ représente l'hydrogène,
R⁵ représente le chlore ou le brome,
A est un groupe méthanediyle, éthanediyle ou propane-1,3-diyle, chacun pouvant être substitué par un radical hydroxy,
a a la valeur 0 ou 1,
B est un groupe 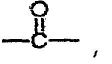
b a la valeur 0 ou 1,
D est un noyau pyrrolidine, un noyau pipéridine ou un noyau pipérazine,
qui peuvent être substitués une ou deux fois, indépendamment l'une de l'autre, par un radical méthyle, éthyle, n-propyle ou isopropyle,
lesquels peuvent être eux-mêmes substitués par un radical hydroxy ou pyridyle,
ou par un radical pyridyle,
qui peut lui-même être substitué par un radical amino ou méthyle,
ou bien
un groupe alkyle en C₁ à C₃,
qui peut être substitué par un groupe amidino, pipéridinyle éventuellement substitué par un radical pyridyle, ou acétylamino,
ainsi que leurs sels, leurs hydrates, les hydrates de leurs sels et leurs produits de solvatation.

4. Procédé de production de composés de formule (I) telle que définie dans la revendication 1, **caractérisé en ce que** :
[A.1] on transforme des composés de formule (VII) 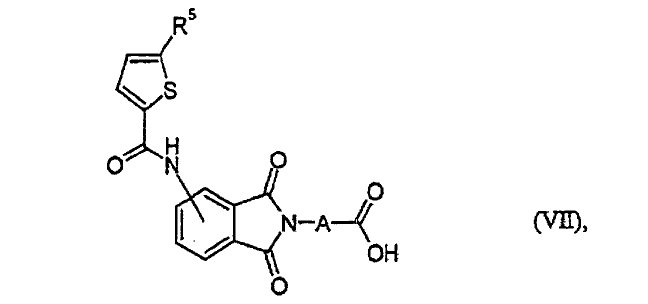 dans lequel A et R⁵ ont la définition indiquée dans la revendication 1,
par réaction avec des amines ou des alcools
en composés de formule (I)
ou bien
[A.2] on fait réagir des composés de formule (IX) 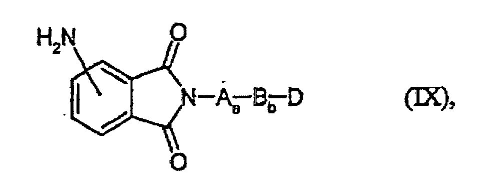 dans laquelle a, b, A, B et D ont la définition indiquée dans la revendication 1,
avec des composés de formule (III) 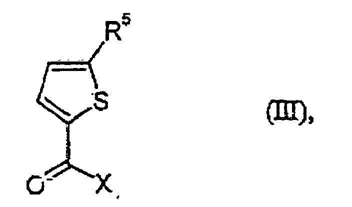 dans laquelle R⁵ a la définition indiquée dans la revendication 1 et X représente un groupe partant,
pour obtenir des composés de formule (I)
ou bien
[B.1] on fait réagir des composés de formule (XI) 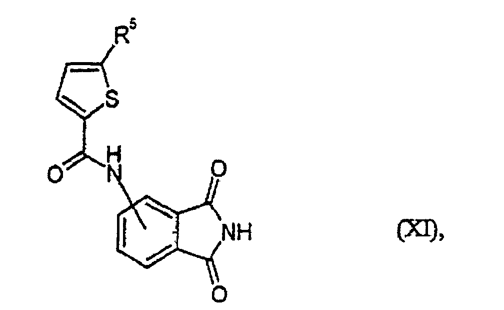 dans laquelle R⁵ a la définition indiquée dans la revendication 1,
avec des composés de formule (XII)
HO - Aₐ - B_{b} - D (XII),
dans laquelle A, a, B, b et D ont la définition indiquée dans la revendication 1,
pour obtenir des composés de formule (I)
ou bien
[C] on fait réagir des composés de formule (XVI) 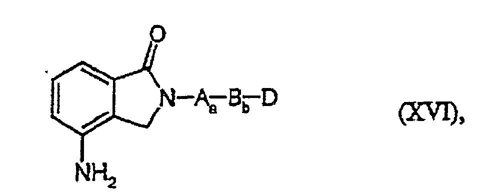 dans laquelle A, a, B, b et D ont la définition indiquée dans la revendication 1,
avec des composés de formule (III) pour obtenir des composés de formule (I),
les composés de formule (I) ainsi obtenus pouvant éventuellement être soumis à d'autres dérivatisations qui peuvent être conduites selon des modes opératoires usuels.

5. Composés de formule (I) telle que définie dans la revendication 1, destinés à la prévention et/ou au traitement de maladies.

6. Médicaments contenant au moins un composé de formule (I) telle que définie dans la revendication 1 et au moins une autre substance auxiliaire.

7. Médicaments contenant au moins un composé de formule (I) telle que définie dans la revendication 1 et au moins une autre substance active.

8. Utilisation de composés de formule (I) telle que définie dans la revendication 1 pour la préparation d'un médicament destiné à la prévention et/au traitement de maladies thromboemboliques, en particulier de l'infarctus du myocarde, de l'angine de poitrine (y compris l'angine instable), de réocclusions et de resténoses après une angioplastie ou un pontage aorto-coronarien, l'apoplexie cérébrale, des attaques ischémiques transitoires, des artériopathies oblitérantes périphériques, des embolies pulmonaires ou des thromboses veineuses profondes.

9. Utilisation de composés de formule (I) telle que définie dans la revendication 1 pour la préparation d'un médicament destiné à la prévention et/ou au traitement de la coagulation intravasculaire disséminée (CIVD).

10. Utilisation de composés de formule (I) telle que définie dans la revendication 1 pour la préparation d'un médicament destiné à la prévention et/ou au traitement de maladies telles que l'athérosclérose, l'arthrite, la maladie d'Alzheimer ou le cancer.

11. Méthode d'inhibition de la coagulation sanguine in vitro, en particulier dans des conserves de sang ou des échantillons biologiques qui contiennent le facteur Xa, **caractérisée en ce qu'**on ajoute des composés de formule (I) telle que définie dans la revendication 1.
